# EUROPEAN PATENT SPECIFICATION

(11) **EP 2 887 964 B1**
(45) Date of publication and mention of the grant of the patent: **03.07.2019**
(21) Application number: 13753904.5
(22) Date of filing: 20.08.2013
(51) Int. Cl.: A61K 47/55, A61K 47/60, A61P 1/00, A61P 9/04, A61P 9/12, A61P 13/12, A61K 31/18, A61K 31/4025, A61K 31/4545, C07D 207/09, C07D 207/12, C07D 211/14, C07D 211/56

(54) **COMPOUNDS AND METHODS FOR INHIBITING NHE-MEDIATED ANTIPORT IN THE TREATMENT OF DISORDERS ASSOCIATED WITH FLUID RETENTION OR SALT OVERLOAD AND GASTROINTESTINAL TRACT DISORDERS**
VERBINDUNGEN UND VERFAHREN ZUR HEMMUNG VON NHE-VERMITTELTEM ANTIPORT BEI DER BEHANDLUNG VON ERKRANKUNGEN IM ZUSAMMENHANG MIT FLÜSSIGKEITSRETENTION ODER SALZÜBERLASTUNG UND ERKRANKUNGEN DES GASTROINTESTINALTRAKTES
COMPOSÉS ET PROCÉDÉS D'INHIBITION D'UN ANTIPORT À MÉDIATION PAR NHE DANS LE TRAITEMENT DE TROUBLES ASSOCIÉS À UNE RÉTENTION DE FLUIDE OU À UNE SURCHARGE DE SEL ET DE TROUBLES DU TRACTUS GASTRO-INTESTINAL

(30) Priority: 21.08.2012 US 201261691635 P
(43) Date of publication of application: 01.07.2015
(73) Proprietor: Ardelyx, Inc., Fremont, California 94555 (US)
(72) Inventor: LEADBETTER, Michael, Fremont, California 94555 (US); BELL, Noah, Fremont, California 94555 (US); LEWIS, Jason, Fremont, California 94555 (US); JACOBS, Jeffrey, Fremont, California 94555 (US); CARRERAS, Christopher, Fremont, California 94555 (US)
(74) Representative: Grund, Martin
(86) International application number: PCT/GB2013/052192
(87) International publication number: WO 2014/029983

(56) References cited:
- WO-A1-2010/025856
- WO-A2-2010/078449
- COLIN A ET AL: "Characterisation of SB-221420-A - a neuronal Ca2+ and Na+ channel antagonist in experimental models of stroke", EUROPEAN JOURNAL OF PHARMACOLOGY,, vol. 401, 1 January 2000 (2000-01-01), pages 419-428, XP002552396, DOI: 10.1016/S0014-2999(00)00470-2

## Description

### BACKGROUND

### Field

The present disclosure is directed to compounds that are substantially active in the gastrointestinal tract to inhibit NHE-mediated antiport of sodium ions and hydrogen ions, and to such compounds for use in the treatment of disorders associated with fluid retention or salt overload and in the treatment of gastrointestinal tract disorders, including the treatment or reduction of pain associated with a gastrointestinal tract disorder.

### Description of the Related Art

### Disorders Associated with Fluid Retention and Salt Overload

According to the American Heart Association, more than 5 million Americans have suffered from heart failure, and an estimated 550,000 cases of congestive heart failure (CHF) occur each year (Schocken, D. D. et al., Prevention of heart failure: a scientific statement from the American Heart Association Councils on Epidemiology and Prevention, Clinical Cardiology, Cardiovascular Nursing, and High Blood Pressure Research*;* Quality of Care and Outcomes Research Interdisciplinary Working Group; and Functional Genomics and Translational Biology Interdisciplinary Working Group: Circulation, v. 117, no. 19, p. 2544-2565 (2008)). The clinical syndrome of congestive heart failure occurs when cardiac dysfunction prevents adequate perfusion of peripheral tissues. The most common form of heart failure leading to CHF is systolic heart failure, caused by contractile failure of the myocardium. A main cause of CHF is due to ischemic coronary artery disease, with or without infarction. Long standing hypertension, particularly when it is poorly controlled, may lead to CHF.

In patients with CHF, neurohumoral compensatory mechanisms (i.e., the sympathetic nervous system and the renin-angiotensin system) are activated in an effort to maintain normal circulation. The renin-angiotensin system is activated in response to decreased cardiac output, causing increased levels of plasma renin, angiotensin II, and aldosterone. As blood volume increases in the heart, cardiac output increases proportionally, to a point where the heart is unable to dilate further. In the failing heart, contractility is reduced, so the heart operates at higher volumes and higher filling pressures to maintain output. Filling pressures may eventually increase to a level that causes transudation of fluid into the lungs and congestive symptoms (e.g., edema, shortness of breath). All of these symptoms are related to fluid volume and salt retention, and this chronic fluid and salt overload further contribute to disease progression.

Compliance with the medication regimen and with dietary sodium restrictions is a critical component of self-management for patients with heart failure and may lengthen life, reduce hospitalizations and improve quality of life. Physicians often recommend keeping salt intake below 2.3 g per day and no more than 2 g per day for people with heart failure. Most people eat considerably more than this, so it is likely that a person with congestive heart failure will need to find ways to reduce dietary salt.

A number of drug therapies currently exist for patients suffering from CHF. For example, diuretics may be used or administered to relieve congestion by decreasing volume and, consequently, filling pressures to below those that cause pulmonary edema. By counteracting the volume increase, diuretics reduce cardiac output; however, fatigue and dizziness may replace CFIF symptoms. Among the classes or types of diuretics currently being used is thiazides. Thiazides inhibit NaCl transport in the kidney, thereby preventing reabsorption of Na in the cortical diluting segment at the ending portion of the loop of Henle and the proximal portion of the distal convoluted tubule. However, these drugs are not effective when the glomerular filtration rate (GFR) is less than 30 ml/min. Additionally, thiazides, as well as other diuretics, may cause hypokalemia. Also among the classes or types of diuretics currently being used is loop diuretics (e.g., furosemide). These are the most potent diuretics and are particularly effective in treating pulmonary edema. Loop diuretics inhibit the NaKCl transport system, thus preventing reabsorption of Na in the loop of Henle.

Patients that have persistent edema despite receiving high doses of diuretics may be or become diuretic-resistant. Diuretic resistance may be caused by poor availability of the drug. In patients with renal failure, which has a high occurrence in the CHF population, endogenous acids compete with loop diuretics such as furosemide for the organic acid secretory pathway in the tubular lumen of the nephron. Higher doses, or continuous infusion, are therefore needed to achieve entrance of an adequate amount of drug into the nephron. However, recent meta-analysis have raised awareness about the long-term risk of chronic use of diuretics in the treatment of CHF. For instance, in a recent study (Ahmed et al., Int J Cardiol. 2008 April 10; 125(2): 246-253) it was shown that chronic diuretic use was associated with significantly increased mortality and hospitalization in ambulatory older adults with heart failure receiving angiotensin converting enzyme inhibitor and diuretics.

Angiotensin-converting enzyme ("ACE") inhibitors are an example of another drug therapy that may be used to treat congestive heart failure. ACE inhibitors cause vasodilatation by blocking the renin-angiotensin-aldosterone system. Abnormally low cardiac output may cause the renal system to respond by releasing renin, which then converts angiotensinogen into angiotensin I. ACE converts angiotensin I into angiotensin II. Angiotensin II stimulates the thirst centers in the hypothalamus and causes vasoconstriction, thus increasing blood pressure and venous return. Angiotensin II also causes aldosterone to be released, causing reabsorption of Na and concomitant passive reabsorption of fluid, which in turn causes the blood volume to increase. ACE inhibitors block this compensatory system and improve cardiac performance by decreasing systemic and pulmonary vascular resistance. ACE inhibitors have shown survival benefit and conventionally have been a treatment of choice for CHF. However, since ACE inhibitors lower aldosterone, the K-secreting hormone, one of the side-effects of their use is hyperkalemia. In addition, ACE inhibitors have been show to lead to acute renal failure in certain categories of CHF patients. (See, e.g., C.S. Cruz et al., "Incidence and Predictors of Development of Acute Renal Failure Related to the Treatment of Congestive Heart Failure with ACE Inhibitors, Nephron Clin. Pract, v. 105, no. 2, pp c77-c83 (2007)).

Patients with end stage renal disease ("ESRD"), i.e., stage 5 chronic kidney failure, must undergo hemodialysis three times per week. The quasi-absence of renal function and ability to eliminate salt and fluid results in large fluctuations in body weight as fluid and salt build up in the body (sodium/volume overload). The fluid overload is characterized as interdialytic weight gain. High fluid overload is also worsened by heart dysfunction, specifically CHF. Dialysis is used to remove uremic toxins and also adjust salt and fluid homeostasis. However, symptomatic intradialytic hypotension (SIH) may occur when patients are over-dialyzed. SIH is exhibited in about 15% to 25% of the ESRD population (Davenport, A., C. Cox, and R. Thuraisingham, Blood pressure control and symptomatic intradialytic hypotension in diabetic haemodialysis patients: a cross-sectional survey; Nephron Clin. Pract., v. 109, no. 2, p. c65-c71 (2008)). Like in hypertensive and CHF patients, dietary restrictions of salt and fluid are highly recommended but poorly followed because of the poor palatability of low-salt food

The cause of primary or "essential" hypertension is elusive. However, several observations point to the kidney as a primary factor. The strongest data for excess salt intake and elevated blood pressure come from INTERSALT, a cross-sectional study of greater than 10,000 participants. For individuals, a significant, positive, independent linear relation between 24-hour sodium excretion and systolic blood pressure was found. Higher individual 24-hour urinary sodium excretions were found to be associated with higher systolic/diastolic blood pressure on average, by 6-3/3-0 mm Hg. Primary hypertension is a typical example of a complex, multifactorial, and polygenic trait. All these monogenic hypertensive syndromes are virtually confined to mutated genes involving gain of function of various components of the renin-angiotensin-aldosterone system, resulting in excessive renal sodium retention. In a broad sense, these syndromes are characterized by increased renal sodium reabsorption arising through either primary defects in sodium transport systems or stimulation of mineralocorticoid receptor activity (Altun, B., and M. Arid, 2006, Salt and blood pressure: time to challenge; Cardiology, v. 105, no. 1, p. 9-16 (2006)). A much larger number of controlled studies have been performed on hypertensive subjects during the last three decades to determine whether sodium reduction will reduce established high blood pressure. Meta-analyses of these studies have clearly shown a large decrease in blood pressure in hypertensive patients.

In end stage liver disease (ESLD), accumulation of fluid as ascites, edema or pleural effusion due to cirrhosis is common and results from a derangement in the extracellular fluid volume regulatory mechanisms. Fluid retention is the most frequent complication of ESLD and occurs in about 50% of patients within 10 years of the diagnosis of cirrhosis. This complication significantly impairs the quality of life of cirrhotic patients and is also associated with poor prognosis. The one-year and five-year survival rate is 85% and 56%, respectively (Kashani et al., Fluid retention in cirrhosis: pathophysiology and management; QJM, v. 101, no. 2, p. 71-85 (2008)). The most acceptable theories postulate that the initial event in ascites formation in the cirrhotic patient is sinusoidal hypertension. Portal hypertension due to an increase in sinusoidal pressure activates vasodilatory mechanisms. In advanced stages of cirrhosis, arteriolar vasodilation causes underfilling of systemic arterial vascular space. This event, through a decrease in effective blood volume, leads to a drop in arterial pressure. Consequently, baroreceptor-mediated activation of renin-angiotensin aldosterone system, sympathetic nervous system and nonosmotic release of antidiuretic hormone occur to restore the normal blood homeostasis. These events cause further retention of renal sodium and fluid. Splanchnic vasodilation increases splanchnic lymph production, exceeding the lymph transportation system capacity, and leads to lymph leakage into the peritoneal cavity. Persistent renal sodium and fluid retention, alongside increased splanchnic vascular permeability in addition to lymph leakage into the peritoneal cavity, play a major role in a sustained ascites formation.

Thiazolidinediones (TZD's), such as rosiglitazone, are peroxisome proliferator-activated receptor (PPAR) gamma agonist agents used for the treatment of type-2 diabetes and are widely prescribed. Unfortunately, fluid retention has emerged as the most common and serious side-effect of TZD's and has become the most frequent cause of discontinuation of therapy. The incidence of TZD-induced fluid retention ranges from 7% in monotherapy and to as high as 15% when combined with insulin (Yan, T., Soodvilai, S., PPAR Research volume 2008, article ID 943614). The mechanisms for such side-effects are not fully understood but may be related in Na and fluid re-absorption in the kidney. However TZD-induced fluid retention is resistant to loop diuretics or thiazide diuretics, and combination of peroxisome proliferator-activated receptor (PPAR) alpha with PPAR gamma agonists, which were proposed to reduce such fluid overload, are associated with major adverse cardiovascular events.

In view of the foregoing, it is recognized that salt and fluid accumulation contribute to the morbidity and mortality of many diseases, including heart failure (in particular, congestive heart failure), chronic kidney disease, end-stage renal disease, liver disease and the like. It is also accepted that salt and fluid accumulation are risk factors for hypertension. Accordingly, there is a clear need for a medicament that, when administered to a patient in need, would result in a reduction in sodium retention, fluid retention, or preferably both. Such a medicament would more preferably also not involve or otherwise impair renal mechanisms of fluid/Na homeostasis.

One option to consider for treating excessive fluid overload is to induce diarrhea. Diarrhea may be triggered by several agents including, for example, laxatives such as sorbitol, polyethyleneglycol, bisacodyl and phenolphthaleine. Sorbitol and polyethyleneglycol triggers osmotic diarrhea with low levels of secreted electrolytes; thus, their utility in removing sodium salt from the GI tract is limited. The mechanism of action of phenolphthalein is not clearly established, but is thought to be caused by inhibition of the Na/K ATPase and the CI/HCO₃ anion exchanger and stimulation of electrogenic anion secretion (see, e.g., Eherer, A. J., C. A. Santa Ana, J. Porter, and J. S. Fordtran, 1993, Gastroenterology, v. 104, no. 4, p. 1007-1012). However, some laxatives, such as phenolphthalein, are not viable options for the chronic treatment of fluid overload, due to the potential risk of carcinogenicity in humans. Furthermore, laxatives may not be used chronically, as they have been shown to be an irritant and cause mucosal damage. Accordingly, it should also be recognized that the induction of chronic diarrhea as part of an effort to control salt and fluid overload would be an undesired treatment modality for most patients. Any medicament utilizing the GI tract for this purpose would therefore need to control diarrhea in order to be of practical benefit.

One approach for the treatment of mild diarrhea is the administration of a fluid-absorbing polymer, such as the natural plant fiber psyllium. Polymeric materials, and more specifically hydrogel polymers, may also be used for the removal of fluid from the gastrointestinal (GI) tract. The use of such polymers is described in, for example, U.S. Pat. No. 4,470,975 and No. 6,908,609. However, for such polymers to effectively remove significant quantities of fluid, they must desirably resist the static and osmotic pressure range existing in the GI tract. Many mammals, including humans, make a soft feces with a water content of about 70%, and do so by transporting fluid against the high hydraulic resistance imposed by the fecal mass. Several studies show that the pressure required to dehydrate feces from about 80% to about 60% is between about 500 kPa and about 1000 kPa (i.e., about 5 to about 10 atm). (See, e.g., McKie, A. T., W. Powrie, and R. J. Naftalin, 1990, Am J Physiol, v. 258, no. 3 Pt 1, p. G391-G394; Bleakman, D., and R. J. Naftalin, 1990, Am J Physiol, v. 258, no. 3 Pt 1, p. G377-G390; Zammit, P. S., M. Mendizabal, and R. J. Naftalin, 1994, J Physiol, v. 477 (Pt 3), p. 539- 548.) However, the static pressure measured intraluminally is usually between about 6 kPa and about 15 kPa. The rather high pressure needed to dehydrate feces is essentially due to an osmotic process and not a mechanical process produced by muscular forces. The osmotic pressure arises from the active transport of salt across the colonic mucosa that ultimately produces a hypertonic fluid absorption. The osmotic gradient produced drives fluid from the lumen to the serosal side of the mucosa. Fluid-absorbing polymers, such as those described in for example U.S. Patent Nos. 4,470,975 and 6,908,609, may not be able to sustain such pressure. Such polymers may collapse in a normal colon where the salt absorption process is intact, hence removing a modest quantity of fluid and thereby salt.

Synthetic polymers that bind sodium have also been described. For example, ion-exchange polymeric resins, such as Dowex-type cation exchange resins, have been known since about the 1950's. However, with the exception of Kayexalate™ (or Kionex™), which is a polystyrene sulfonate salt approved for the treatment of hyperkalemia, cation exchange resins have very limited use as drugs, due at least in part to their limited capacity and poor cation binding selectivity. Additionally, during the ion- exchange process, the resins may release a stochiometric amount of exogenous cations (e.g., H, K, Ca), which may in turn potentially cause acidosis (H), hyperkalemia (K) or contribute to vascular calcification (Ca). Such resins may also cause constipation.

### Gastrointestinal Tract Disorders

Constipation is characterized by infrequent and difficult passage of stool and becomes chronic when a patient suffers specified symptoms for over 12 non- consecutive weeks within a 12-month period. Chronic constipation is idiopathic if it is not caused by other diseases or by use of medications. An evidence-based approach to the management of chronic constipation in North America (Brandt et al., 2005, Am. J. Gastroenterol. 100(Suppl. 1):S5-S21) revealed that prevalence is approximately 15% of the general population. Constipation is reported more commonly in women, the elderly, non- whites, and individuals from lower socioeconomic groups.

Irritable bowel syndrome (IBS) is a common GI disorder associated with alterations in motility, secretion and visceral sensation. A range of clinical symptoms characterizes this disorder, including stool frequency and form, abdominal pain and bloating. The recognition of clinical symptoms of IBS are yet to be defined, but it is now common to refer to diarrhea-predominant IBS (D-IBS) and constipation-predominant IBS (C-IBS), wherein D-IBS is defined as continuous passage of loose or watery stools and C- IBS as a group of functional disorders which present as difficult, infrequent or seemingly incomplete defecation. The pathophysiology of IBS is not fully understood, and a number of mechanisms have been suggested. Visceral hypersensitivity is often considered to play a major etiologic role and has been proposed to be a biological marker even useful to discriminate IBS from other causes of abdominal pain. In a recent clinical study (Posserud, I. et al, Gastroenterology, 2007; 133 : 1113-1123) IBS patients were submitted to a visceral sensitivity test (Balloon distention) and compared with healthy subjects. It revealed that 61% of the IBS patients had an altered visceral perception as measured by pain and discomfort threshold. Other reviews have documented the role of visceral hypersensitivity in abdominal pain symptomatic of various gastrointestinal tract disorders (Akbar, A, et al, Aliment. Pharmaco. Ther., 2009, 30, 423-435; Bueno et al., Neurogastroenterol Motility (2007) 19 (suppl. 1), 89-119). Colonic and rectal distention have been widely used as a tool to assess visceral sensitivity in animal and human studies. The type of stress used to induce visceral sensitivity varies upon the models (see for instance Eutamen, H Neurogastroenterol Motil. 2009 Aug 25. [Epub ahead of print]), however stress such as Partial restraint stress (PRS) is a relatively mild, non-ulcerogenic model that is considered more representative of the IBS setting.

Constipation is commonly found in the geriatric population, particularly patients with osteoporosis who have to take calcium supplements. Calcium supplements have shown to be beneficial in ostoporotic patients to restore bone density but compliance is poor because of calcium-induced constipation effects.

Opioid-induced constipation (OIC) (also referred to as opioid-induced bowel dysfunction or opioid bowel dysfuntion (OBD)) is a common adverse effect associated with opioid therapy. OIC is commonly described as constipation; however, it is a constellation of adverse gastrointestinal (GI) effects, which also includes abdominal cramping, bloating, and gastroesophageal reflux. Patients with cancer may have disease- related constipation, which is usually worsened by opioid therapy. However, OIC is not limited to cancer patients. A recent survey of patients taking opioid therapy for pain of non-cancer origin found that approximately 40% of patients experienced constipation related to opioid therapy (<3 complete bowel movements per week) compared with 7.6% in a control group. Of subjects who required laxative therapy, only 46% of opioid-treated patients (control subjects, 84%) reported achieving the desired treatment results >50% of the time (Pappagallo, 2001, Am. J. Surg. 182(5A Suppl.): 11S-18S).

Some patients suffering from chronic idiopathic constipation can be successfully treated with lifestyle modification, dietary changes and increased fluid and fiber intake, and these treatments are generally tried first. For patients who fail to respond to these approaches, physicians typically recommend laxatives, most of which are available over-the-counter. Use of laxatives provided over-the-counter is judged inefficient by about half of the patients (Johanson and Kralstein, 2007, Aliment. Pharmacol. Ther. 25(5):599-608). Other therapeutic options currently prescribed or in clinical development for the treatment of IBS and chronic constipation including OIC are described in, for example: Chang et al., 2006, Curr. Teat. Options Gastroenterol. 9(4):314-323; Gershon and Tack, 2007, Gastroenterology 132(1):397-414; and, Hammerle and Surawicz, 2008, World J. Gastroenterol. 14(17): 2639-2649. Such treatments include but are not limited to serotonin receptor ligands, chloride channel activators, opioid receptor antagonists, guanylate-cyclase receptor agonists and nucleotide P2Y(2) receptor agonists. Many of these treatment options are inadequate, as they may be habit forming, ineffective in some patients, may cause long term adverse effects, or otherwise are less than optimal.

### Na⁺/ H⁺ Exchanger (NHE) Inhibitors

A major function of the GI tract is to maintain water/Na homeostasis by absorbing virtually all water and Na to which the GI tract is exposed. The epithelial layer covering the apical surface of the mammalian colon is a typical electrolyte-transporting epithelium, which is able to move large quantities of salt and water in both directions across the mucosa. For example, each day the GI tract processes about 9 liters of fluid and about 800 meq of Na. (See, e.g., Zachos et al., Molecular physiology of intestinal Na+/H+ exchange; Annu. Rev. Physiol., v. 67, p. 411-443 (2005).) Only about 1.5 liters of this fluid and about 150 meq of this sodium originates from ingestion; rather, the majority of the fluid (e.g., about 7.5 liters) and sodium (about 650 meq) is secreted via the GI organs as part of digestion. The GI tract therefore represents a viable target for modulating systemic sodium and fluid levels.

Many reviews have been published on the physiology and secretory and/or absorption mechanisms of the GI tract (see, e.g., Kunzelmann et al., Electrolyte transport in the mammalian colon: mechanisms and implications for disease; Physiol. Rev., v. 82, no. 1, p. 245-289 (2002); Geibel, J. P.; Secretion and absorption by colonic crypts; Annu. Rev. Physiol, v. 67, p. 471-490 (2005); Zachos et al., supra; Kiela, P. R. et al., Apical NA+/H+ exchangers in the mammalian gastrointestinal tract; J. Physiol. Pharmacol., v. 57 Suppl. 7, p. 51-79 (2006)). The two main mechanisms of Na absorption are electroneutral and electrogenic transport. Electroneutral transport is essentially due to the Na⁺/H⁺ antiport NHE (e.g., NHE-3) and is responsible for the bulk of Na absorption. Electrogenic transport is provided by the epithelium sodium channel ("ENaC"). Electroneutral transport is located primarily in the ileal segment and proximal colon and electrogenic transport is located in the distal colon.

Plasma membrane NHEs contribute to maintenance of intracellular pH and volume, transcellular absorption of NaCl and NaHCO₃, and fluid balance carried out by epithelial cells, especially in the kidney, intestine, gallbladder, and salivary glands, as well as regulation of systemic pH. There exists a body of literature devoted to the role and clinical intervention on systemic NHEs to treat disorders related to ischemia and reperfusion for cardioprotection or renal protection. Nine isoforms of NHEs have been identified (Kiela, P. R., et al.; Apical NA+/H+ exchangers in the mammalian gastrointestinal tract; J. Physiol. Pharmacol., v. 57 Suppl 7, p. 51-79 (2006)), of which NHE-2, NHE-3 and NHE-8 are expressed on the apical side of the GI tract, with NHE-3 providing a larger contribution to transport. Another, yet to be identified, Cl-dependant NHE has been identified in the crypt of rat cells. In addition, much research has been devoted to identifying inhibitors of NHEs. The primary targets of such research have been NHE-1 and NHE-3. Small molecule NHE inhibitors are, for example, described in: U.S. Patent Nos. 5,866,610; 6,399,824; 6,911,453; 6,703,405; 6,005,010; 6,736,705; 6,887,870; 6,737,423; 7,326,705; 5,824,691 (WO 94/026709); 6,399,824 (WO 02/024637); U.S. Pat. Pub. Nos. 2004/0039001 (WO 02/020496); 2005/0020612 (WO 03/055490); 2004/0113396 (WO 03/051866); 2005/0020612; 2005/0054705; 2008/0194621; 2007/0225323; 2004/0039001; 2004/0224965; 2005/0113396; 2007/0135383; 2007/0135385; 2005/0244367; 2007/0270414; International Publication Nos. WO 01/072742; WO 01/021582 (CA2387529); WO 97/024113 (CA02241531) and European Pat. No. EP0744397 (CA2177007).

However, such research failed to develop or recognize the value or importance of NHE inhibitors that are not absorbed (i.e., not systemic) and target the gastrointestinal tract, as disclosed recently in WO 2010/078449. Such inhibitors can be utilized in the treatment of disorders associated with fluid retention and salt overload and in the treatment of GI tract disorders, including the treatment or reduction of pain associated with a gastrointestinal tract disorder. Such inhibitors are particular advantageous because they can be delivered with reduced fear of systemic on-target or off- target effects (e.g., little or no risk of renal involvement or other systemic effects.

WO 2010/025856 A1 discloses substituted aminoindanes and pharmacological uses thereof.

Accordingly, while progress has been made in the foregoing fields, there remains a need in the art for novel compounds for use in the disorders associated with fluid retention and salt overload and in the treatment of gastrointestinal tract disorders, including the treatment or reduction of pain associated with a gastrointestinal tract disorder. The present invention fulfills this need and provides further related advantages.

### BRIEF SUMMARY

The invention is defined by the appended claims. Any references in the description to methods of treatment refer to the compounds, pharmaceutical compositions and medicaments of the present invention for use in a method for treatment of the human (or animal) body by therapy.

In brief, the present invention is directed to compounds that are substantially active in the gastrointestinal tract to inhibit NHE-mediated antiport of sodium ions and hydrogen ions, and to such compounds for use in the treatment of disorders associated with fluid retention and salt overload and in the treatment of gastrointestinal tract disorders, including the treatment or reduction of pain associated with a gastrointestinal tract disorder.

In one embodiment, a compound is provided having the following structure of Formula (I):

Core(̵L-NHE)ₙ (I)

or a pharmaceutically acceptable salt thereof,
wherein:
(a) n is 2 or 3;
(b) Core is a Core moiety having two or three sites thereon for attachment to two or three NHE-inhibiting small molecule moieties;
(c) L is a bond or linker connecting the Core moiety to the two or three NHE-inhibitory small molecule moieties; and
(d) NHE is a NHE-inhibiting small molecule moiety having the following structure of Formula (XI): 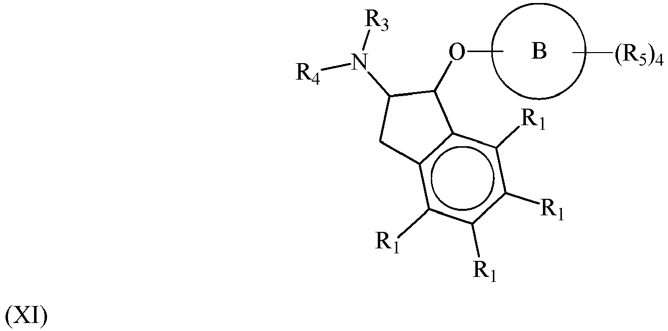 wherein:
   B is aryl;
   each R⁵ is independently selected from the group consisting of hydrogen, halogen, optionally substituted C₁₋₄alkyl, optionally substituted C₁₋₄alkoxy, optionally substituted C₁₋₄thioalkyl, optionally substituted heterocyclyl, optionally substituted heterocyclylalkyl, optionally substituted aryl, optionally substituted heteroaryl, hydroxyl, oxo, cyano, nitro, -NR₇R₈, -NR₇C(=O)R₈, -NR₇C(=O)OR₈, -NR₇C(=O)NR₈R₉, -NR₇SO₂R₈, -NR₇S(O)₂NR₈R₉, -C(=O)OR₇, -C(=O)R₇, -C(=O)NR₇R₈, -S(O)₁₋₂R₇, and -SO₂NR₇R₈, wherein R₇, R₈, and R₉ are independently selected from the group consisting of hydrogen, C₁₋₄alkyl or a bond linking the NHE-inhibiting small molecule moiety to L, provided at least one is a bond linking the NHE-inhibiting small molecule moiety to L;
   R₃ and R₄ are independently selected from the group consisting of hydrogen, optionally substituted C₁₋₄alkyl, optionally substituted cycloalkyl, optionally substituted cycloalkylalkyl, optionally substituted aryl, optionally substituted aralkyl, optionally substituted heterocyclyl and optionally substituted heteroaryl; or
   R₃ and R₄ form together with the nitrogen to which they are bonded an optionally substituted 4-8 membered heterocyclyl;
   and each R₁ is independently selected from the group consisting of hydrogen, halogen, optionally substituted C₁₋₆alkyl and optionally substituted C₁₋₆alkoxy.

In more specific embodiments, n is 2.

In other more specific embodiments, L is a polyalkylene glycol linker. For example, in certain embodiments, L is a polyethylene glycol linker.

In other more specific embodiments, the Core has the following structure: 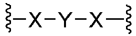 wherein:
X is selected from the group consisting of a bond, -O-, -NH-, -S-, C₁₋₆alkylene, -NHC(=O)-, -C(=O)NH-, -NHC(=O)NH-, -SO₂NH-, and -NHSO₂-;
Y is selected from the group consisting of a bond, optionally substituted C₁₋₈alkylene, optionally substituted aryl, optionally substituted heteroaryl, a polyethylene glycol linker, -(CH₂)₁₋₆O(CH₂)₁₋₆- and -(CH₂)₁₋₆NY₁(CH₂)₁₋₆-; and
Y₁ is selected from the group consisting of hydrogen, optionally substituted C₁₋₈alkyl, optionally substituted aryl or optionally substituted heteroaryl.

For example, in certain embodiments, the Core is selected from the group consisting of:

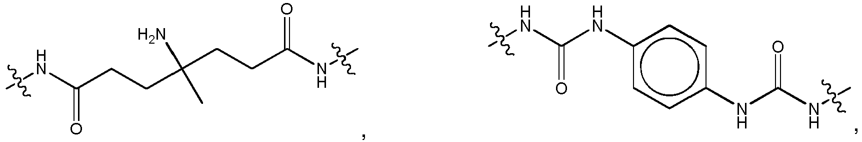

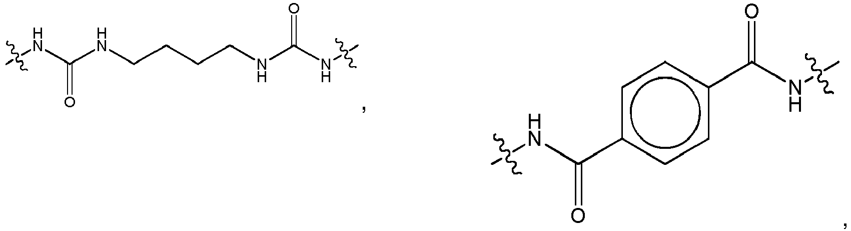

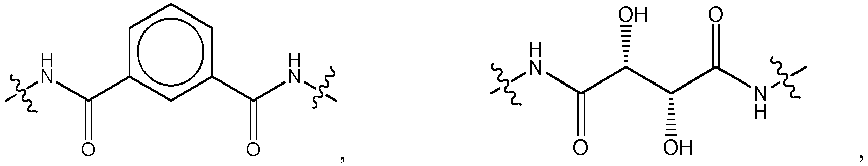

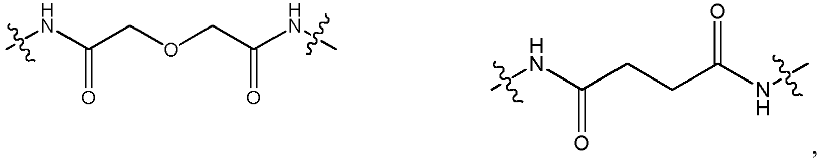

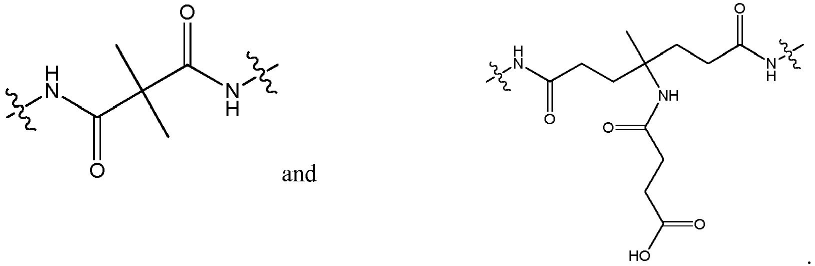

In other more specific embodiments, the NHE-inhibiting small molecule moiety has the following structure of Formula (XII): 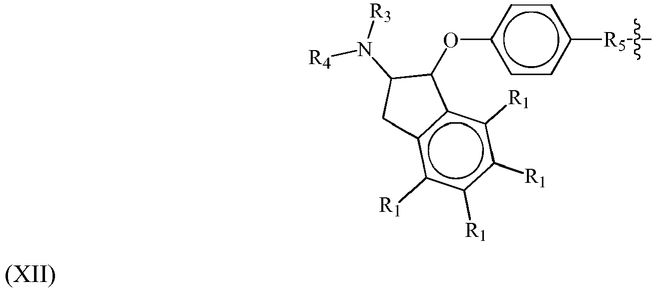 wherein:
each R₃ and R₄ are independently selected from the group consisting of hydrogen and optionally substituted C₁₋₄alkyl, or R₃ and R₄, taken together with the nitrogen to which they are bonded, form an optionally substituted 4-8 membered heterocyclyl;
each R₁ is independently selected from the group consisting of hydrogen, halogen, C₁₋₆alkyl, and C₁₋₆haloalkyl; and
R₅ is selected from the group consisting of -SO₂-R₇- and NHC(=O)NH-, wherein R₇ is hydrogen or C₁₋₄alkyl.

In further more specific embodiments, R₃ and R₄, taken together with the nitrogen to which they are bonded, form an optionally substituted 5 or 6 membered heterocyclyl. For example, in certain embodiments, (i) the optionally substituted 5 or 6 membered heterocyclyl is pyrrolidinyl or piperidinyl, or (ii) the optionally substituted 5 or 6 membered heterocyclyl is pyrrolidinyl or piperidinyl, each substituted with at least one amino or hydroxyl.

In other further more specific embodiments, R₃ and R₄ are independently C₁₋₄alkyl. For example, in certain embodiments, R₃ and R₄ are methyl.

In other further more specific embodiments, each R₁ is independently selected from the group consisting of hydrogen or halogen. For example, in certain embodiments, each R₁ is independently selected from the group consisting of hydrogen, F and Cl.

In another embodiment, a pharmaceutical composition is provided comprising a compound as set forth above, or a pharmaceutically acceptable salt thereof, and a pharmaceutically acceptable carrier, diluent or excipient.

In further embodiments, the composition further comprises a fluid-absorbing polymer. In further embodiments, the fluid-absorbing polymer is delivered directly to the colon. In further embodiments, the fluid-absorbing polymer has a fluid absorbency of at least about 15 g of isotonic fluid per g of polymer under a static pressure of about 5 kPa. In further embodiments, the fluid-absorbing polymer has a fluid absorbency of at least about 15 g of isotonic fluid per g of polymer under a static pressure of about 10 kPa. In further embodiments, the fluid-absorbing polymer is characterized by a fluid absorbency of at least about 10 g/g. In further embodiments, the fluid-absorbing polymer is characterized by a fluid absorbency of at least about 15 g/g. In further embodiments, the fluid-absorbing polymer is superabsorbent. In further embodiments, the fluid-absorbing polymer is a crosslinked, partially neutralized polyelectrolyte hydrogel. In further embodiments, the fluid-absorbing polymer is a crosslinked polyacrylate. In further embodiments, the fluid-absorbing polymer is a polyelectrolyte. In further embodiments, the fluid-absorbing polymer is calcium Carbophil. In further embodiments, the fluid- absorbing polymer is prepared by a high internal phase emulsion process. In further embodiments, the fluid-absorbing polymer is a foam. In further embodiments, the fluid- absorbing polymer is prepared by a aqueous free radical polymerization of acrylamide or a derivative thereof, a crosslinker and a free radical initiator redox system in water. In further embodiments, the fluid-absorbing polymer is a hydrogel. In further embodiments, the fluid-absorbing polymer is an N-alkyl acrylamide. In further embodiments, the fluid- absorbing polymer is a superporous gel. In further embodiments, the fluid-absorbing polymer is naturally occurring. In further embodiments, the fluid-absorbing polymer is selected from the group consisting of xanthan, guar, wellan, hemicelluloses, alkyl-cellulose hydro-alkyl-cellulose, carboxy-alkyl-cellulose, carrageenan, dextran, hyaluronic acid and agarose. In further embodiments, the fluid-absorbing polymer is psyllium. In further embodiments, the fluid-absorbing polymer is a polysaccharide that includes xylose and arabinose. In further embodiments, the fluid-absorbing polymer is a polysaccharide that includes xylose and arabinose, wherein the ratio of xylose to arabinose is at least about 3 : 1, by weight.

In further embodiments, the composition further comprises another pharmaceutically active agent or compound. In further embodiments, the composition further comprises another pharmaceutically active agent or compound selected from the group consisting of a diuretic, cardiac glycoside, ACE inhibitor, angiotensin-2 receptor antagonist, aldosterone antagonist, aldosterone synthase inhibitor, renin inhibitor, calcium channel blocker, beta blocker, alpha blocker, central alpha agonist, vasodilator, blood thinner, anti-platelet agent, lipid-lowering agent, and peroxisome proliferator-activated receptor (PPAR) gamma agonist agent. In further embodiments, the diuretic is selected from the group consisting of a high ceiling loop diuretic, a benzothiadiazide diuretic, a potassium sparing diuretic, and a osmotic diuretic. In further embodiments, the composition further comprises another pharmaceutically active agent or compound selected from the group consisting of an analgesic peptide or agent. In further embodiments, the composition further comprises another pharmaceutically active agent or compound selected from the group consisting of a laxative agent selected from a bulk- producing agent (e.g. psyllium husk (Metamucil)), methylcellulose (Citrucel), polycarbophil, dietary fiber, apples, stool softeners/surfactant (e.g., docusate, Colace, Diocto), a hydrating or osmotic agent (e.g., dibasic sodium phosphate, magnesium citrate, magnesium hydroxide (Milk of magnesia), magnesium sulfate (which is Epsom salt), monobasic sodium phosphate, sodium biphosphate), a hyperosmotic agent (e.g., glycerin suppositories, sorbitol, lactulose, and polyethylene glycol (PEG)).

In another embodiment, a method for inhibiting NHE-mediated antiport of sodium and hydrogen ions is provided.

In another embodiment, a compound or pharmaceutically acceptable salt thereof for use in a method for treating a disorder associated with fluid retention or salt overload is provided.

In another embodiment, a compound or pharmaceutically acceptable salt thereof for use in a method for treating hypertension is provided.

In further embodiments, the compound or composition is to be administered to treat hypertension. In further embodiments, the compound or composition is to be administered to treat hypertension associated with dietary salt intake. In further embodiments, administration of the compound or composition allows the mammal to intake a more palatable diet. In further embodiments, the compound or composition is to be administered to treat fluid overload. In further embodiments, the fluid overload is associated with congestive heart failure. In further embodiments, the fluid overload is associated with end stage renal disease. In further embodiments, the fluid overload is associated with peroxisome proliferator-activated receptor (PPAR) gamma agonist therapy. In further embodiments, the compound or composition is to be administered to treat sodium overload. In further embodiments, the compound or composition is to be administered to reduce interdialytic weight gain in ESRD patients. In further embodiments, the compound or composition is to be administered to treat edema. In further embodiments, the edema is caused by chemotherapy, pre-menstrual fluid overload or preeclampsia.

In further embodiments, the compound or composition is to be administered orally, by rectal suppository, or enema.

In further embodiments, the method comprises administering a pharmaceutically effective amount of the compound or composition in combination with one or more additional pharmaceutically active compounds or agents. In further embodiments, the one or more additional pharmaceutically active compounds or agents is selected from the group consisting of a diuretic, cardiac glycoside, ACE inhibitor, angiotensin-2 receptor antagonist, aldosterone antagonist, aldosterone synthase inhibitor, renin inhibitor, calcium channel blocker, beta blocker, alpha blocker, central alpha agonist, vasodilator, blood thinner, anti-platelet agent, lipid-lowering agent, and peroxisome proliferator-activated receptor (PPAR) gamma agonist agent. In further embodiments, the diuretic is selected from the group consisting of a high ceiling loop diuretic, a benzothiadiazide diuretic, a potassium sparing diuretic, and a osmotic diuretic. In further embodiments, the pharmaceutically effective amount of the compound or composition, and the one or more additional pharmaceutically active compounds or agents, are administered as part of a single pharmaceutical preparation. In further embodiments, the pharmaceutically effective amount of the compound or composition, and the one or more additional pharmaceutically active compounds or agents, are administered as individual pharmaceutical preparations. In further embodiments, the individual pharmaceutical preparation are administered sequentially. In further embodiments, the individual pharmaceutical preparation are administered simultaneously.

In another embodiment, a compound or pharmaceutically acceptable salt thereof for use in a method for treating a gastrointestinal tract disorder is provided.

In further embodiments, the gastrointestinal tract disorder is a gastrointestinal motility disorder. In further embodiments, the gastrointestinal tract disorder is irritable bowel syndrome. In further embodiments, the gastrointestinal tract disorder is chronic constipation. In further embodiments, the gastrointestinal tract disorder is chronic idiopathic constipation. In further embodiments, the gastrointestinal tract disorder is chronic constipation occurring in cystic fibrosis patients. In further embodiments, the gastrointestinal tract disorder is opioid-induced constipation. In further embodiments, the gastrointestinal tract disorder is a functional gastrointestinal tract disorder. In further embodiments, the gastrointestinal tract disorder is selected from the group consisting of chronic intestinal pseudo-obstruction and colonic pseudo-obstruction. In further embodiments, the gastrointestinal tract disorder is Crohn's disease. In further embodiments, the gastrointestinal tract disorder is ulcerative colitis. In further embodiments, the gastrointestinal tract disorder is a disease referred to as inflammatory bowel disease. In further embodiments, the gastrointestinal tract disorder is associated with chronic kidney disease (stage 4 or 5). In further embodiments, the gastrointestinal tract disorder is constipation induced by calcium supplement. In further embodiments, the gastrointestinal tract disorder is constipation, and the constipation to be treated is associated with the use of a therapeutic agent. In further embodiments, the gastrointestinal tract disorder is constipation, and the constipation to be treated is associated with a neuropathic disorder. In further embodiments, the gastrointestinal tract disorder is constipation, and the constipation to be treated is post-surgical constipation (postoperative ileus). In further embodiments, the gastrointestinal tract disorder is constipation, and the constipation to be treated is idiopathic (functional constipation or slow transit constipation). In further embodiments, the gastrointestinal tract disorder is constipation, and the constipation to be treated is associated with neuropathic, metabolic or an endocrine disorder (e.g., diabetes mellitus, renal failure, hypothyroidism, hyperthyroidism, hypocalcaemia, Multiple Sclerosis, Parkinson's disease, spinal cord lesions, neurofibromatosis, autonomic neuropathy, Chagas disease, Hirschsprung's disease or cystic fibrosis, and the like). In further embodiments, the gastrointestinal tract disorder is constipation, and the constipation to be treated is due the use of drugs selected from analgesics (e.g., opioids), antihypertensives, anticonvulsants, antidepressants, antispasmodics and antipsychotics.

In another embodiment, a compound or pharmaceutically acceptable salt thereof for use in a method for treating irritable bowel syndrome is provided.

In further embodiments of the above embodiments, the compound or composition is to be administered to treat or reduce pain associated with a gastrointestinal tract disorder. In further embodiments, the compound or composition is to be administered to treat or reduce visceral hypersensitivity associated with a gastrointestinal tract disorder. In further embodiments, the compound or composition is to be administered to treat or reduce inflammation of the gastrointestinal tract. In further embodiments, the compound or composition is to be administered to reduce gastrointestinal transit time.

In further embodiments, the compound or composition is to be administered either orally or by rectal suppository.

In further embodiments, the method comprises administering a pharmaceutically effective amount of the compound or composition, in combination with one or more additional pharmaceutically active compounds or agents. In further embodiments, the one or more additional pharmaceutically active agents or compounds are an analgesic peptide or agent. In further embodiments, the one or more additional pharmaceutically active agents or compounds are selected from the group consisting of a laxative agent selected from a bulk-producing agent (e.g. psyllium husk (Metamucil)),methylcellulose (Citrucel), polycarbophil, dietary fiber, apples, stool softeners/surfactant (e.g., docusate, Colace, Diocto), a hydrating or osmotic agent (e.g., dibasic sodium phosphate, magnesium citrate, magnesium hydroxide (Milk of magnesia), magnesium sulfate (which is Epsom salt), monobasic sodium phosphate, sodium biphosphate), and a hyperosmotic agent (e.g., glycerin suppositories, sorbitol, lactulose, and polyethylene glycol (PEG)). In further embodiments, the pharmaceutically effective amount of the compound or composition, and the one or more additional pharmaceutically active compounds or agents, are administered as part of a single pharmaceutical preparation. In further embodiments, the pharmaceutically effective amount of the compound or composition, and the one or more additional pharmaceutically active compounds or agents, are administered as individual pharmaceutical preparations. In further embodiments, the individual pharmaceutical preparation are administered sequentially. In further embodiments, the individual pharmaceutical preparation are administered simultaneously.

These and other aspects of the invention will be apparent upon reference to the following detailed description.

### DETAILED DESCRIPTION

In accordance with the present disclosure, and as further detailed herein below, it has been found that the inhibition of NHE-mediated antiport of sodium ions (Na⁺) and hydrogen ions (H⁺) in the gastrointestinal tract, and more particularly the gastrointestinal epithelia, is a powerful approach to the treatment of various disorders that may be associated with or caused by fluid retention and/or salt overload, and/or disorders such as heart failure (in particular, congestive heart failure), chronic kidney disease, end-stage renal disease, liver disease, and/or peroxisome proliferator-activated receptor (PPAR) gamma agonist-induced fluid retention. More specifically, it has been found that the inhibition of the NHE-mediated antiport of sodium ions and hydrogen ions in the GI tract increases the fecal excretion of sodium, effectively reducing systemic levels of sodium and fluid. This, in turn, improves the clinical status of a patient suffering from, for example, CHF, ESRD/CKD and/or liver disease. It has further been found that such a treatment may optionally be enhanced by the co-administration of other beneficial compounds or compositions, such as for example a fluid-absorbing polymer. The fluid-absorbing polymer may optimally be chosen so that it does not block or otherwise negatively interfere with the mechanism of action of the co-dosed NHE-inhibiting compound.

Additionally, and also as further detailed herein below, it has further been found that the inhibition of NHE-mediated antiport of sodium ions (Na⁺) and hydrogen ions (H⁺) in the gastrointestinal tract, and more particularly the gastrointestinal epithelia, is a powerful approach to the treatment of hypertension, that may be associated with or caused by fluid retention and/or salt overload. More specifically, it has been found that the inhibition of the NHE-mediated antiport of sodium ions and hydrogen ions in the GI tract increases the fecal excretion of sodium, effectively reducing systemic levels of sodium and fluid. This, in turn, improves the clinical status of a patient suffering from hypertension. Such a treatment may optionally be enhanced by the co-administration of other beneficial compounds or compositions, such as for example a fluid-absorbing polymer. The fluid- absorbing polymer may optimally be chosen so that it does not block or otherwise negatively interfere with the mechanism of action of the co-dosed NHE-inhibiting compound.

Additionally, and also as further detailed herein below, it has further been found that the inhibition of NHE-mediated antiport of sodium ions (Na⁺) and hydrogen ions (H⁺) in the gastrointestinal tract, and more particularly the gastrointestinal epithelia, is a powerful approach to the treatment of various gastrointestinal tract disorders, including the treatment or reduction of pain associated with gastrointestinal tract disorders, and more particularly to the restoration of appropriate fluid secretion in the gut and the improvement of pathological conditions encountered in constipation states. Applicants have further recognized that by blocking sodium ion re-absorption, the compounds of the present disclosure restore fluid homeostasis in the GI tract, particularly in situations wherein fluid secretion/ab sorption is altered in such a way that it results in a high degree of feces dehydration, low gut motility, and/or a slow transit-time producing constipation states and GI discomfort generally. It has further been found that such a treatment may optionally be enhanced by the co-administration of other beneficial compounds or compositions, such as for example a fluid-absorbing polymer. The fluid-absorbing polymer may optimally be chosen so that it does not block or otherwise negatively interfere with the mechanism of action of the co-dosed NHE-inhibiting compound.

Due to the presence of NHEs in other organs or tissues in the body, the method of the present disclosure employs the use of compounds and compositions that are desirably highly selective or localized, thus acting substantially in the gastrointestinal tract without exposure to other tissues or organs. In this way, any systemic effects can be minimized (whether they are on-target or off-target). Accordingly, it is to be noted that, as used herein, and as further detailed elsewhere herein, "substantially active in the gastrointestinal tract" generally refers to compounds that are substantially systemically non-bioavailable and/or substantially impermeable to the layer of epithelial cells, and more specifically epithelium of the GI tract. It is to be further noted that, as used herein, and as further detailed elsewhere herein, "substantially impermeable" more particularly encompasses compounds that are impermeable to the layer of epithelial cells, and more specifically the gastrointestinal epithelium (or epithelial layer). "Gastrointestinal epithelium" refers to the membranous tissue covering the internal surface of the gastrointestinal tract. Accordingly, by being substantially impermeable, a compound has very limited ability to be transferred across the gastrointestinal epithelium, and thus contact other internal organs (e.g., the brain, heart, liver, etc.). The typical mechanism by which a compound can be transferred across the gastrointestinal epithelium is by either transcellular transit (a substance travels through the cell, mediated by either passive or active transport passing through both the apical and basolateral membranes) and/or by paracellular transit, where a substance travels between cells of an epithelium, usually through highly restrictive structures known as "tight junctions".

The compounds of the present disclosure may therefore not be absorbed, and are thus essentially not systemically bioavailable at all (e.g., impermeable to the gastrointestinal epithelium at all), or they show no detectable concentration of the compound in serum. Alternatively, the compounds may: (i) exhibit some detectable permeability to the layer of epithelial cells, and more particularly the epithelium of the GI tract, of less than about 20% of the administered compound (e.g., less than about 15%, about 10%), or even about 5%, and for example greater than about 0.5%, or 1%>), but then are rapidly cleared in the liver (i.e., hepatic extraction) via first-pass metabolism; and/or (ii) exhibit some detectable permeability to the layer of epithelial cells, and more particularly the epithelium of the GI tract, of less than about 20% of the administered compound (e.g., less than about 15%, about 10%, or even about 5%, and for example greater than about 0.5%, or 1%), but then are rapidly cleared in the kidney (i.e., renal excretion).

Compounds may also be cleared from circulation unchanged into the bile by biliary excretion. The compounds of the present disclosure may therefore not exhibit detectable concentrations in the bile. Alternatively, the compounds may exhibit some detectable concentration in the bile and more particularly the epithelium of the biliary tract and gallbladder of 10 µM, less than 1 µM, less than 0.1 µM, less than 0.01 µM or less than about 0.001 µM.

In this regard it is to be still further noted that, as used herein, "substantially systemically non-bioavailable" generally refers to the inability to detect a compound in the systemic circulation of an animal or human following an oral dose of the compound. For a compound to be bioavailable, it must be transferred across the gastrointestinal epithelium (that is, substantially permeable as defined above), be transported via the portal circulation to the liver, avoid substantial metabolism in the liver, and then be transferred into systemic circulation.

Without being held to any particular theory, the NHE-inhibiting compounds (e.g., NHE-3, -2 and/or -8 inhibitors) of the present disclosure are believed to act via a distinct and unique mechanism, causing the retention of fluid and ions in the GI tract (and stimulating fecal excretion) rather than stimulating increased secretion of said fluid and ions. For example, lubiprostone (Amitiza® Sucampo/Takeda) is a bicyclic fatty acid prostaglandin El analog that activates the Type 2 Chloride Channel (ClC-2) and increases chloride-rich fluid secretion from the serosal to the mucosal side of the GI tract (see, e.g., Pharmacological Reviews for Amitiza®, DA package). Linaclotide (MD-1100 acetate, Microbia/Forest Labs) is a 14 amino acid peptide analogue of an endogenous hormone, guanylin, and indirectly activates the Cystic Fibrosis Transmembrane Conductance Regulator (CFTR) thereby inducing fluid and electrolyte secretion into the GI (see, e.g., Li et al., J. Exp. Med., vol. 202 (2005), pp. 975-986). The substantially impermeable NHE-inhibiting compounds of the present disclosure act to inhibit the reuptake of salt and fluid rather than promote secretion. Since the GI tract processes about 9 liters of fluid and about 800 meq of Na each day, it is anticipated that NHE inhibition could permit the removal of substantial quantities of systemic fluid and sodium to resorb edema and resolve CHF symptoms.

### I. Substantially Impermeable or Substantially Systemically Non-Bioavailable NHE-Inhibiting Compounds

In one embodiment, the compounds of the present disclosure may be generally represented by Formula (I):

Core(̵L-NHE)ₙ (I)

or a pharmaceutically acceptable salt thereof, wherein: (i) NHE represents a NHE-inhibiting small molecule moiety as set forth below, (ii) n is 2 or 3, (iii) Core is a Core moiety having two or three sites thereon for attachment to two or three NHE-inhibiting small molecule moieties, and (iv) L is a bond or linker connecting the Core moiety to the two or three NHE-inhibitory small molecule moieties, the resulting NHE-inhibiting compound (i.e., a compound of Formula (I)) possessing overall physicochemical properties that render it substantially impermeable or substantially systemically non-bioavailable. The Core moiety may be bound to essentially any position on, or within, the NHE-inhibiting small molecule moiety, provided that the installation thereof does not significantly adversely impact NHE-inhibiting activity.

It is to be noted that, in the many structures illustrated herein, all of the various linkages or bonds will not be shown in every instance. For example, in one or more of the structures illustrated above, a bond or connection between the NHE-inhibiting small molecule moiety and the Core moiety is not always shown. However, this should not be viewed in a limiting sense. Rather, it is to be understood that the NHE-inhibiting small molecule moiety is bound or connected in some way (e.g., by a bond or linker of some kind) to the Core moiety, such that the resulting NHE-inhibiting compound is suitable for use (i.e., substantially impermeable or substantially systemically non- bioavailable in the GI tract).

NHE-inhibiting small molecule moieties suitable for use (i.e., suitable for modification or functionalization in accordance with the present disclosure) in the prepartion of the substantially impermeable or substantially systemically nonbioavailable NHE-inhibiting compounds of the present disclosure are disclosed in WO 2010/025856.

The NHE-inhibiting small molecule moiety has the following structure of Formula (XI): 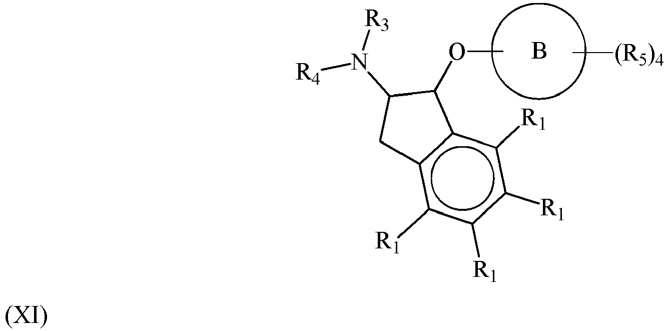 wherein:
B is aryl;
each R₅ is independently selected from the group consisting of hydrogen, halogen, optionally substituted C₁₋₄alkyl, optionally substituted C₁₋₄alkoxy, optionally substituted C₁₋₄thioalkyl, optionally substituted heterocyclyl, optionally substituted heterocyclylalkyl, optionally substituted aryl, optionally substituted heteroaryl, hydroxyl, oxo, cyano, nitro, -NR₇R₈, -NR₇C(=O)R₈, -NR₇C(=O)OR₈, -NR₇C(=O)NR₈R₉, -NR₇SO₂R₈, -NR₇S(O)₂NR₈R₉, -C(=O)OR₇, -C(=O)R₇, -C(=O)NR₇R₈, -S(O)₁₋₂R₇, and -SO₂NR₇R₈, wherein R₇, R₈, and R₉ are independently selected from the group consisting of hydrogen, C₁₋₄alkyl or a bond linking the NHE-inhibiting small molecule moiety to L, provided at least one is a bond linking the NHE-inhibiting small molecule moiety to L;
R₃ and R₄ are independently selected from the group consisting of hydrogen, optionally substituted C₁₋₄alkyl, optionally substituted cycloalkyl, optionally substituted cycloalkylalkyl, optionally substituted aryl, optionally substituted aralkyl, optionally substituted heterocyclyl and optionally substituted heteroaryl; or
R₃ and R₄ form together with the nitrogen to which they are bonded an optionally substituted 4-8 membered heterocyclyl; and
each R₁ is independently selected from the group consisting of hydrogen, halogen, optionally substituted C₁₋₆alkyl and optionally substituted C₁₋₆alkoxy.

In yet further more specific embodiments, the NHE-inhibiting small molecule moiety has the following structure of Formula (XII): 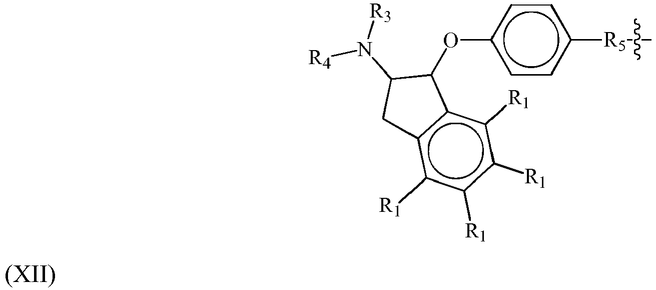 wherein:
each R₃ and R₄ are independently selected from the group consisting of hydrogen and optionally substituted C₁₋₄alkyl, or R₃ and R₄, taken together with the nitrogen to which they are bonded, form an optionally substituted 4-8 membered heterocyclyl;
each R₁ is independently selected from the group consisting of hydrogen, halogen, C₁₋₆alkyl, and C₁₋₆haloalkyl; and
R5 is selected from the group consisting of -SO₂-NR₇- and NHC(=O)NH-, wherein R₇ is hydrogen or C₁₋₄alkyl.

In the foregoing polyvalent embodiments, L may be a polyalkylene glycol linker, such as a polyethylene glycol linker; and/or the Core may have the following structure: 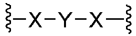 wherein: X is selected from the group consisting of a bond, -O-, -NH-, -S-, C₁₋₆alkylene, -NHC(=O)-, -C(=O)NH-, -NHC(=O)NH-, -SO₂NH-, and -NHSO₂-; Y is selected from the group consisting of a bond, optionally substituted C₁₋₈alkylene, optionally substituted aryl, optionally substituted heteroaryl, a polyethylene glycol linker, -(CH₂)₁₋₆O(CH₂)₁₋₆- and -(CH₂)₁₋₆NY₁(CH₂)₁₋₆-; and Y₁ is selected from the group consisting of hydrogen, optionally substituted C₁₋₈alkyl, optionally substituted aryl or optionally substituted heteroaryl. For example, in more specific embodiments, the Core may be selected, for example, from the group consisting of: 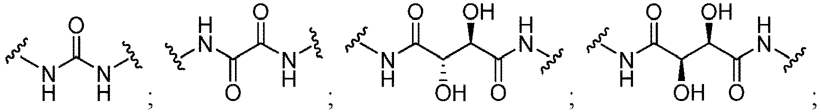 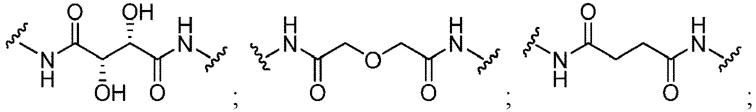 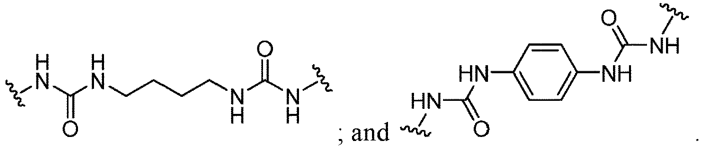 In other more specific embodiments, the Core may be selected, for example, from the group consisting of:

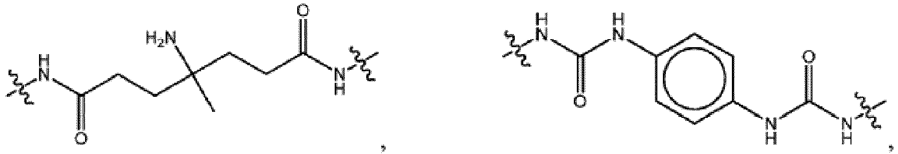

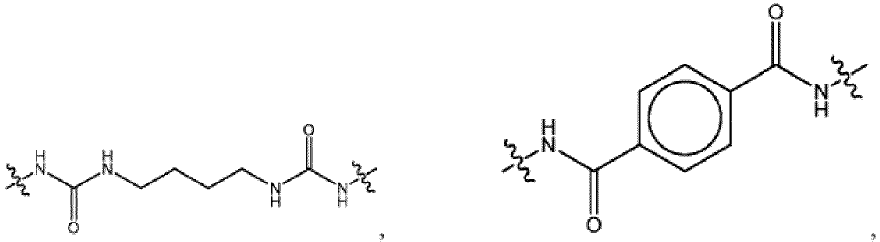

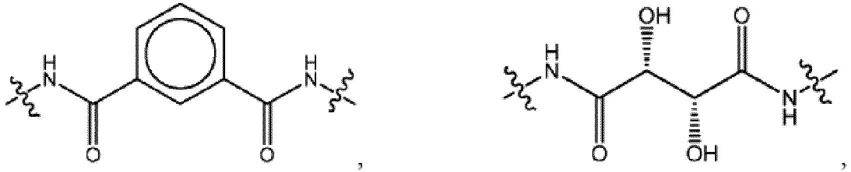

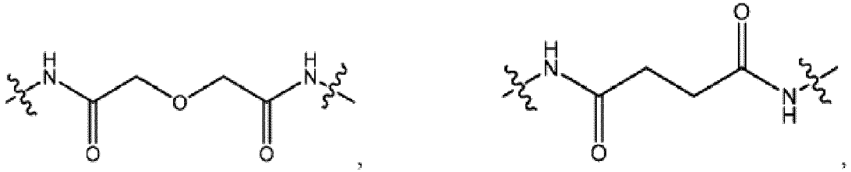

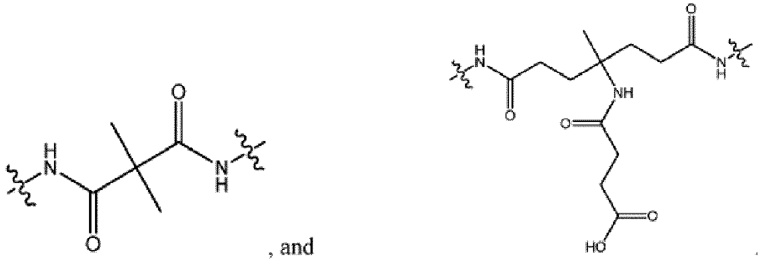

The above noted embodiments are further illustrated herein below. For example, the first representation below of an exemplary oligomer compound, wherein the various parts of the compound are identified, is intended to provide a broad context for the disclosure provided herein. It is to be noted that while each NHE-inhibiting small molecule moiety in the structure below is the same, it is within the scope of this disclosure that each is independently selected and may be the same or different. In the illustration below, the linker moiety is a polyethylene glycol (PEG) motif. PEG derivatives are advantageous due in part to their aqueous solubility, which may help avoid hydrophobic collapse (the intramolecular interaction of hydrophobic motifs that can occur when a hydrophobic molecule is exposed to an aqueous environment (*see*, e.g., Wiley, R. A.; Rich, D. H. Medical Research Reviews 1993, 13(3), 327-384). The core moiety illustrated below is also advantageous because it provides some rigidity to the molecule, allowing an increase in distance between the NHE-inhibiting small molecule moieties while minimally increasing rotational degrees of freedom.

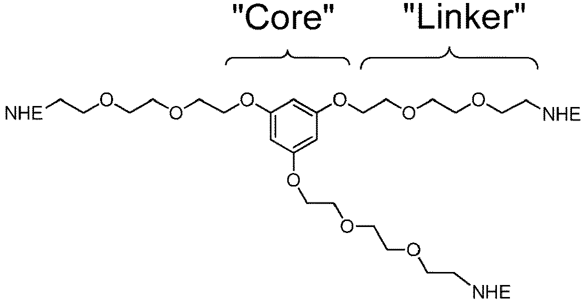

In an alternative embodiment, wherein m = 0, the structure may be, for example: 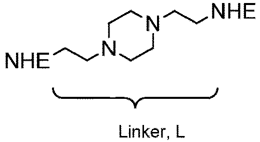 or 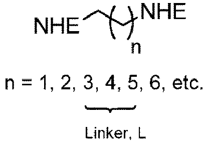 or

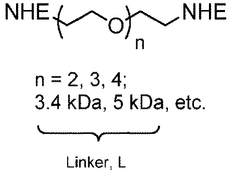

Within the polyvalent compounds utilized for treatments according to the present disclosure, n and m (when m is not zero) may be independently selected from the range of from about 1 to about 10, more preferably from about 1 to about 5, and even more preferably from about 1 to about 2. In alternative embodiments, however, n and m may be independently selected from the range of from about 1 to about 500, preferably from about 1 to about 300, more preferably from about 1 to about 100, and most preferably from about 1 to about 50. In these or other particular embodiments, E, n and m may be within the range of from about 1 to about 50, or from about 1 to about 20.

In designing and making the substantially impermeable or substantially systemically non-bioavailable NHE-inhibiting compounds that may be utilized for the treatments detailed in the instant disclosure, it may in some cases be advantageous to first determine a likely point of attachment on a NHE-inhibiting small molecule moiety, where a core or linker might be installed or attached before making a series of candidate multivalent or polyvalent compounds. This may be done by one skilled in the art via known methods by systematically installing functional groups, or functional groups displaying a fragment of the desired core or linker, onto various positions of the NHE-inhibiting small molecule moiety and then testing these adducts to determine whether the modified compound still retains desired biological properties (e.g., NHE-inhibiting activity). An understanding of the SAR of the compound also allows the design of cores and/or linkers that contribute positively to the activity of the resulting compounds.

Another aspect to be considered in the design of cores and linkers is the limiting or preventing of hydrophobic collapse. Compounds with extended hydrocarbon functionalities may collapse upon themselves in an intramolecular fashion, causing an increased enthalpic barrier for interaction with the desired biological target. Accordingly, when designing cores and linkers, these are preferably designed to be resistant to hydrophobic collapse. For example, conformational constraints such as rigid monocyclic, bicyclic or polycyclic rings can be installed in a core or linker to increase the rigidity of the structure. Unsaturated bonds, such as alkenes and alkynes, may also or alternatively be installed. Such modifications may ensure the NHE-inhibiting compound is accessible for productive binding with its target. Furthermore, the hydrophilicity of the linkers may be improved by adding hydrogen bond donor or acceptor motifs, or ionic motifs such as amines that are protonated in the GI, or acids that are deprotonated. Such modifications will increase the hydrophilicity of the core or linker and help prevent hydrophobic collapse. Furthermore, such modifications will also contribute to the impermeability of the resulting compounds by increasing tPSA.

One skilled in the art may consider a variety of functional groups that will allow the facile and specific attachment of a NHE-inhibiting small molecule moiety to a core or linker. These functional groups can include electrophiles, which can react with nucleophilic cores or linkers, and nucleophiles, which can react with electrophilic cores or linkers. NHE-inhibiting small molecule moieties may be similarly derivatized with, for example, boronic acid groups which can then react with appropriate cores or linkers via palladium mediated cross-coupling reactions. The NHE-inhibiting small molecule moiety may also contain olefins which can then react with appropriate cores or linkers via olefin metathesis chemistry, or alkynes or azides which can then react with appropriate cores or linkers via [2 + 3] cycloaddition.

It is to be noted that one skilled in the art can envision a number of core or linker moieties that may be functionalized with an appropriate electrophile or nucleophile. Shown below are a series of such compounds selected based on several design considerations, including solubility, steric effects, and their ability to confer, or be consistent with, favorable structure-activity relationships.

Exemplary electrophilic and nucleophilic linker moieties include the linker moieties illustrated in the Examples and the following:

### Nucleophilic linkers (for use with electrophilic NHEs)

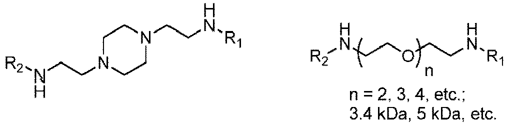

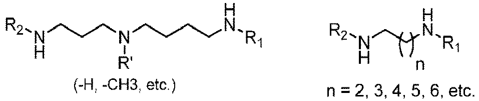

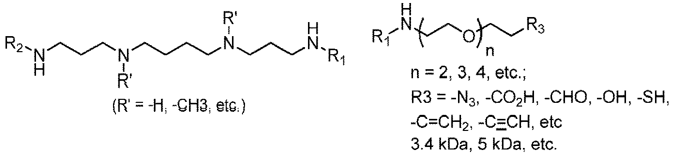

### Electrophilic linkers (for use with nucleophilic NHEs)

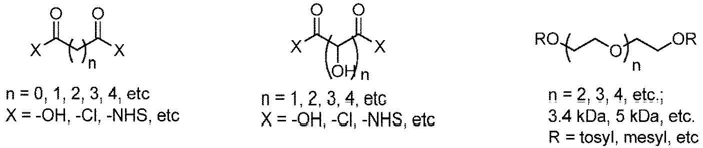

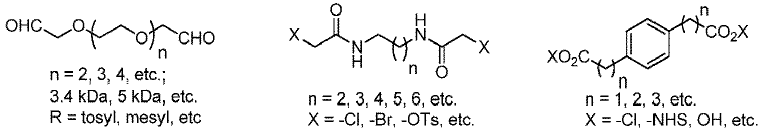

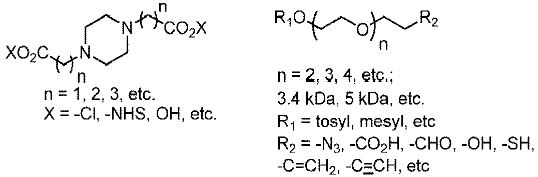

The linking moiety, L, in each of the described embodiments (including embodiments in which a NHE-inhibiting small molecule moiety is linked to a Core such as an atom, another small molecule, a polymer moiety, an oligomer moiety, or a nonrepeating moiety) can be a chemical linker, such as a bond or other moiety, for example, comprising about 1 to about 200 atoms, or about 1 to about 100 atoms, or about 1 to about 50 atoms, that can be hydrophilic and/or hydrophobic. In one embodiment, the linking moiety can be a polymer moiety grafted onto a polymer backbone, for example, using living free radical polymerization approaches known in the art. Preferred L structures or moieties may also be selected from, for example, oligoethylene glycol, oligopeptide, oligoethyleneimine, oligotetramethylene glycol and oligocaprolactone.

As noted, the core moiety can be an atom, a small molecule, an oligomer, a dendrimer or a polymer moiety, in each case having one or more sites of attachment for L. For example, the core moiety can be a non-repeating moiety (considered as a whole including linking points to the NHE-inhibiting small molecule moieties), selected for example from the group consisting of alkyl, phenyl, aryl, alkenyl, alkynyl, heterocyclic, amine, ether, sulfide, disulfide, hydrazine, and any of the foregoing substituted with oxygen, sulfur, sulfonyl, phosphonyl, hydroxyl, alkoxyl, amine, thiol, ether, carbonyl, carboxyl, ester, amide, alkyl, alkenyl, alkynyl, aryl, heterocyclic, and moieties comprising combinations thereof (in each permutation). A non-repeating moiety can include repeating units (e.g., methylene) within portions or segments thereof (e.g., within an alkyl segment), without having discrete repeat units that constitute the moiety as a whole (e.g., in the sense of a polymer or oligomer).

Exemplary core moieties include the core moieties illustrated in the Examples and ether moieties, ester moieties, sulfide moieties, disulfide moieties, amine moieties, aryl moieties, alkoxyl moieties, etc., such as, for example, the following: 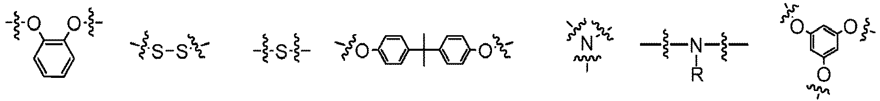 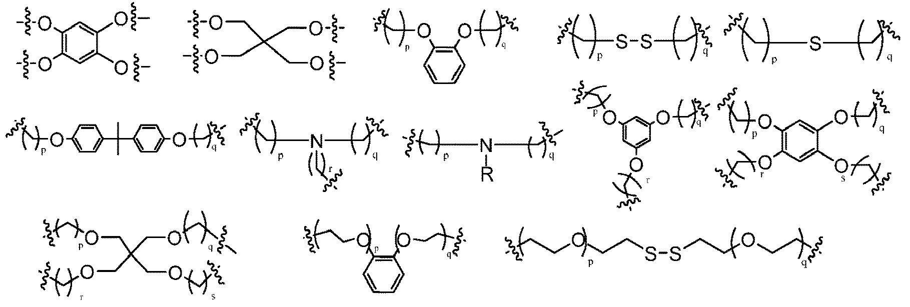 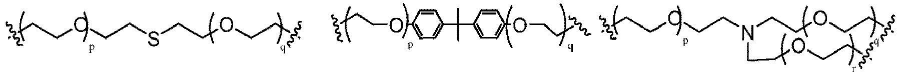 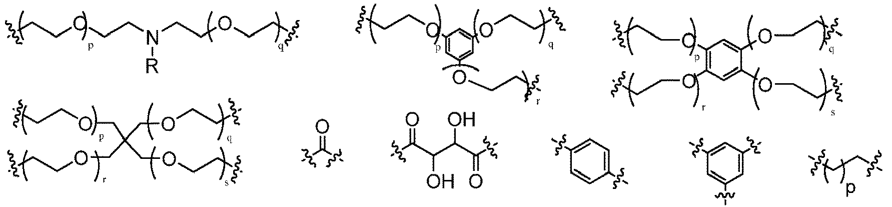 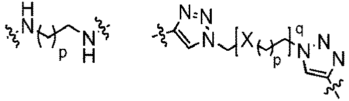 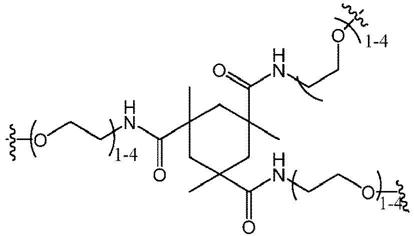 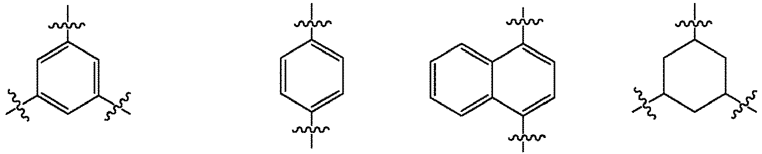 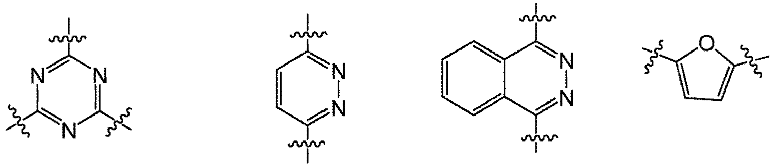 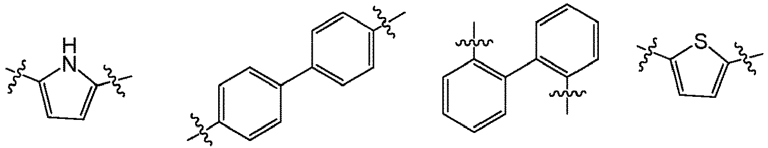 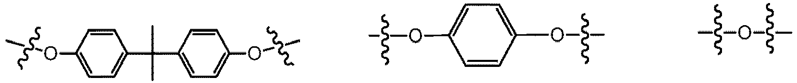 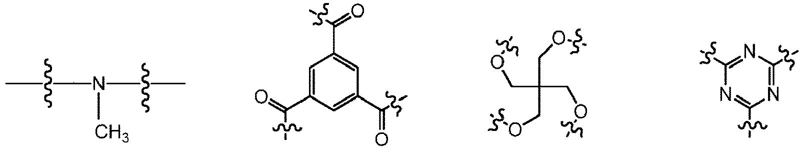 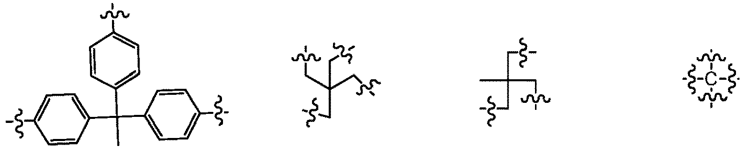 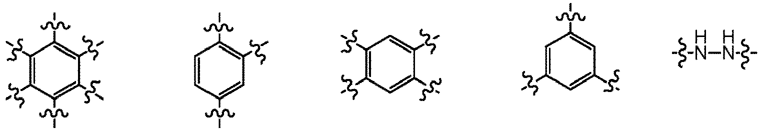 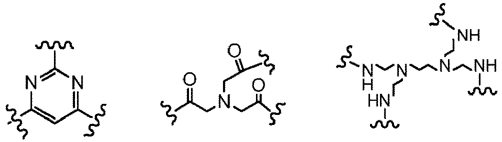 wherein the broken bonds (i.e., those having a wavy bond, 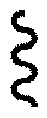 , through them) are points of connection to either a NHE-inhibiting small molecule moiety or a linker moiety displaying a NHE-inhibiting small molecule moiety, where said points of connection can be made using chemistries and functional groups known to the art of medicinal chemistry; and further wherein each p, q, r and s is an independently selected integer ranging from about 0 to about 48, preferably from about 0 to about 36, or from about 0 to about 24, or from about 0 to about 16. In some instances, each p, q, r and s can be an independently selected integer ranging from about 0 to 12. Additionally, R can be a substituent moiety generally selected from halide, hydroxyl, amine, thiol, ether, carbonyl, carboxyl, ester, amide, carbocyclic, heterocyclic, and moieties comprising combinations thereof.

In yet another approach, the core moiety may be a polymer moiety or an oligomer moiety. The polymer or oligomer may, in each case, be independently considered and comprise repeat units consisting of a repeat moiety selected from alkyl (e.g., -CH₂-), substituted alkyl (e.g., -CHR- , wherein, for example, R is hydroxy), alkenyl, substituted alkenyl, alkynyl, substituted alkynyl, phenyl, aryl, heterocyclic, amine, ether, sulfide, disulfide, hydrazine, and any of the foregoing substituted with oxygen, sulfur, sulfonyl, phosphonyl, hydroxyl, alkoxyl, amine, thiol, ether, carbonyl, carboxyl, ester, amide, alkyl, alkenyl, alkynyl, aryl, heterocyclic, as well as moieties comprising combinations thereof. In still another approach, the core moiety comprises repeat units resulting from the polymerization of ethylenic monomers (e.g., such as those ethylenic monomers listed elsewhere herein below).

Preferred polymers for polymeric moieties useful in constructing substantially impermeable or substantially systemically non-bio available NHE-inhibiting compounds that are multivalent, for use in the treatment various treatment methods disclosed herein, can be prepared by any suitable technique, such as by free radical polymerization, condensation polymerization, addition polymerization, ring-opening polymerization, and/or can be derived from naturally occurring polymers, such as saccharide polymers. Further, in some embodiments, any of these polymer moieties may be functionalized.

Examples of polysaccharides useful in preparation of such compounds include but are not limited to materials from vegetable or animal origin, including cellulose materials, hemicellulose, alkyl cellulose, hydroxyalkyl cellulose, carboxymethylcellulose, sulfoethylcellulose, starch, xylan, amylopectine, chondroitin, hyarulonate, heparin, guar, xanthan, mannan, galactomannan, chitin, and/or chitosan. More preferred, in at least some instances, are polymer moieties that do not degrade, or that do not degrade significantly, under the physiological conditions of the GI tract (such as, for example, carboxymethylcellulose, chitosan, and sulfoethylcellulose).

When free radical polymerization is used, the polymer moiety can be prepared from various classes of monomers including, for example, acrylic, methacrylic, styrenic, vinylic, and dienic, whose typical examples are given thereafter: styrene, substituted styrene, alkyl acrylate, substituted alkyl acrylate, alkyl methacrylate, substituted alkyl methacrylate, acrylonitrile, methacrylonitrile, acrylamide, methacrylamide, N-alkylacrylamide, N-alkylmethacrylamide, N,N-dialkylacrylamide, N,N-dialkylmethacrylamide, isoprene, butadiene, ethylene, vinyl acetate, and combinations thereof. Functionalized versions of these monomers may also be used and any of these monomers may be used with other monomers as comonomers. For example, specific monomers or comonomers that may be used in this disclosure include methyl methacrylate, ethyl methacrylate, propyl methacrylate (all isomers), butyl methacrylate (all isomers), 2-ethylhexyl methacrylate, isobromyl methacrylate, methacrylic acid, benzyl methacrylate, phenyl methacrylate, methacrylonitrile, a-methyl styrene, methyl acrylate, ethyl acrylate, propyl acrylate (all isomers), butyl acrylate (all isomers), 2-ethylhexyl acrylate, isobromyl acrylate, acrylic acid, benzyl acrylate, phenyl acrylate, acrylonitrile, styrene, glycidyl methacrylate, 2-hydroxyethyl methacrylate, hydroxypropyl methacrylate (all isomers), hydroxybutyl methacrylate (all isomers), N,N-dimethylaminoethyl methacrylate, N,N-diethylaminoethyl methacrylate, triethyleneglycol methacrylate, itaconic anhydride, itaconic acid, glycidyl acrylate, 2-hydroxyethyl acrylate, hydroxypropyl acrylate (all isomers), hydroxybutyl acrylate (all isomers), N,N-dimethylaminoethyl acrylate, N,N-diethylaminoethyl acrylate, triethyleneglycol acrylate, methacryl amide, N-methylacrylamide, N,N-dimethylacrylamide, N-tert-butylmethacrylamide, N-n-butylmethacrylamide, N-methylolmethacrylamide, N-ethylolmethacrylamide, N-tert-butyl acrylamide, N-N-butyl acrylamide, N- methylol acrylamide, N-ethylol acrylamide, 4-acryloylmorpholine, vinyl benzoic acid (all isomers), diethylaminostyrene (all isomers), a-methylvinyl benzoic acid (all isomers), diethylamino α-methylstyrene (all isomers), p-vinylbenzene sulfonic acid, p-vinylbenzene sulfonic sodium salt, alkoxy and alkyl silane functional monomers, maleic anhydride, N- phenylmaleimide, N-butylmaleimide, butadiene, isoprene, chloroprene, ethylene, vinyl acetate, vinylformamide, allylamine, vinylpyridines (all isomers), fluorinated acrylate, methacrylates, and combinations thereof. Main chain heteroatom polymer moieties can also be used, including polyethyleneimine and polyethers such as polyethylene oxide and polypropylene oxide, as well as copolymers thereof.

In one particular embodiment, the polymer to which the NHE-inhibiting small molecule moiety is attached, or otherwise a part of, is a polyol (e.g., a polymer having a repeat unit of, for example, a hydroxyl-substituted alkyl, such as -CH(OH)-). Polyols, such as mono- and disaccharides, with or without reducing or reducible end groups thereon, may be good candidates, for example, for installing additional functionality that could render the compound substantially impermeable.

In one particular embodiment, the NHE-inhibiting small molecule moiety is attached at one or both ends of the polymer chain. More specifically, in yet another alternative approach to the polyvalent embodiment of the present disclosure, a macromolecule (e.g., a polymer or oligomer) having one of the following exemplary structures (wherein is a NHE-inhibiting small molecule moiety) may be designed and constructed as described herein:

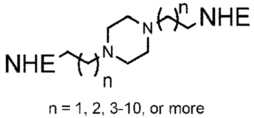

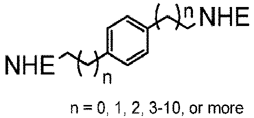

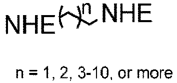

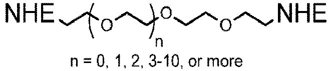

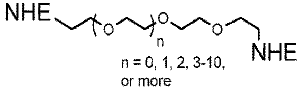

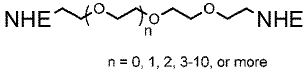

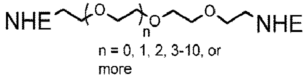

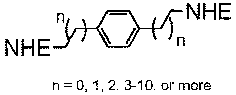

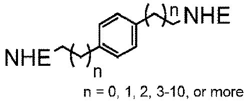

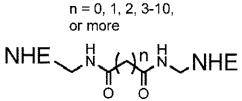

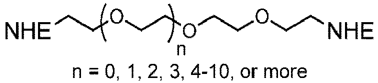

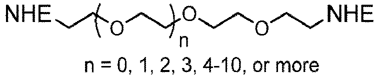

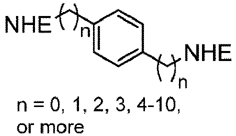

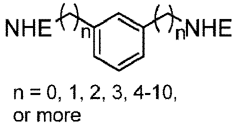

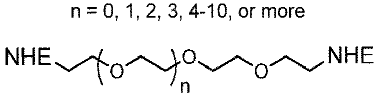

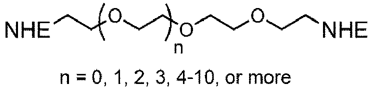

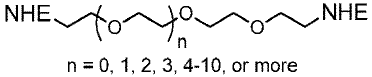

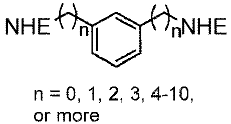

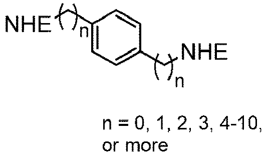

It is understood that any embodiment of the compounds of the present invention, as set forth above, and any specific substituent set forth herein in such compounds, as set forth above, may be independently combined with other embodiments and/or substituents of such compounds to form embodiments of the inventions not specifically set forth above. In addition, in the event that a list of substituents is listed for any particular substituent in a particular embodiment and/or claim, it is understood that each individual substituent may be deleted from the particular embodiment and/or claim and that the remaining list of substituents will be considered to be within the scope of the invention. Furthermore, it is understood that in the present description, combinations of substituents and/or variables of the depicted formulae are permissible only if such contributions result in stable compounds.

### II. Terminology, Physical and Performance Properties

### A. Terminology

Unless the context requires otherwise, throughout the present specification and claims, the word "comprise" and variations thereof, such as, "comprises" and "comprising" are to be construed in an open, inclusive sense, that is as "including, but not limited to".

Reference throughout this specification to "one embodiment" or "an embodiment" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

"Amino" refers to the -NH₂ radical.

"Cyano" refers to the -CN radical.

"Hydroxy" or "hydroxyl" refers to the -OH radical.

"Imino" refers to the = H substituent.

"Nitro" refers to the -NO₂ radical.

"Oxo" refers to the =O substituent.

"Thioxo" refers to the =S substituent.

"Alkyl" refers to a straight or branched hydrocarbon chain radical consisting solely of carbon and hydrogen atoms, which is saturated or unsaturated (i.e., contains one or more double and/or triple bonds), having from one to twelve carbon atoms (C₁-C₁₂ alkyl), preferably one to eight carbon atoms (C₁-C₈ alkyl) or one to six carbon atoms (C₁-C₆ alkyl), and which is attached to the rest of the molecule by a single bond, e.g., methyl, ethyl, *n-*propyl, 1-methylethyl (*iso*-propyl), *n*-butyl, *n*-pentyl, 1,1-dimethylethyl (t-butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, hexynyl, and the like. Unless stated otherwise specifically in the specification, an alkyl group may be optionally substituted.

"Alkylene" or "alkylene chain" refers to a straight or branched divalent hydrocarbon chain linking the rest of the molecule to a radical group, consisting solely of carbon and hydrogen, which is saturated or unsaturated (i.e., contains one or more double and/or triple bonds), and having from one to twelve carbon atoms, e.g., methylene, ethylene, propylene, *n*-butylene, ethenylene, propenylene, *n*-butenylene, propynylene, *n*-butynylene, and the like. The alkylene chain is attached to the rest of the molecule through a single or double bond and to the radical group through a single or double bond. The points of attachment of the alkylene chain to the rest of the molecule and to the radical group can be through one carbon or any two carbons within the chain. Unless stated otherwise specifically in the specification, an alkylene chain may be optionally substituted.

"Alkoxy" refers to a radical of the formula -ORₐ where Rₐ is an alkyl radical as defined above containing one to twelve carbon atoms. Unless stated otherwise specifically in the specification, an alkoxy group may be optionally substituted.

"Alkylamino" refers to a radical of the formula -NHRₐ or -NRₐRₐ where each Rₐ is, independently, an alkyl radical as defined above containing one to twelve carbon atoms. Unless stated otherwise specifically in the specification, an alkylamino group may be optionally substituted.

"Thioalkyl" refers to a radical of the formula -SRₐ where Rₐ is an alkyl radical as defined above containing one to twelve carbon atoms. Unless stated otherwise specifically in the specification, a thioalkyl group may be optionally substituted.

"Aryl" refers to a hydrocarbon ring system radical comprising hydrogen, 6 to 18 carbon atoms and at least one aromatic ring. For purposes of this invention, the aryl radical may be a monocyclic, bicyclic, tricyclic or tetracyclic ring system, which may include fused or bridged ring systems. Aryl radicals include, but are not limited to, aryl radicals derived from aceanthrylene, acenaphthylene, acephenanthrylene, anthracene, azulene, benzene, chrysene, fluoranthene, fluorene, *as*-indacene, *s*-indacene, indane, indene, naphthalene, phenalene, phenanthrene, pleiadene, pyrene, and triphenylene. Unless stated otherwise specifically in the specification, the term "aryl" or the prefix "ar-" (such as in "aralkyl") is meant to include aryl radicals that are optionally substituted.

"Aralkyl" refers to a radical of the formula -R_{b}-R_{c} where R_{b} is an alkylene chain as defined above and R_{c} is one or more aryl radicals as defined above, for example, benzyl, diphenylmethyl and the like. Unless stated otherwise specifically in the specification, an aralkyl group may be optionally substituted.

"Cycloalkyl" or "carbocyclic ring" refers to a stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, 7,7-dimethyl-bicyclo[2.2.1]heptanyl, and the like. Unless otherwise stated specifically in the specification, a cycloalkyl group may be optionally substituted.

"Cycloalkylalkyl" refers to a radical of the formula -R_{b}R_{d} where R_{d} is an alkylene chain as defined above and R_{g} is a cycloalkyl radical as defined above. Unless stated otherwise specifically in the specification, a cycloalkylalkyl group may be optionally substituted.

"Fused" refers to any ring structure described herein which is fused to an existing ring structure in the compounds of the invention. When the fused ring is a heterocyclyl ring or a heteroaryl ring, any carbon atom on the existing ring structure which becomes part of the fused heterocyclyl ring or the fused heteroaryl ring may be replaced with a nitrogen atom.

"Halo" or "halogen" refers to bromo, chloro, fluoro or iodo.

"Haloalkyl" refers to an alkyl radical, as defined above, that is substituted by one or more halo radicals, as defined above, e.g., trifluoromethyl, difluoromethyl, trichloromethyl, 2,2,2-trifluoroethyl, 1,2-difluoroethyl, 3-bromo-2-fluoropropyl, 1,2-dibromoethyl, and the like. Unless stated otherwise specifically in the specification, a haloalkyl group may be optionally substituted.

"Heterocyclyl" or "heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, a heterocyclyl group may be optionally substituted.

"*N*-heterocyclyl" refers to a heterocyclyl radical as defined above containing at least one nitrogen and where the point of attachment of the heterocyclyl radical to the rest of the molecule is through a nitrogen atom in the heterocyclyl radical. Unless stated otherwise specifically in the specification, a *N*-heterocyclyl group may be optionally substituted.

"Heterocyclylalkyl" refers to a radical of the formula -R_{b}Rₑ where R_{b} is an alkylene chain as defined above and Rₑ is a heterocyclyl radical as defined above, and if the heterocyclyl is a nitrogen-containing heterocyclyl, the heterocyclyl may be attached to the alkyl radical at the nitrogen atom. Unless stated otherwise specifically in the specification, a heterocyclylalkyl group may be optionally substituted.

"Heteroaryl" refers to a 5- to 14-membered ring system radical comprising hydrogen atoms, one to thirteen carbon atoms, one to six heteroatoms selected from the group consisting of nitrogen, oxygen and sulfur, and at least one aromatic ring. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; the nitrogen atom may be optionally quaternized. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[£][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl- 1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e., thienyl). Unless stated otherwise specifically in the specification, a heteroaryl group may be optionally substituted.

"*N*-heteroaryl" refers to a heteroaryl radical as defined above containing at least one nitrogen and where the point of attachment of the heteroaryl radical to the rest of the molecule is through a nitrogen atom in the heteroaryl radical. Unless stated otherwise specifically in the specification, an *N*-heteroaryl group may be optionally substituted.

"Heteroarylalkyl" refers to a radical of the formula R_{b}R_{f} where R_{b} is an alkylene chain as defined above and R_{f} is a heteroaryl radical as defined above. Unless stated otherwise specifically in the specification, a heteroarylalkyl group may be optionally substituted.

The term "substituted" used herein means any of the above groups (i.e., alkyl, alkylene, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl) wherein at least one hydrogen atom is replaced by a bond to a non- hydrogen atoms such as, but not limited to: a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (e.g., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with -NR_{g}Rₕ, -NR_{g}C(=O)Rₕ, -NR_{g}C(=O)NR_{g}Rₕ, -NR_{g}C(=O)ORₕ, NR_{g}SO₂Rₕ,-OC(=O)NR_{g}Rₕ, -OR_{g}, -SR_{g}, -SOR_{g}, -SO₂R_{g}, -OSO₂R_{g}, -SO₂OR_{g}, =NSO₂R_{g}, and -SO₂R_{g}Rₕ. "Substituted" also means any of the above groups in which one or more hydrogen atoms are replaced with -C(=O)R_{g}, -C(=O)OR_{g}, -C(=O)NR_{g}Rₕ,-CH₂SO₂R_{g}, -CH₂SO₂R_{g}Rₕ, -(CH₂CH₂O)₂₋₁₀R_{g}. In the foregoing, R_{g} and Rₕ are the same or different and independently hydrogen, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl. "Substituted" further means any of the above groups in which one or more hydrogen atoms are replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, *N*-heteroaryl and/or heteroarylalkyl group. In addition, each of the foregoing substituents may also be optionally substituted with one or more of the above substituents.

"Prodrug" is meant to indicate a compound that may be converted under physiological conditions or by solvolysis to a biologically active compound. Thus, the term "prodrug" refers to a metabolic precursor of a compound that is pharmaceutically acceptable. A prodrug may be inactive when administered to a subject in need thereof, but is converted *in vivo* to an active compound. Prodrugs are typically rapidly transformed *in vivo* to yield the parent compound, for example, by hydrolysis in blood. The prodrug compound often offers advantages of solubility, tissue compatibility or delayed release in a mammalian organism (see, Bundgard, H., Design of Prodrugs (1985), pp. 7-9, 21-24 (Elsevier, Amsterdam)). A discussion of prodrugs is provided in Higuchi, T., et al., A.C.S. Symposium Series, Vol. 14, and in Bioreversible Carriers in Drug Design, Ed. Edward B. Roche, American Pharmaceutical Association and Pergamon Press, 1987.

The term "prodrug" is also meant to include any covalently bonded carriers, which release the active compound *in vivo* when such prodrug is administered to a mammalian subject. Prodrugs of a compound may be prepared by modifying functional groups present in the compound in such a way that the modifications are cleaved, either in routine manipulation or *in vivo,* to the parent compound. Prodrugs include compounds wherein a hydroxy, amino or mercapto group is bonded to any group that, when the prodrug of the compound is administered to a mammalian subject, cleaves to form a free hydroxy, free amino or free mercapto group, respectively. Examples of prodrugs include, but are not limited to, acetate, formate and benzoate derivatives of alcohol or amide derivatives of amine functional groups in the compounds and the like.

The invention disclosed herein is also meant to encompass the in vivo metabolic products of the disclosed compounds. Such products may result from, for example, the oxidation, reduction, hydrolysis, amidation, esterification, and the like of the administered compound, primarily due to enzymatic processes. Accordingly, the invention includes compounds produced by a process comprising administering a compound of this invention to a mammal for a period of time sufficient to yield a metabolic product thereof. Such products are typically identified by administering a radio labelled compound of the invention in a detectable dose to an animal, such as rat, mouse, guinea pig, monkey, or to human, allowing sufficient time for metabolism to occur, and isolating its conversion products from the urine, blood or other biological samples.

"Stable compound" and "stable structure" are meant to indicate a compound that is sufficiently robust to survive isolation to a useful degree of purity from a reaction mixture, and formulation into an efficacious therapeutic agent.

"Optional" or "optionally" means that the subsequently described event or circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor- 10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p*-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like. Particularly preferred organic bases are isopropylamine, diethylamine, ethanolamine, trimethylamine, dicyclohexylamine, choline and caffeine.

Often crystallizations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate and the like, as well as the corresponding solvated forms. The compound of the invention may be true solvates, while in other cases, the compound of the invention may merely retain adventitious water or be a mixture of water plus some adventitious solvent.

A "pharmaceutical composition" refers to a formulation of a compound of the invention and a medium generally accepted in the art for the delivery of the biologically active compound to mammals, e.g., humans. Such a medium includes all pharmaceutically acceptable carriers, diluents or excipients therefor.

The compounds of the invention, or their pharmaceutically acceptable salts may contain one or more asymmetric centers and may thus give rise to enantiomers, diastereomers, and other stereoisomeric forms that may be defined, in terms of absolute stereochemistry, as (*R*)- or (*S*)- or, as (D)- or (L)- for amino acids. The present invention is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centres of geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are nonsuperimposeable mirror images of one another.

A "tautomer" refers to a proton shift from one atom of a molecule to another atom of the same molecule. The present invention includes tautomers of any said compounds.

In accordance with the present disclosure, the compounds described herein are designed to be substantially active or localized in the gastrointestinal lumen of a human or animal subject. The term "gastrointestinal lumen" is used interchangeably herein with the term "lumen," to refer to the space or cavity within a gastrointestinal tract (GI tract, which can also be referred to as the gut), delimited by the apical membrane of GI epithelial cells of the subject. In some embodiments, the compounds are not absorbed through the layer of epithelial cells of the GI tract (also known as the GI epithelium). "Gastrointestinal mucosa" refers to the layer(s) of cells separating the gastrointestinal lumen from the rest of the body and includes gastric and intestinal mucosa, such as the mucosa of the small intestine. A "gastrointestinal epithelial cell" or a "gut epithelial cell" as used herein refers to any epithelial cell on the surface of the gastrointestinal mucosa that faces the lumen of the gastrointestinal tract, including, for example, an epithelial cell of the stomach, an intestinal epithelial cell, a colonic epithelial cell, and the like.

"Substantially systemically non-bioavailable" and/or "substantially impermeable" as used herein (as well as variations thereof) generally refer to situations in which a statistically significant amount, and in some embodiments essentially all of the compound of the present disclosure (which includes the NHE-inhibitor small molecule), remains in the gastrointestinal lumen. For example, in accordance with one or more embodiments of the present disclosure, preferably at least about 70%, about 80%, about 90%, about 95%, about 98%, about 99%, or even about 99.5%, of the compound remains in the gastrointestinal lumen. In such cases, localization to the gastrointestinal lumen refers to reducing net movement across a gastrointestinal layer of epithelial cells, for example, by way of both transcellular and paracellular transport, as well as by active and/or passive transport. The compound in such embodiments is hindered from net permeation of a layer of gastrointestinal epithelial cells in transcellular transport, for example, through an apical membrane of an epithelial cell of the small intestine. The compound in these embodiments is also hindered from net permeation through the "tight junctions" in paracellular transport between gastrointestinal epithelial cells lining the lumen.

In this regard it is to be noted that, in one particular embodiment, the compound is essentially not absorbed at all by the GI tract or gastrointestinal lumen. As used herein, the terms "substantially impermeable" or "substantially systemically non-bioavailable" refers to embodiments wherein no detectable amount of absorption or permeation or systemic exposure of the compound is detected, using means generally known in the art.

In this regard it is to be further noted, however, that in alternative embodiments "substantially impermeable" or "substantially systemically non-bioavailable" provides or allows for some limited absorption in the GI tract, and more particularly the gut epithelium, to occur (e.g., some detectable amount of absorption, such as for example at least about 0.1%, 0.5%, 1% or more and less than about 30%, 20%, 10%, 5%, etc., the range of absorption being for example between about 1% and 30%, or 5% and 20%, etc.; stated another way, "substantially impermeable" or "substantially systemically non-bioavailable" refers to compounds that exhibit some detectable permeability to an epithelium layer of cells in the GI tract of less than about 20% of the administered compound (e.g., less than about 15%, about 10%, or even about 5%, and for example greater than about 0.5%, or 1%), but then are cleared by the liver (i.e., hepatic extraction) and/or the kidney (i.e., renal excretion).

### B. Permeability

In this regard it is to be noted that, in various embodiments, the ability of a compound to be substantially systemically non-bioavailable is based on the compound charge, size, and/or other physicochemical parameters (e.g., polar surface area, number of hydrogen bond donors and/or acceptors therein, number of freely rotatable bonds, etc.). More specifically, it is to be noted that the absorption character of a compound can be selected by applying principles of pharmacodynamics, for example, by applying Lipinski's rule, also known as "the rule of five." Although not a rule, but rather a set of guidelines, Lipinski shows that small molecule drugs with (i) a molecular weight, (ii) a number of hydrogen bond donors, (iii) a number of hydrogen bond acceptors, and/or (iv) a water/octanol partition coefficient (Moriguchi Log P), greater than a certain threshold value, generally do not show significant systemic concentration (i.e., are generally not absorbed to any significant degree). (See, e.g., Lipinski et al., Advanced Drug Delivery Reviews, 46, 2001 3-26) Accordingly, substantially systemically non-bioavailable compounds (e.g., substantially systemically non- bioavailable NHE-inhibiting compounds) can be designed to have molecular structures exceeding one or more of Lipinski's threshold values. (See also Lipinski et al., Experimental and Computational Approaches to Estimate Solubility and Permeability in Drug Discovery and Development Settings, Adv. Drug Delivery Reviews, 46:3-26 (2001); and Lipinski, Drug-like Properties and the Causes of Poor Solubility and Poor Permeability, J. Pharm. & Toxicol. Methods, 44:235-249 (2000)) In some embodiments, for example, a substantially impermeable or substantially systemically non-bioavailable NHE-inhibiting compound of the present disclosure can be constructed to feature one or more of the following characteristics: (i) a MW greater than about 500 Da, about 1000 Da, about 2500 Da, about 5000 Da, about 10,000 Da or more (in the non-salt form of the compound); (ii) a total number of NH and/or OH and/or other potential hydrogen bond donors greater than about 5, about 10, about 15 or more; (iii) a total number of O atoms and/or N atoms and/or other potential hydrogen bond acceptors greater than about 5, about 10, about 15 or more; and/or (iv) a Moriguchi partition coefficient greater than about 10⁵ (i.e., Log P greater than about 5, about 6, about 7, etc.), or alternatively less than about 10 (i.e., a Log P of less than 1, or even 0).

In addition to the parameters noted above, the molecular polar surface area (i.e., "PSA"), which may be characterized as the surface belonging to polar atoms, is a descriptor that has also been shown to correlate well with passive transport through membranes and, therefore, allows prediction of transport properties of drugs. It has been successfully applied for the prediction of intestinal absorption and Caco2 cell monolayer penetration. (For Caco2 cell monolayer penetration test details, see for example the description of the Caco2 Model provided in Example 31 of U.S. Pat. No. 6,737,423, and the text of Example 31 in particular, which may be applied for example to the evaluation or testing of the compounds of the present disclosure.) PSA is expressed in Å² (squared angstroms) and is computed from a three-dimensional molecular representation. A fast calculation method is now available (see, e.g., Ertl et al., Journal of Medicinal Chemistry, 2000, 43, 3714-3717) using a desktop computer and commercially available chemical graphic tools packages, such as ChemDraw. The term "topological PSA" (tPSA) has been coined for this fast-calculation method. IPSA is well correlated with human absorption data with common drugs (see, e.g., Table 1, below):

**Table 1**

| name | % FA*^{a}* | TPSA*^{b}* |
|---|---|---|
| metaprolol | 102 | 50.7 |
| nordiazepam | 99 | 41.5 |
| diazepam | 97 | 32.7 |
| oxprenolol | 97 | 50.7 |
| phenazone | 97 | 26.9 |
| oxazepam | 97 | 61.7 |
| alprenolol | 96 | 41.9 |
| practolol | 95 | 70.6 |
| pindolol | 92 | 57.3 |
| ciprofloxacin | 69 | 74.6 |
| metalazone | 84 | 92.5 |
| tranexamic acid | 55 | 63.3 |
| atenolol | 54 | 84.6 |
| sulpiride | 36 | 101.7 |
| mannitol | 26 | 121.4 |
| foscarnet | 17 | 94.8 |
| sulfasalazine | 12 | 141.3 |
| alsalazine | 2.3 | 139.8 |
| lactulose | 0.6 | 197.4 |
| raffinase | 0.3 | 268.7 |

(from Ertl et al., J. Med. Chem., 2000, 43 :3714-3717). Accordingly, in some preferred embodiments, the compounds of the present disclosure may be constructed to exhibit a IPSA value greater than about 100 Å², about 120 Å², about 130 Å², or about 140 Å², and in some instances about 150 Å², about 200 Å², about 250 Å², about 270 Å², about 300 Å², about 400 Å²,or even about 500 Å², such that the compounds are substantially impermeable or substantially systemically non-bioavailable (as defined elsewhere herein).

Because there are exceptions to Lipinski's "rule," or the tPSA model, the permeability properties of the compounds of the present disclosure may be screened experimentally. The permeability coefficient can be determined by methods known to those of skill in the art, including for example by Caco-2 cell permeability assay and/or using an artificial membrane as a model of a gastrointestinal epithelial cell. (As previously noted above, see for example U.S. Patent No. 6,737,423, Example 31 for a description of the Caco-2 Model). A synthetic membrane impregnated with, for example, lecithin and/or dodecane to mimic the net permeability characteristics of a gastrointestinal mucosa, may be utilized as a model of a gastrointestinal mucosa. The membrane can be used to separate a compartment containing the compound of the present disclosure from a compartment where the rate of permeation will be monitored. Also, parallel artificial membrane permeability assays (PAMPA) can be performed. Such *in vitro* measurements can reasonably indicate actual permeability *in vivo.* (See, for example, Wohnsland et al., J. Med. Chem., 2001, 44:923-930; Schmidt et al., Millipore Corp. Application Note, 2002, n° AN1725EN00, and n° AN1728EN00)

Accordingly, in some embodiments, the compounds utilized in the methods of the present disclosure may have a permeability coefficient, Pₐₚₚ, of less than about 100 x 10⁻⁶ cm/s, or less than about 10 x 10⁻⁶ cm/s, or less than about 1 x 10⁻⁶ cm/s, or less than about 0.1 x 10⁻⁶ cm/s, when measured using means known in the art (such as for example the permeability experiment described in Wohnsland et al., J. Med. Chem., 2001, 44. 923- 930).

As previously noted, in accordance with the present disclosure, a NHE-inhibiting small molecule moiety is modified as described above to hinder the net absorption through a layer of gut epithelial cells, rendering the resulting compound substantially systemically non-bioavailable. In various embodiments, the compounds of the present disclosure comprise an NHE-inhibiting small molecule moiety linked, coupled or otherwise attached to a moiety which renders the overall compound substantially impermeable or substantially systemically non-bioavailable. More specifically, the NHE- inhibiting small molecule moiety is coupled to a dimer, multimer or polymer moiety, such that the resulting compound is substantially impermeable or substantially systemically non- bioavailable. The dimer, multimer or polymer portion or moiety may be of a molecular weight greater than about 500 Daltons (Da), about 1000 Da, about 2500 Da, about 5000 Da, about 10,000 Da or more, and in particular may have a molecular weight in the range of about 1000 Daltons (Da) to about 500,000 Da, preferably in the range of about 5000 to about 200,000 Da, and more preferably may have a molecular weight that is sufficiently high to essentially preclude any net absorption through a layer of gut epithelial cells of the compound.

### C. Persistent Inhibitory Effect

In other embodiments, the substantially impermeable or substantially systemically non-bioavailable NHE-inhibiting compounds utilized in the treatment methods of the present disclosure may additionally exhibit a persistent inhibitor effect. This effect manifests itself when the inhibitory action of a compound at a certain concentration in equilibrium with the epithelial cell (e.g., at or above its inhibitory concentration, IC) does not revert to baseline (i.e., sodium transport without inhibitor) after the compound is depleted by simple washing of the luminal content.

This effect can be interpreted as a result of the tight binding of the NHE-inhibiting compounds to the NHE protein at the intestinal apical side of the gut epithelial cell. The binding can be considered as quasi-irreversible to the extent that, after the compound has been contacted with the gut epithelial cell and subsequently washed off said gut epithelial cell, the flux of sodium transport is still significantly lower than in the control without the compound. This persistent inhibitory effect has the clear advantage of maintaining drug activity within the GI tract even though the residence time of the active in the upper GI tract is short, and when no entero-biliary recycling process is effective to replenish the compound concentration near its site of action.

Such a persistent inhibitory effect has an obvious advantage in terms of patient compliance, but also in limiting drug exposure within the GI tract.

The persistence effect can be determined using in vitro methods; in one instance, cell lines expressing NHE transporters are split in different vials and treated with a NHE-inhibiting compound and sodium solution to measure the rate of sodium uptake. The cells in one set of vials are washed for different periods of time to remove the inhibitor, and sodium uptake measurement is repeated after the washing. Compounds that maintain their inhibitory effect after multiple/lengthy washing steps (compared to the inhibitory effect measured in the vials where washing does not occur) are persistent inhibitors. Persistence effect can also be characterized *ex vivo* by using the everted sac technique, whereby transport of Na is monitored using an excised segment of GI perfused with a solution containing the inhibitor and shortly after flushing the bathing solution with a buffer solution free from inhibitor. A persistence effect can also be characterized *in vivo* by observing the time needed for sodium balance to return to normal when the inhibitor treatment is discontinued. The limit of the method resides in the fact that apical cells (and therefore apical NHE transporters) are sloughed off after a period of 3 to 4 days, the typical turnover time of gut epithelial cells. A persistence effect can be achieved by increasing the residence time of the active compound at the apical surface of the gut epithelial cells; this can be obtained by designing NHE antiport inhibitors with several NHE-inhibiting small molecule moieties built-in the small molecule or oligomer (wherein "several" as used herein typically means at least about 2, about 4, about 6 or more). Examples of such structures in the context of analogs of the antibiotic vancomycin are given in Griffin, et al., J. Am. Chem. Soc., 2003, 125, 6517-6531. Alternatively the compound comprises groups that contribute to increase the affinity towards the gut epithelial cell so as to increase the time of contact with the gut epithelial cell surface. Such groups are referred to as being "mucoadhesive." More specifically, the Core or L moiety can be substituted by such mucoadhesive groups, such as polyacrylates, partially deacetylated chitosan or polyalkylene glycol. (See also Patil, S.B. et al., Curr. Drug. Deliv., 2008, Oct. 5(4), pp. 312-8.)

### D. GI Enzyme Resistance

Because the compounds utilized in the treatment methods of the present disclosure are preferably substantially systemically non-bioavailable, and/or preferably exhibit a persistent inhibitory effect, it is also desirable that, during their prolonged residence time in the gut, these compounds sustain the hydrolytic conditions prevailing in the upper GI tract. In such embodiments, compounds of the present disclosure are resistant to enzymatic metabolism. For example, administered compounds are preferably resistant to the activity of P450 enzymes, glucurosyl transferases, sulfotransferases, glutathione S- transferases, and the like, in the intestinal mucosa, as well as gastric (e.g., gastric lipase, and pepsine), pancreatic (e.g., trypsin, triglyceride pancreatic lipase, phospholipase A2, endonucleases, nucleotidases, and alpha-amylase), and brush-border enzymes (e.g., alkaline phosphatase, glycosidases, and proteases) generally known in the art.

The compounds that are utilized in methods of the present disclosure are also preferably resistant to metabolism by the bacterial flora of the gut; that is, the compounds are not substrates for enzymes produced by bacterial flora. In addition, the compounds administered in accordance with the methods of the present disclosure may be substantially inactive towards the gastrointestinal flora, and do not disrupt bacterial growth or survival. As a result, in various embodiments herein, the minimal inhibitory concentration (or "MIC") against GI flora is desirably greater than about 15 µg/ml, about 30 µg/ml, about 60 µg/ml, about 120 µg/ml, or even about 240 µg/ml, the MIC in various embodiments being for example between about 16 and about 32 µg/ml, or between about 64 and about 128 µg/ml, or greater than about 256 µg/ml.

To one skilled in the art of medicinal chemistry, metabolic stability can be achieved in a number of ways. Functionality susceptible to P450-mediated oxidation can be protected by, for example, blocking the point of metabolism with a halogen or other functional group. Alternatively, electron withdrawing groups can be added to a conjugated system to generally provide protection to oxidation by reducing the electrophilicity of the compound. Proteolytic stability can be achieved by avoiding secondary amide bonds, or by incorporating changes in stereochemistry or other modifications that prevent the drug from otherwise being recognized as a substrate by the metabolizing enzyme.

### E. Sodium and/or Fluid Output

It is also to be noted that, in various embodiments of the present disclosure, one or more of the NHE-inhibiting compounds detailed herein, when administered either alone or in combination with one or more additional pharmaceutically active compounds or agents (including, for example, a fluid-absorbing polymer) to a patient in need thereof, may act to increase the patient's daily fecal output of sodium by at least about 20, about 30 mmol, about 40 mmol, about 50 mmol, about 60 mmol, about 70 mmol, about 80 mmol, about 90 mmol, about 100 mmol, about 125 mmol, about 150 mmol or more, the increase being for example within the range of from about 20 to about 150 mmol/day, or from about 25 to about 100 mmol/day, or from about 30 to about 60 mmol/day

Additionally, or alternatively, it is also to be noted that, in various embodiments of the present disclosure, one or more of the NHE-inhibiting compounds detailed herein, when administered either alone or in combination with one or more additional pharmaceutically active compounds or agents (including, for example, a fluid- absorbing polymer) to a patent in need thereof, may act to increase the patient's daily fluid output by at least about 100 ml, about 200 ml, about 300 ml, about 400 ml, about 500 ml, about 600 ml, about 700 ml, about 800 ml, about 900 ml, about 1000 ml or more, the increase being for example within the range of from about 100 to about 1000 ml/day, or from about 150 to about 750 ml/day, or from about 200 to about 500 ml/day (assuming isotonic fluid).

### F. Cₘₐₓ and IC₅₀

It is also to be noted that, in various embodiments of the present disclosure, one or more of the NHE-inhibiting compounds detailed herein, when administered either alone or in combination with one or more additional pharmaceutically active compounds or agents (including, for example, a fluid-absorbing polymer) to a patient in need thereof at a dose resulting in at least a 10% increase in fecal water content, has a Cₘₐₓ that is less than the IC₅₀ for NHE-3, more specifically, less than about 10X (10 times) the IC₅₀, and, more specifically still, less than about 100X (100 times) the IC₅₀.

Additionally, or alternatively, it is also to be noted that, in various embodiments of the present disclosure, one or more of the NHE-inhibiting compounds detailed herein, when administered either alone or in combination with one or more additional pharmaceutically active compounds or agents (including, for example, a fluid- absorbing polymer) to a patient in need thereof, may have a Cₘₐₓ of less than about 10 ng/ml, about 7.5 ng/ml, about 5 ng/ml, about 2.5 ng/ml, about 1 ng/ml, or about 0.5 ng/ml, the Cₘₐₓ being for example within the range of about 1 ng/ml to about 10 ng/ml, or about 2.5 ng/ml to about 7.5 ng/ml.

Additionally, or alternatively, it is also to be noted that, in various embodiments of the present disclosure, one or more of the NHE-inhibiting compounds detailed herein, when administered either alone or in combination with one or more additional pharmaceutically active compounds or agents (including, for example, a fluid- absorbing polymer) to a patient in need thereof, may have a IC₅₀ of less than about 10 µM, about 7.5 µM, about 5 µM, about 2.5 µM, about 1 µM, or about 0.5 µM, the IC₅₀ being for example within the range of about 1 µM to about 10 µM, or about 2.5 µM to about 7.5 µM.

Additionally, or alternatively, it is also to be noted that, in various embodiments of the present disclosure, one or more of the NHE-inhibiting compounds detailed herein, when administered to a patient in need thereof, may have a ratio of IC₅₀:Cₘₐₓ, wherein IC₅₀ and Cₘₐₓ are expressed in terms of the same units, of at least about 10, about 50, about 100, about 250, about 500, about 750, or about 1000.

Additionally, or alternatively, it is also to be noted that, in various embodiments of the present disclosure, wherein one or more of the NHE-inhibiting compounds as detailed herein is orally administered to a patent in need thereof, within the therapeutic range or concentration, the maximum compound concentration detected in the serum, defined as Cₘₐₓ, is lower than the NHE inhibitory concentration IC₅₀ of said compound. As previously noted, as used herein, IC₅₀ is defined as the quantitative measure indicating the concentration of the compound required to inhibit 50% of the NHE-mediated Na / H antiport activity in a cell based assay.

### III. Pharmaceutical Compositions and Methods of Treatment

### A. Compositions and Methods

### 1. Fluid Retention and/or Salt Overload Disorders

A pharmaceutical composition or preparation that may be used in accordance with the present disclosure for the treatment of various disorders associated with fluid retention and/or salt overload in the gastrointestinal tract (e.g., hypertension, heart failure (in particular, congestive heart failure), chronic kidney disease, end-stage renal disease, liver disease and/or peroxisome proliferator-activated receptor (PPAR) gamma agonist-induced fluid retention) comprises, in general, the substantially impermeable or substantially systemically non-bioavailable NHE-inhibiting compound of the present disclosure, as well as various other optional components as further detailed herein below (e.g., pharmaceutically acceptable excipients, etc.). The compounds utilized in the treatment methods of the present disclosure, as well as the pharmaceutical compositions comprising them, may accordingly be administered alone, or as part of a treatment protocol or regiment that includes the administration or use of other beneficial compounds (as further detailed elsewhere herein). In some particular embodiments, the NHE-inhibiting compound, including any pharmaceutical composition comprising the compound, is administered with a fluid-absorbing polymer (as more fully described below).

A "subject" or "mammal" is preferably a human, but can also be an animal in need of treatment with a compound of the disclosure, e.g., companion animals (e.g., dogs, cats, and the like), farm animals (e.g., cows, pigs, horses and the like) and laboratory animals (e.g., rats, mice, guinea pigs and the like).

Subjects "in need of treatment" with a compound of the present disclosure, or subjects "in need of NHE inhibition" include subjects with diseases and/or conditions that can be treated with substantially impermeable or substantially systemically non- bioavailable NHE-inhibiting compounds, with or without a fluid-absorbing polymer, to achieve a beneficial therapeutic and/or prophylactic result. A beneficial outcome includes a decrease in the severity of symptoms or delay in the onset of symptoms, increased longevity and/or more rapid or more complete resolution of the disease or condition. For example, a subject in need of treatment may be suffering from hypertension; from salt- sensitive hypertension which may result from dietary salt intake; from a risk of a cardiovascular disorder (e.g., myocardial infarction, congestive heart failure and the like) resulting from hypertension; from heart failure (e.g., congestive heart failure) resulting in fluid or salt overload; from chronic kidney disease resulting in fluid or salt overload, from end stage renal disease resulting in fluid or salt overload; from liver disease resulting in fluid or salt overload; from peroxisome proliferator-activated receptor (PPAR) gamma agonist-induced fluid retention; or from edema resulting from congestive heart failure or end stage renal disease. In various embodiments, a subject in need of treatment typically shows signs of hypervolemia resulting from salt and fluid retention that are common features of congestive heart failure, renal failure or liver cirrhosis. Fluid retention and salt retention manifest themselves by the occurrence of shortness of breath, edema, ascites or interdialytic weight gain. Other examples of subjects that would benefit from the treatment are those suffering from congestive heart failure and hypertensive patients and, particularly, those who are resistant to treatment with diuretics, i.e., patients for whom very few therapeutic options are available. A subject "in need of treatment" also includes a subject with hypertension, salt-sensitive blood pressure and subjects with systolic / diastolic blood pressure greater than about 130-139 / 85-89 mm Hg.

Administration of NHE-inhibiting compounds, with or without administration of fluid-absorbing polymers, may be beneficial for patients put on "non- added salt" dietary regimen (i.e., 60-100 mmol of Na per day), to liberalize their diet while keeping a neutral or slightly negative sodium balance (i.e., the overall uptake of salt would be equal of less than the secreted salt). In that context, "liberalize their diet" means that patients treated may add salt to their meals to make the meals more palatable, or/and diversify their diet with salt-containing foods, thus maintaining a good nutritional status while improving their quality of life.

The treatment methods described herein may also help patients with edema associated with chemotherapy, pre-menstrual fluid overload and preeclampsia (pregnancy-induced hypertension).

Accordingly, it is to be noted that the present disclosure is further directed to methods of treatment involving the administration of the compound of the present disclosure, or a pharmaceutical composition comprising such a compound. Such methods may include, for example, a method for treating hypertension, the method comprising administering to the patient a substantially impermeable or substantially systemically non- bioavailable NHE-inhibiting compound, or a pharmaceutical composition comprising it. The method may be for reducing fluid overload associated with heart failure (in particular, congestive heart failure), the method comprising administering to the patient a substantially impermeable or substantially systemically non-bioavailable NHE-inhibiting compound or pharmaceutical composition comprising it. The method may be for reducing fluid overload associated with end stage renal disease, the method comprising administering to the patient a substantially impermeable or substantially systemically non- bioavailable NHE-inhibiting compound or composition comprising it. The method may be for reducing fluid overload associated with peroxisome proliferator-activated receptor (PPAR) gamma agonist therapy, the method comprising administering to the patient a substantially impermeable or substantially systemically non-bioavailable NHE-inhibiting compound or composition comprising it. Additionally, or alternatively, the method may be for decreasing the activity of an intestinal NHE transporter in a patient, the method comprising: administering to the patient a substantially impermeable or substantially systemically non-bioavailable NHE-inhibiting compound, or a composition comprising it.

### 2. Gastrointestinal Tract Disorders

A pharmaceutical composition or preparation that may be used in accordance with the present disclosure for the treatment of various gastrointestinal tract disorders, including the treatment or reduction of pain associated with gastrointestinal tract disorders, comprises, the substantially impermeable or substantially systemically non- bioavailable NHE-inhibiting compound of the present disclosure, as well as various other optional components as further detailed herein below (e.g., pharmaceutically acceptable excipients, etc.). The compounds utilized in the treatment methods of the present disclosure, as well as the pharmaceutical compositions comprising them, may accordingly be administered alone, or as part of a treatment protocol or regiment that includes the administration or use of other beneficial compounds (as further detailed elsewhere herein). In some particular embodiments, the NHE-inhibiting compound, including any pharmaceutical composition comprising the compound, is administered with a fluid- absorbing polymer (as more fully described below).

A "subject" is preferably a human, but can also be an animal in need of treatment with a compound of the disclosure, e.g., companion animals (e.g., dogs, cats, and the like), farm animals (e.g., cows, pigs, horses and the like) and laboratory animals (e.g., rats, mice, guinea pigs and the like).

Subjects "in need of treatment" with a compound of the present disclosure, or subjects "in need of NHE inhibition" include subjects with diseases and/or conditions that can be treated with substantially impermeable or substantially systemically non- bioavailable NHE-inhibiting compounds, with or without a fluid-absorbing polymer, to achieve a beneficial therapeutic and/or prophylactic result. A beneficial outcome includes a decrease in the severity of symptoms or delay in the onset of symptoms, increased longevity and/or more rapid or more complete resolution of the disease or condition. For example, a subject in need of treatment is suffering from a gastrointestinal tract disorder; the patient is suffering from a disorder selected from the group consisting of: a gastrointestinal motility disorder, irritable bowel syndrome, chronic constipation, chronic idiopathic constipation, chronic constipation occurring in cystic fibrosis patients, chronic constipation occurring in chronic kidney disease patients, calcium-induced constipation in osteoporotic patients, opioid-induced constipation, a functional gastrointestinal tract disorder, gastroesophageal reflux disease, functional heartburn, dyspepsia, functional dyspepsia, non-ulcer dyspepsia, gastroparesis, chronic intestinal pseudo-obstruction, Crohn's disease, ulcerative colitis and related diseases referred to as inflammatory bowel syndrome, colonic pseudo-obstruction, and the like.

In various preferred embodiments, the constipation to be treated is: associated with the use of a therapeutic agent; associated with a neuropathic disorder; postsurgical constipation (postoperative ileus); associated with a gastrointestinal tract disorder; idiopathic (functional constipation or slow transit constipation); associated with neuropathic, metabolic or endocrine disorder (e.g., diabetes mellitus, renal failure, hypothyroidism, hyperthyroidism, hypocalcaemia, Multiple Sclerosis, Parkinson's disease, spinal cord lesions, neurofibromatosis, autonomic neuropathy, Chagas disease, Hirschsprung's disease or cystic fibrosis, and the like). Constipation may also be the result of surgery (postoperative ileus) or due the use of drugs such as analgesics (e.g., opioids), antihypertensives, anticonvulsants, antidepressants, antispasmodics and antipsychotics.

Accordingly, it is to be noted that the present disclosure is further directed to methods of treatment involving the administration of the compound of the present disclosure, or a pharmaceutical composition comprising such a compound. Such methods may include, for example, a method for increasing gastrointestinal motility in a patient, the method comprising administering to the patient a substantially non-permeable or substantially non-bioavailable NHE-inhibiting compound, or a pharmaceutical composition comprising it. Additionally, or alternatively, the method may be for decreasing the activity of an intestinal NHE transporter in a patient, the method comprising administering to the patient a substantially non-permeable or substantially non-bioavailable NHE-inhibiting compound, or a pharmaceutical composition comprising it. Additionally, or alternatively, the method may be for treating a gastrointestinal tract disorder, a gastrointestinal motility disorder, irritable bowel syndrome, chronic calcium-induced constipation in osteoporotic patients, chronic constipation occurring in cystic fibrosis patients, chronic constipation occurring in chronic kidney disease patients, a functional gastrointestinal tract disorder, gastroesophageal reflux disease, functional heartburn, dyspepsia, functional dyspepsia, non-ulcer dyspepsia, gastroparesis, chronic intestinal pseudo-obstruction, colonic pseudoobstruction, Crohn's disease, ulcerative colitis, inflammatory bowel disease, the method comprising administering an antagonist of the intestinal NHE, and more specifically, a substantially non-permeable or substantially non-bioavailable NHE-inhibiting compound, or a pharmaceutical composition comprising it, either orally or by rectal suppository. Additionally, or alternatively, the method may be for treating or reducing pain, including visceral pain, pain associated with a gastrointestinal tract disorder or pain associated with some other disorder, the method comprising administering to a patient a substantially non- permeable or substantially non-bioavailable NHE-inhibiting compound, or a pharmaceutical composition comprising it. Additionally, or alternatively, the method may be for treating inflammation, including inflammation of the gastrointestinal tract, e.g., inflammation associated with a gastrointestinal tract disorder or infection or some other disorder, the method comprising administering to a patient a substantially non-permeable or substantially non-bioavailable NHE-inhibiting compound, or a pharmaceutical composition comprising it.

### 3. Metabolic disorders

A pharmaceutical composition or preparation that may be used in accordance with the present disclosure for the treatment of various metabolic disorders including the treatment or reduction of type II diabetes mellitus (T2DM), metabolic syndrome, and/or symptoms associated with such disorders comprises, in general, the substantially impermeable or substantially systemically non-bioavailable NHE-inhibiting compound of the present disclosure, as well as various other optional components as further detailed herein below (e.g., pharmaceutically acceptable excipients, etc.). The compounds utilized in the treatment methods of the present disclosure, as well as the pharmaceutical compositions comprising them, may accordingly be administered alone, or as part of a treatment protocol or regiment that includes the administration or use of other beneficial compounds (as further detailed elsewhere herein).

Obesity is becoming a worldwide epidemic. In the United States, approximately 2/3rds of the population is either overweight (body mass index [BMI] 25 to 29.9) or obese (BMI ≥ 30) (Ogden, CL et al, "Prevalence of overweight and obesity in the united states, 1999-2004" JAMA 2006, 295, 1549-1555). Obesity is a major risk factor for the development of diabetes and related complications, including cardiovascular disease and chronic kidney disease (CKD). The prevalence of T2DM has increased alarmingly in the United States. The American Diabetes Associated (ADA) estimates that more than 23 million U.S. adults aged 20 years or older have diabetes, with T2DM accounting for approximately 95% of these cases. The World Health Organization (WHO) has put the number of persons with diabetes worldwide at approximately 170 million (Campbell, R. K. "Type 2 diabetes: where we are today: an overview of disease burden, current treatments, and treatment strategies" Journal of the American Pharmacists Association 2009, 49(5), S3-S9).

Obesity is also a major risk factor for the development of metabolic syndrome, and subsequently the development of CKD. Metabolic syndrome, previously known as Syndrome X, the plurimetabolic syndrome, the dysmetabolic syndrome, and other names, consists of a clustering of metabolic abnormalities including abdominal obesity, hypertriglyceridemia, low levels of high-density lipoprotein (HDL) cholesterol, elevated blood pressure (BP), and elevations in fasting glucose or diabetes (Townsend, R. R. et al "Metabolic Syndrome, Components, and Cardiovascular Disease Prevalence in Chronic Kidney Disease: Findings from the Chronic Renal Insufficiency Cohort (CRIC) Study" American Journal of Nephrology 2011, 33, 477-484). Metabolic syndrome is common in patients with CKD and an important risk factor for the development and progression of CKD.

Hemodynamic factors appear to play a significant role in obesity-induced renal dysfunction. Hypertension, which is closely linked to obesity, appears to be a major cause of renal dysfunction in obese patients (Wahba, I. M. et al "Obesity and obesity- initiated metabolic syndrome: mechanistic links to chronic kidney disease" Clinical Journal of the American Society of Nephrology 2007, 2, 550-562). Studies in animals and in humans have shown that obesity is associated with elevated glomerular filtration rate (GFR) and increased renal blood flow. This likely occurs because of afferent arteriolar dilation as a result of proximal salt reabsorption, coupled with efferent renal arteriolar vasoconstriction as a result of elevated angiotensin II levels. These effects may contribute to hyperfiltration, glomerulomegaly, and later focal glomerulosclerosis. Even though GFR is increased in obesity, urinary sodium excretion in response to a saline load is often delayed, and individuals exhibit an abnormal pressure natriuresis, indicating avid proximal tubular sodium reabsorption. In addition, increased fat distribution can cause increased intra-abdomial pressure, leading to renal vein compression, thus raising renal venous pressure and diminishing renal perfusion. Increased fat, through a variety of mechanisms, can cause elevated renal interstitial fluid hydrostatic fluid and may stimulate renal sodium retention the thereby contribute to hypertension (Wahba_2007).

In view of the above, there exists a need in the art for agents that can divert sodium and fluid from a subject via mechanisms that either avoid the kidney, or do not depend upon normal kidney function. A "subject" with metabolic disease, including T2DM, metabolic syndrome, and the like, is preferably a human, but can also be an animal in need of treatment with a compound of the disclosure, e.g., companion animals (e.g., dogs, cats, and the like), farm animals (e.g., cows, pigs, horses and the like) and laboratory animals (e.g., rats, mice, guinea pigs and the like).

Subjects "in need of treatment" with a compound of the present disclosure, or subjects "in need of NHE inhibition" include subjects with diseases and/or conditions that can be treated with substantially impermeable or substantially systemically non- bioavailable NHE-inhibiting compounds, with or without a fluid-absorbing polymer, to achieve a beneficial therapeutic and/or prophylactic result. A beneficial outcome includes a decrease in the severity of symptoms or delay in the onset of symptoms, increased longevity and/or more rapid or more complete resolution of the disease or condition. For example, a subject with a metabolic disorder causing or exacerbating chronic kidney disease would benefit from a treatment modality that could divert excess sodium and fluid from the body by a method that does not require normally functionaling kidneys. Such a treatment would include the method comprising administering to a patient a substantially non-permeable or substantially non-bioavailable NHE-inhibiting compound, or a pharmaceutical composition comprising it.

The compounds utilized in the treatment methods of the present disclosure, as well as the pharmaceutical compositions comprising them, may accordingly be administered alone, or as part of a combination therapy or regimen that includes the administration or use of other therapeutic compounds related to the treatment of metabolic disorders such as T2DM and metabolic syndrome. In some particular embodiments, the NHE-inhibiting compound, including any pharmaceutical composition comprising the compound, is administered with a fluid absorbing polymer.

### B. Combination Therapies

### 1. Fluid Retention and/or Salt Overload Disorders

As previously noted, the compounds described herein can be used alone or in combination with other agents. For example, the compounds can be administered together with a diuretic (i.e., High Ceiling Loop Diuretics, Benzothiadiazide Diuretics, Potassium Sparing Diuretics, Osmotic Diuretics), cardiac glycoside, ACE inhibitor, angiotensin-2 receptor antagonist, aldosterone antagonist, aldosterone synthase inhibitor, renin inhibitor, calcium channel blocker, beta blocker, alpha blocker, central alpha agonist, vasodilator, blood thinner, anti-platelet agent, lipid-lowering agent, peroxisome proliferator-activated receptor (PPAR) gamma agonist agent or compound or with a fluid- absorbing polymer as more fully described below. The agent can be covalently attached to a compound described herein or it can be a separate agent that is administered together with or sequentially with a compound described herein in a combination therapy.

Combination therapy can be achieved by administering two or more agents, e.g., a substantially non-permeable or substantially systemically non-bioavailable NHE-inhibiting compound described herein and a diuretic, cardiac glycoside, ACE inhibitor, angiotensin-2 receptor antagonist, aldosterone antagonist, aldosterone synthase inhibitor, renin inhibitor, calcium channel blocker, beta blocker, alpha blocker, central alpha agonist, vasodilator, blood thinner, anti-platelet agent or compound, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or weeks of each other. In some cases even longer intervals are possible. While in many cases it is desirable that the two or more agents used in a combination therapy be present in within the patient's body at the same time, this need not be so.

Combination therapy can also include two or more administrations of one or more of the agents used in the combination. For example, if agent X and agent Y are used in a combination, one could administer them sequentially in any combination one or more times, e.g., in the order X-Y-X, X-X-Y, Y-X-Y, Y-Y-X, X-X-Y-Y, etc.

The compounds described herein can be used in combination therapy with a diuretic. Among the useful diuretic agents are, for example: High Ceiling Loop Diuretics [Furosemide (Lasix), Ethacrynic Acid (Edecrin), Bumetanide (Bumex)], Benzothiadiazide Diuretics [Hydrochlorothiazide (Hydrodiuril), Chlorothiazide (Diuril), Clorthalidone (Hygroton), Benzthiazide (Aguapres), Bendroflumethiazide (Naturetin), Methyclothiazide (Aguatensen), Polythiazide (Renese), Indapamide (Lozol), Cyclothiazide (Anhydron), Hydroflumethiazide (Diucardin), Metolazone (Diulo), Quinethazone (Hydromox), Trichlormethiazide (Naqua)], Potassium Sparing Diuretics [Spironolactone (Aldactone), Triamterene (Dyrenium), Amiloride (Midamor)], and Osmotic Diuretics [Mannitol (Osmitrol)]. Diuretic agents in the various classes are known and described in the literature.

Cardiac glycosides (cardenolides) or other digitalis preparations can be administered with the compounds of the disclosure in co-therapy. Among the useful cardiac glycosides are, for example: Digitoxin (Crystodigin), Digoxin (Lanoxin) or Deslanoside (Cedilanid-D). Cardiac glycosides in the various classes are described in the literature.

Angiotensin Converting Enzyme Inhibitors (ACE Inhibitors) can be administered with the compounds of the disclosure in co-therapy. Among the useful ACE inhibitors are, for example: Captopril (Capoten), Enalapril (Vasotec), Lisinopril (Prinivil). ACE inhibitors in the various classes are described in the literature.

Angiotensin-2 Receptor Antagonists (also referred to as AT 1-antagonists or angiotensin receptor blockers, or ARB's) can be administered with the compounds of the disclosure in co-therapy. Among the useful Angiotensin-2 Receptor Antagonists are, for example: Candesartan (Atacand), Eprosartan (Teveten), Irbesartan (Avapro), Losartan (Cozaar), Telmisartan (Micardis), Valsartan (Diovan). Angiotensin-2 Receptor Antagonists in the various classes are described in the literature.

Calcium channel blockers such as Amlodipine (Norvasc, Lotrel), Bepridil (Vascor), Diltiazem (Cardizem, Tiazac), Felodipine (Plendil), Nifedipine (Adalat, Procardia), Nimodipine (Nimotop), Nisoldipine (Sular), Verapamil (Calan, Isoptin, Verelan) and related compounds described in, for example, EP 625162B1, U.S. Pat. No. 5,364,842, U.S. Pat. No. 5,587,454, U.S. Pat. No. 5,824,645, U.S. Pat. No. 5,859, 186, U.S. Pat. No. 5,994,305, U.S. Pat. No. 6,087,091, U.S. Pat. No. 6,136,786, WO 93/13128 A1, EP 1336409 A1, EP 835126 A1, EP 835126 B1, U.S. Pat. No. 5,795,864, U.S. Pat. No. 5,891,849, U.S. Pat. No. 6,054,429, WO 97/01351 A1, can be used with the compounds of the disclosure.

Beta blockers can be administered with the compounds of the disclosure in co-therapy. Among the useful beta blockers are, for example: Acebutolol (Sectral), Atenolol (Tenormin), Betaxolol (Kerlone), Bisoprolol/hydrochlorothiazide (Ziac), Bisoprolol (Zebeta), Carteolol (Cartrol), Metoprolol (Lopressor, Toprol XL), Nadolol (Corgard), Propranolol (Inderal), Sotalol (Betapace), Timolol (Blocadren). Beta blockers in the various classes are described in the literature.

PPAR gamma agonists such as thiazolidinediones (also called glitazones) can be administered with the compounds of the disclosure in co-therapy. Among the useful PPAR agonists are, for example: rosiglitazone (Avandia), pioglitazone (Actos) and rivoglitazone.

Aldosterone antagonists can be administered with the compounds of the disclosure in co-therapy. Among the useful Aldosterone antagonists are, for example: eplerenone, spironolactone, and canrenone.

Renin inhibitor can be administered with the compounds of the disclosure in co-therapy. Among the useful Renin inhibitors is, for example: aliskiren.

Alpha blockers can be administered with the compounds of the disclosure in co-therapy. Among the useful Alpha blockers are, for example: Doxazosin mesylate (Cardura), Prazosin hydrochloride (Minipress). Prazosin and polythiazide (Minizide), Terazosin hydrochloride (Hytrin). Alpha blockers in the various classes are described in the literature.

Central alpha agonists can be administered with the compounds of the disclosure in co-therapy. Among the useful Central alpha agonists are, for example: Clonidine hydrochloride (Catapres), Clonidine hydrochloride and chlorthalidone (Clorpres, Combipres), Guanabenz Acetate (Wytensin), Guanfacine hydrochloride (Tenex), Methyldopa (Aldomet), Methyldopa and chlorothiazide (Aldochlor), Methyldopa and hydrochlorothiazide (Aldoril). Central alpha agonists in the various classes are described in the literature.

Vasodilators can be administered with the compounds of the disclosure in co-therapy. Among the useful vasodilators are, for example: Isosorbide dinitrate (Isordil), Nesiritide (Natrecor), Hydralazine (Apresoline), Nitrates / nitroglycerin, Minoxidil (Loniten). Vasodilators in the various classes are described in the literature.

Blood thinners can be administered with the compounds of the disclosure in co-therapy. Among the useful blood thinners are, for example: Warfarin (Coumadin) and Heparin. Blood thinners in the various classes are described in the literature.

Anti-platelet agents can be administered with the compounds of the disclosure in co-therapy. Among the useful anti-platelet agents are, for example: Cyclooxygenase inhibitors (Aspirin), Adenosine diphosphate (ADP) receptor inhibitors [Clopidogrel (Plavix), Ticlopidine (Ticlid)], Phosphodiesterase inhibitors [Cilostazol (Pletal)], Glycoprotein IIB/IIIA inhibitors [Abciximab (ReoPro), Eptifibatide (Integrilin), Tirofiban (Aggrastat), Defibrotide], Adenosine reuptake inhibitors [Dipyridamole (Persantine)]. Anti-platelet agents in the various classes are described in the literature.

Lipid-lowering agents can be administered with the compounds of the disclosure in co-therapy. Among the useful lipid-lowering agents are, for example: Statins (HMG CoA reductase inhibitors), [Atorvastatin (Lipitor), Fluvastatin (Lescol), Lovastatin (Mevacor, Altoprev), Pravastatin (Pravachol), Rosuvastatin Calcium (Crestor), Simvastatin (Zocor)], Selective cholesterol absorption inhibitors [ezetimibe (Zetia)], Resins (bile acid sequestrant or bile acid-binding drugs) [Cholestyramine (Questran, Questran Light, Prevalite, Locholest, Locholest Light), Colestipol (Colestid), Colesevelam Hcl (WelChol)], Fibrates (Fibric acid derivatives) [Gemfibrozil (Lopid), Fenofibrate (Antara, Lofibra, Tricor, and Triglide), Clofibrate (Atromid-S)], Niacin (Nicotinic acid). Lipid- lowering agents in the various classes are described in the literature.

The compounds of the disclosure can be used in combination with peptides or peptide analogs that activate the Guanylate Cyclase-receptor in the intestine and results in elevation of the intracellular second messenger, or cyclic guanosine monophosphate (cGMP), with increased chloride and bicarbonate secretion into the intestinal lumen and concomitant fluid secretion. Example of such peptides are Linaclotide (MD-1100 Acetate), endogenous hormones guanylin and uroguanylin and enteric bacterial peptides of the heat stable enterotoxin family (ST peptides) and those described in US 5140102, US 5489670, US 5969097, WO 2006/001931A2, WO 2008/002971A2, WO 2008/106429A2, US 2008/0227685 A1 and US 7041786.

The compounds of the disclosure can be used in combination with type-2 chloride channel agonists, such as Amitiza (Lubiprostone) and other related compounds described in US 6414016.

The compounds described herein can be used in combination therapy with agents used for the treatment of obesity, T2DM, metabolic syndrome and the like. Among the useful agents include: insulin; insulin secretagogues, such as sulphonylureas; glucose-lowering effectors, such as metformin; activators of the peroxisome proliferator-activated receptor γ (PPARy), such as the thiazolidinediones; incretin-based agents including dipeptidyl peptidase-4 inhibitors such as sitagliptin, and synthetic incretin mimetics such as liraglutide and exenatide; alpha-glucosidase inhibitors, such as acarbose; glinides, such as repaglinide and nateglinide, and the like.

The compounds of the disclosure can be used in combination with P2Y2 receptor agonists, such as those described in EP 1196396B 1 and US 6624150.

Other agents include natriuretic peptides such as nesiritide, a recombinant form of brain-natriuretic peptide (B P) and an atrial -natriuretic peptide (ANP). Vasopressin receptor antagonists such as tolvaptan and conivaptan may be co-administered as well as phosphate binders such as renagel, renleva, phoslo and fosrenol. Other agents include phosphate transport inhibitors (as described in U.S. Pat. Nos. 4,806,532; 6,355,823; 6,787,528; 7, 119,120; 7,109, 184; U.S. Pat. Pub. No. 2007/021509; 2006/0280719; 2006/0217426; International Pat. Pubs. WO 2001/005398, WO 2001/087294, WO 2001/082924, WO 2002/028353, WO 2003/048134, WO 2003/057225, WO2003/080630, WO 2004/085448, WO 2004/085382; European Pat. Nos. 1465638 and 1485391; and JP Patent No. 2007131532, or phosphate transport antagonists such as Nicotinamide.

### 2. Gastrointestinal Tract Disorders

As previously noted, the compounds described herein can be used alone or in combination with other agents. For example, the compounds can be administered together with an analgesic peptide or compound. The analgesic peptide or compound can be covalently attached to a compound described herein or it can be a separate agent that is administered together with or sequentially with a compound described herein in a combination therapy.

Combination therapy can be achieved by administering two or more agents, e.g., a substantially non-permeable or substantially non-bioavailable NHE-inhibiting compound described herein and an analgesic peptide or compound, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or weeks of each other. In some cases even longer intervals are possible. While in many cases it is desirable that the two or more agents used in a combination therapy be present in within the patient's body at the same time, this need not be so.

Combination therapy can also include two or more administrations of one or more of the agents used in the combination. For example, if agent X and agent Y are used in a combination, one could administer them sequentially in any combination one or more times, e.g., in the order X-Y-X, X-X-Y, Y-X-Y, Y-Y-X, X-X-Y-Y, etc.

The compounds described herein can be used in combination therapy with an analgesic agent, e.g., an analgesic compound or an analgesic peptide. The analgesic agent can optionally be covalently attached to a compound described herein. Among the useful analgesic agents are, for example: Ca channel blockers, 5HT3 agonists (e.g., MCK- 733), 5HT4 agonists (e.g., tegaserod, prucalopride), and 5HT1 receptor antagonists, opioid receptor agonists (loperamide, fedotozine, and fentanyl), K1 receptor antagonists, CCK receptor agonists (e.g., loxiglumide), K1 receptor antagonists, K3 receptor antagonists, norepinephrine-serotonin reuptake inhibitors (NSR1), vanilloid and cannabanoid receptor agonists, and sialorphin. Analgesics agents in the various classes are described in the literature.

Opioid receptor antagonists and agonists can be administered with the compounds of the disclosure in co-therapy or linked to the compound of the disclosure, e.g., by a covalent bond. For example, opioid receptor antagonists such as naloxone, naltrexone, methyl nalozone, nalmefene, cypridime, beta funaltrexamine, naloxonazine, naltrindole, and nor-binaltorphimine are thought to be useful in the treatment of opioid- induced constipaption (OIC). It can be useful to formulate opioid antagonists of this type in a delayed or sustained release formulation, such that initial release of the antagonist is in the mid to distal small intestine and/or ascending colon. Such antagonists are described in US 6,734, 188 (WO 01/32180 A2). Enkephalin pentapeptide (HOE825; Tyr-D-Lys-Gly-Phe-L-homoserine) is an agonist of the µ- and γ-opioid receptors and is thought to be useful for increasing intestinal motility (*Eur. J. Pharm.,* 219:445, 1992), and this peptide can be used in conjunction with the compounds of the disclosure. Also useful is trimebutine which is thought to bind to mu/delta/kappa opioid receptors and activate release of motilin and modulate the release of gastrin, vasoactive intestinal peptide, gastrin and glucagons. K-opioid receptor agonists such as fedotozine, ketocyclazocine, and compounds described in US 2005/0176746 (WO 03/097051 A2), can be used with or linked to the compounds of the disclosure. In addition, µ-opioid receptor agonists, such as morphine, diphenyloxylate, frakefamide (H-Tyr-D-Ala-Phe(F)-Phe-NH₂; disclosed in WO 01/019849 A1) and loperamide can be used.

Tyr-Arg (kyotorphin) is a dipeptide that acts by stimulating the release of met-enkephalins to elicit an analgesic effect (J. Biol. Chem. 262:8165, 1987). Kyotorphin can be used with or linked to the compounds of the disclosure. CCK receptor agonists such as caerulein from amphibians and other species are useful analgesic agents that can be used with or linked to the compounds of the disclosure.

Conotoxin peptides represent a large class of analgesic peptides that act at voltage gated Ca channels, NMDA receptors or nicotinic receptors. These peptides can be used with or linked to the compounds of the disclosure.

Peptide analogs of thymulin (US 7,309,690 or FR 2830451) can have analgesic activity and can be used with or linked to the compounds of the disclosure.

CCK (CCKa or CCKb) receptor antagonists, including loxiglumide and dexloxiglumide (the R-isomer of loxiglumide) (US 5, 130,474 or WO 88/05774) can have analgesic activity and can be used with or linked to the compounds of the disclosure.

Other useful analgesic agents include 5-HT4 agonists such as tegaserod/zelnorm and lirexapride. Such agonists are described in: EP1321142 A1, WO 03/053432A1, EP 505322 A1, EP 505322 B1, EP 507672 A1, EP 507672 1, U.S. Pat. No. 5,510,353 and U.S. Pat. No. 5,273,983.

Calcium channel blockers such as ziconotide and related compounds described in, for example, EP 625162 B1, U.S. Pat. No. 5,364,842, U.S. Pat. No. 5,587,454, U.S. Pat. No. 5,824,645, U.S. Pat. No. 5,859,186, U.S. Pat. No. 5,994,305, U.S. Pat. No. 6,087,091, U.S. Pat. No. 6,136,786, WO 93/13128 A1, EP 1336409 A1, EP 835126 A1, EP 835126 B1, U.S. Pat. No. 5,795,864, U.S. Pat. No. 5,891,849, U.S. Pat. No. 6,054,429, WO 97/01351 A1, can be used with or linked to the compounds of the disclosure.

Various antagonists of the NK-1, NK-2, and NK-3 receptors (for a review see Giardina et al. 2003 Drugs 6:758) can be can be used with or linked to the compounds of the disclosure.

NK1 receptor antagonists such as: aprepitant (Merck & Co Inc), vofopitant, ezlopitant (Pfizer, Inc.), R-673 (Hoffmann-La Roche Ltd), SR-14033 and related compounds described in, for example, EP 873753 A1, U.S. 20010006972 A1, U.S. 20030109417 A1, WO 01/52844 A1.

K-2 receptor antagonists such as nepadutant (Menarini Ricerche SpA), saredutant (Sanofi-Synthelabo), SR-144190 (Sanofi-Synthelabo) and UK-290795 (Pfizer Inc) can be used with or linked to the compounds of the disclosure.

K3 receptor antagonists such as osanetant (Sanofi-Synthelabo), talnetant and related compounds described in, for example, WO 02/094187 A2, EP 876347 A1, WO 97/21680 A1, U.S. Pat. No. 6,277,862, WO 98/11090, WO 95/28418, WO 97/19927, and Boden et al. (J Med. Chem. 39: 1664-75, 1996), can be used with or linked to the compounds of the disclosure.

Norepinephrine-serotonin reuptake inhibitors such as milnacipran and related compounds described in WO 03/077897 A1, can be used with or linked to the compounds of the disclosure.

Vanilloid receptor antagonists such as arvanil and related compounds described in WO 01/64212 A1, can be used with or linked to the compounds of the disclosure.

The compounds can be used in combination therapy with a phosphodiesterase inhibitor (examples of such inhibitors can be found in U.S. Pat. No. 6,333,354).

The compounds can be used alone or in combination therapy to treat disorders associated with chloride or bicarbonate secretion that may lead to constipation, e.g., Cystic Fibrosis.

The compounds can also or alternatively be used alone or in combination therapy to treat calcium-induced constipation effects. Constipation is commonly found in the geriatric population, particularly patients with osteoporosis who have to take calcium supplements. Calcium supplements have shown to be beneficial in ostoporotic patients to restore bone density but compliance is poor because of constipation effects associated therewith.

The compounds of the current disclosure have can be used in combination with an opioid. Opioid use is mainly directed to pain relief, with a notable side-effect being GI disorder, e.g. constipation. These agents work by binding to opioid receptors, which are found principally in the central nervous system and the gastrointestinal tract. The receptors in these two organ systems mediate both the beneficial effects, and the undesirable side effects (e.g. decrease of gut motility and ensuing constipation). Opioids suitable for use typically belong to one of the following exemplary classes: natural opiates, alkaloids contained in the resin of the opium poppy including morphine, codeine and thebaine; semi-synthetic opiates, created from the natural opioids, such as hydromorphone, hydrocodone, oxycodone, oxymorphone, desomorphine, diacetylmorphine (Heroin), nicomorphine, dipropanoylmorphine, benzylmorphine and ethylmorphine; fully synthetic opioids, such as fentanyl, pethidine, methadone, tramadol and propoxyphene; endogenous opioid peptides, produced naturally in the body, such as endorphins, enkephalins, dynorphins, and endomorphins.

The compound of the disclosure can be used alone or in combination therapy to alleviate GI disorders encountered with patients with renal failure (stage 3-5). Constipation is the second most reported symptom in that category of patients (Murtagh et al., 2006; Murtagh et al., 2007a; Murtagh et al., 2007b). Without being held by theory, it is believed that kidney failure is accompanied by a stimulation of intestinal Na re-absorption (Hatch and Freel, 2008). A total or partial inhibition of such transport by administration of the compounds of the disclosure can have a therapeutic benefit to improve GI transit and relieve abdominal pain. In that context, the compounds of the disclosure can be used in combination with Angiotensin-modulating agents: Angiotensin Converting Enzyme (ACE) inhibitors (e.g. captopril, enalopril, lisinopril, ramipril) and Angiotensin II receptor antagonist therapy (also referred to as ATi-antagonists or angiotensin receptor blockers, or ARB's); diuretics such as loop diuretics (e.g. furosemide, bumetanide), Thiazide diuretics (e.g. hydrochlorothiazide, chlorthalidone, chlorthiazide) and potassium-sparing diuretics: amiloride; beta blockers: bisoprolol, carvedilol, nebivolol and extended-release metoprolol; positive inotropes: Digoxin, dobutamine; phosphodiesterase inhibitors such as milrinone; alternative vasodilators: combination of isosorbide dinitrate/hydralazine; aldosterone receptor antagonists: spironolactone, eplerenone; natriuretic peptides: Nesiritide, a recombinant form of brain-natriuretic peptide (B P), atrial -natriuretic peptide (ANP); vasopressin receptor antagonists: Tolvaptan and conivaptan; phosphate binder (Renagel, Renleva, Phoslo, Fosrenol); phosphate transport inhibitor such as those described in US 4806532, US 6355823, US 6787528, WO 2001/005398, WO 2001/087294, WO 2001/082924, WO 2002/028353, WO 2003/048134, WO 2003/057225, US 7119120, EP 1465638, US Appl. 2007/021509, WO 2003/080630, US 7109184, US Appl. 2006/0280719 , EP 1485391, WO 2004/085448, WO 2004/085382, US Appl. 2006/0217426, JP 2007/131532, or phosphate transport antagonist (Nicotinamide).

The compounds of the disclosure can be used in combination with peptides or peptide analogs that activate the Guanylate Cyclase-receptor in the intestine and results in elevation of the intracellular second messenger, or cyclic guanosine monophosphate (cGMP), with increased chloride and bicarbonate secretion into the intestinal lumen and concomitant fluid secretion. Example of such peptides are Linaclotide (MD-1100 Acetate), endogenous hormones guanylin and uroguanylin and enteric bacterial peptides of the heat stable enterotoxin family (ST peptides) and those described in US 5140102, US 5489670, US 5969097, WO 2006/001931 A2, WO 2008/002971 A2, WO 2008/106429 A2, US 2008/0227685 A1 and US 7041786.

The compounds of the disclosure can be used in combination with type-2 chloride channel agonists, such as Amitiza (Lubiprostone) and other related compounds described in US 6414016.

The compounds of the disclosure can be used in combination with P2Y2 receptor agonists, such as those described in EP 1196396 B1 and US 6624150.

The compounds of the disclosure can be used in combination with laxative agents such as bulk-producing agents, e.g. psyllium husk (Metamucil), methylcellulose (Citrucel), polycarbophil, dietary fiber, apples, stool softeners/surfactant such as docusate (Colace, Diocto); hydrating agents (osmotics), such as dibasic sodium phosphate, magnesium citrate, magnesium hydroxide (Milk of magnesia), magnesium sulfate (which is Epsom salt), monobasic sodium phosphate, sodium biphosphate; hyperosmotic agents: glycerin suppositories, sorbitol, lactulose, and polyethylene glycol (PEG). The compounds of the disclosure can be also be used in combination with agents that stimulate gut peristalsis, such as Bisacodyl tablets (Dulcolax), Casanthranol, Senna and Aloin, from Aloe Vera.

In one embodiment, the compounds of the disclosure accelerate gastrointestinal transit, and more specifically in the colon, without substantially affecting the residence time in the stomach, i.e. with no significant effect on the gastric emptying time. Even more specifically the compounds of the invention restore colonic transit without the side-effects associated with delayed gastric emptying time, such as nausea. The GI and colonic transit are measured in patients using methods reported in, for example: Burton DD, Camilleri M, Mullan BP, et al., J. Nucl. Med., 1997;38: 1807-1810; Cremonini F, Mullan BP, Camilleri M, et al., Aliment. Pharmacol. Ther., 2002; 16: 1781-1790; Camilleri M, Zinsmeister AR, Gastroenterology, 1992; 103 :36-42; Bouras EP, Camilleri M, Burton DD, et al., Gastroenterology, 2001;120:354-360; Coulie B, Szarka LA, Camilleri M, et al., Gastroenterology, 2000; 119:41-50; Prather CM, Camilleri M, Zinsmeister AR, et al., Gastroenterology, 2000;118:463-468; and, Camilleri M, McKinzie S, Fox J, et al., Clin. Gastroenterol. Hepatol., 2004;2:895-904.

### C. Polymer Combination Therapy

The NHE-inhibiting compounds described therein may be administered to patients in need thereof in combination with a fluid-absorbing polymer ("FAP"). The intestinal fluid-absorbing polymers useful for administration in accordance with embodiments of the present disclosure may be administered orally in combination with non-absorbable NHE-inhibiting compounds (e.g., a NHE-3 inhibitor) to absorb the intestinal fluid resulting from the action of the sodium transport inhibitors. Such polymers swell in the colon and bind fluid to impart a consistency to stools that is acceptable for patients. The fluid-absorbing polymers described herein may be selected from polymers with laxative properties, also referred to as bulking agents (i.e., polymers that retain some of the intestinal fluid in the stools and impart a higher degree of hydration in the stools and facilitate transit). The fluid-absorbing polymers may also be optionally selected from pharmaceutical polymers with anti-diarrhea function, i.e., agents that maintain some consistency to the stools to avoid watery stools and potential incontinence.

The ability of the polymer to maintain a certain consistency in stools with a high content of fluid can be characterized by its "water holding power." Wenzl et al. (in Determinants of decreased fecal consistency in patients with diarrhea; Gastroenterology, v. 108, no. 6, p. 1729-1738 (1995)) studied the determinants that control the consistency of stools of patients with diarrhea and found that they were narrowly correlated with the water holding power of the feces. The water holding power is determined as the water content of given stools to achieve a certain level of consistency (corresponding to "formed stool" consistency) after the reconstituted fecal matter has been centrifuged at a certain g number. Without being held to any particular theory, has been found that the water holding power of the feces is increased by ingestion of certain polymers with a given fluid absorbing profile. More specifically, it has been found that the water-holding power of said polymers is correlated with their fluid absorbancy under load (AUL); even more specifically the AUL of said polymers is greater than 15 g of isotonic fluid/g of polymer under a static pressure of 5kPa, even more preferably under a static pressure of 10 kPa .

The FAP utilized in the treatment method of the present disclosure preferably has a AUL of at least about 10 g, about 15 g, about 20 g, aboug 25 g or more of isotonic fluid/g of polymer under a static pressure of about 5 kPa, and preferably about 10 kPA, and may have a fluid absorbency of about 20 g, about 25 g or more, as determined using means generally known in the art. Additionally or alternatively, the FAP may impart a minimum consistency to fecal matter and, in some embodiments, a consistency graded as "soft" in the scale described in the test method below, when fecal non water-soluble solid fraction is from 10% to 20%, and the polymer concentration is from 1% to 5% of the weight of stool. The determination of the fecal non water-soluble solid fraction of stools is described in Wenz et al. The polymer may be uncharged or may have a low charge density (e.g., 1-2 meq/gr). Alternatively or in addition, the polymer may be delivered directly to the colon using known delivery methods to avoid premature swelling in the esophagus.

In one embodiment of the present disclosure, the FAP is a "superabsorbent" polymer (i.e., a lightly crosslinked, partially neutralized polyelectrolyte hydrogel similar to those used in baby diapers, feminine hygiene products, agriculture additives, etc.). Superabsorbent polymers may be made of a lightly crosslinked polyacrylate hydrogel. The swelling of the polymer is driven essentially by two effects: (i) the hydration of the polymer backbone and entropy of mixing and (ii) the osmotic pressure arising from the counter-ions (e.g., Na ions) within the gel. The gel swelling ratio at equilibrium is controlled by the elastic resistance inherent to the polymer network and by the chemical potential of the bathing fluid, i.e., the gel will de-swell at higher salt concentration because the background electrolyte will reduce the apparent charge density on the polymer and will reduce the difference of free ion concentrations inside and outside the gel that drives osmotic pressure. The swelling ratio SR (g of fluid per g of dry polymer and synonymously "fluid absorbency") may vary from 1000 in pure water down to 30 in 0.9% NaCl solution representative of physiological saline (i.e., isotonic). SR may increase with the degree of neutralization and may decrease with the crosslinking density. SR generally decreases with an applied load with the extent of reduction dependent on the strength of the gel, i.e., the crosslinking density. The salt concentration within the gel, as compared with the external solution, may be lower as a result of the Donnan effect due to the internal electrical potential.

The fluid-absorbing polymer may include crosslinked polyacrylates which are fluid absorbent such as those prepared from α,β-ethylenically unsaturated monomers, such as monocarboxylic acids, polycarboxylic acids, acrylamide and their derivatives. These polymers may have repeating units of acrylic acid, methacrylic acid, metal salts of acrylic acid, acrylamide, and acrylamide derivatives (such as 2-acrylamido-2-methylpropanesulfonic acid) along with various combinations of such repeating units as copolymers. Such derivatives include acrylic polymers which include hydrophilic grafts of polymers such as polyvinyl alcohol. Examples of suitable polymers and processes, including gel polymerization processes, for preparing such polymers are disclosed in U.S. Pat. Nos. 3,997,484; 3,926,891; 3,935,099; 4,090,013; 4,093,776; 4,340,706; 4,446,261; 4,683,274; 4,459,396; 4,708,997; 4,076,663; 4, 190,562; 4,286,082; 4,857,610; 4,985,518; 5,145,906; 5,629,377 and 6,908,609 (in addition, see Buchholz, F. L. and Graham, A. T., "Modern Superabsorbent Polymer Technology," John Wiley & Sons (1998)). A class of preferred polymers for treatment in combination with NHE-inhibitors is poly electrolytes.

The degree of crosslinking can vary greatly depending upon the specific polymer material; however, in most applications the subject superabsorbent polymers are only lightly crosslinked, that is, the degree of crosslinking is such that the polymer can still absorb over 10 times its weight in physiological saline (i.e., 0.9% saline). For example, such polymers typically include less than about 0.2 mole % crosslinking agent.

In some embodiments, the FAP's utilized for treatment are Calcium Carbophil (Registry Number: 9003-97-8, also referred as Carbopol EX-83), and Carpopol 934P.

In some embodiments, the fluid-absorbing polymer is prepared by high internal phase emulsion ("HIPE") processes. The HIPE process leads to polymeric foam slabs with a very large porous fraction of interconnected large voids (about 100 microns) (i.e., open-cell structures). This technique produces flexible and collapsible foam materials with exceptional suction pressure and fluid absorbency (see U.S. Patent Nos. 5,650,222; 5,763,499 and 6,107,356). The polymer is hydrophobic and, therefore, the surface should be modified so as to be wetted by the aqueous fluid. This is accomplished by post-treating the foam material by a surfactant in order to reduce the interfacial tension. These materials are claimed to be less compliant to loads, i.e., less prone to de-swelling under static pressure.

In some embodiments, fluid-absorbing gels are prepared by aqueous free radical polymerization of acrylamide or a derivative thereof, a crosslinker (e.g., methylene-bis-acrylamide) and a free radical initiator redox system in water. The material is obtained as a slab. Typically the swelling ratio of crosslinked polyacrylamide at low crosslinking density (e.g., 2%-4% expressed as weight % of methylene-bis-acrylamide) is between 25 and 40 (F. Horkay, Macromolecules, 22, pp. 2007-09 (1989)). The swelling properties of these polymers have been extensively studied and are essentially the same of those of crosslinked polyacrylic acids at high salt concentration. Under those conditions, the osmotic pressure is null due to the presence of counter-ions and the swelling is controlled by the free energy of mixing and the network elastic energy. Stated differently, a crosslinked polyacrylamide gel of same crosslink density as a neutralized polyacrylic acid will exhibit the same swelling ratio (i.e., fluid absorbing properties) and it is believed the same degree of deswelling under pressure, as the crosslinked polyelectrolyte at high salt content (e.g., 1 M). The properties (e.g., swelling) of neutral hydrogels will not be sensitive to the salt environment as long as the polymer remains in good solvent conditions. Without being held to any particular theory, it is believed that the fluid contained within the gel has the same salt composition than the surrounding fluid (i.e., there is no salt partitioning due to Donnan effect).

Another subclass of fluid-absorbing polymers that may be utilized is hydrogel materials that include N-alkyl acrylamide polymers (e.g., N-isopropylacrylamide (NIP AM)). The corresponding aqueous polyNIPAM hydrogel shows a temperature transition at about 35°C. Above this temperature the hydrogel may collapse. The mechanism is generally reversible and the gel re-swells to its original swelling ratio when the temperature reverts to room temperature. This allows production of nanoparticles by emulsion polymerization (R. Pelton, Advances in Colloid and Interface Science, 85, pp. 1-33, (2000)). The swelling characteristics of poly-NIPAM nanoparticles below the transition temperature have been reported and are similar to those reported for bulk gel of polyNIPAM and equivalent to those found for polyacrylamide (i.e. 30-50 g/g) (W. McPhee, Journal of Colloid and Interface Science, 156, pp. 24-30 (1993); and, K. Oh, Journal of Applied Polymer Science, 69, pp. 109-114 (1997)).

In some embodiments, the FAP utilized for treatment in combination with a NHE-inhibitor is a superporous gel that may delay the emptying of the stomach for the treatment of obesity (J. Chen, Journal of Controlled Release, 65, pp. 73-82 (2000), or to deliver proteins. Polyacrylate-based SAP's with a macroporous structure may also be used. Macroporous SAP and superporous gels differ in that the porous structure remains almost intact in the dry state for superporous gels, but disappears upon drying for macroporous SAP's. The method of preparation is different although both methods use a foaming agent (e.g., carbonate salt that generates C0 2bubbles during polymerization). Typical swelling ratios, SR, of superporous materials are around 10. Superporous gels keep a large internal pore volume in the dry state.

Macroporous hydrogels may also be formed using a method whereby polymer phase separation in induced by a non-solvent. The polymer may be poly-NIPAM and the non-solvent utilized may be glucose (see, e.g., Z. Zhang, J. Org. Chem., 69, 23 (2004)) or NaCl (see, e.g., Cheng et al., Journal of Biomedical Materials Research - Part A, Vol. 67, Issue 1, 1 October 2003, Pages 96-103). The phase separation induced by the presence of NaCl leads to an increase in swelling ratio. These materials are preferred if the swelling ratio of the material, SR, is maintained in salt isotonic solution and if the gels do not collapse under load. The temperature of "service" should be shifted beyond body temperature, e.g. by diluting NIP AM in the polymer with monomer devoid of transition temperature phenomenon.

In some embodiments, the fluid-absorbing polymer may be selected from certain naturally-occurring polymers such as those containing carbohydrate moieties. In a preferred embodiment, such carbohydrate-containing hydrogels are non-digestible, have a low fraction of soluble material and a high fraction of gel-forming materials. In some embodiments, the fluid-absorbing polymer is selected from xanthan, guar, wellan, hemicelluloses, alkyl-cellulose, hydro-alkyl-cellulose, carboxy-alkyl-cellulose, carrageenan, dextran, hyaluronic acid and agarose. In a preferred embodiment, the gel forming polymer is psyllium. Psyllium (or "ispaghula") is the common name used for several members of the plant genus *Plantago* whose seeds are used commercially for the production of mucilage. Most preferably, the fluid-absorbing polymer is in the gel-forming fraction of psyllium, i.e., a neutral saccharide copolymer of arabinose (25%) and xylose (75%) as characterized in (J. Marlett, Proceedings of the Nutrition Society, 62, pp. 2-7-209 (2003); and, M. Fischer, Carbohydrate Research, 339, 2009-2012 (2004)), and further described in U.S. Pat. Nos. 6,287,609; 7,026,303; 5,126, 150; 5,445,831; 7,014,862; 4,766,004; 4,999,200, and over-the-counter psillium-containing agents such as those marketed under the name Metamucil (The Procter and Gamble company). Preferably the a psyllium- containing dosage form is suitable for chewing, where the chewing action disintegrates the tablet into smaller, discrete particles prior to swallowing but which undergoes minimal gelling in the mouth, and has acceptable mouthfeel and good aesthetics as perceived by the patient.

The psyllium-containing dosage form includes physically discrete unit suitable as a unitary dosage for human subjects and other mammals, each containing a predetermined quantity of active material (e.g. the gel-forming polysaccharide) calculated to produce the desired therapeutic effect. Solid oral dosage forms that are suitable for the present compositions include tablets, pills, capsules, lozenges, chewable tablets, troches, cachets, pellets, wafer and the like.

In some embodiments, the FAP is a polysaccharide particle wherein the polysaccharide component includes xylose and arabinose. The ratio of the xylose to the arabinose may be at least about 3 : 1 by weight, as described in U.S. Pat. Nos. 6,287,609; 7,026,303 and 7,014,862.

The fluid-absorbing polymers described herein may be used in combination with the NHE-inhibiting compound or a pharmaceutical composition containing it. The NHE-inhibiting compound and the FAP may also be administered with other agents including those described under the heading "Combination Therapies" without departing from the scope of the present disclosure. As described above, the NHE-inhibiting compound may be administered alone without use of a fluid-absorbing polymer to resolve symptoms without eliciting significant diarrhea or fecal fluid secretion that would require the co-administration of a fluid-absorbing polymer.

The fluid-absorbing polymers described herein may be selected so as to not induce any substantial interaction with the NHE-inhibiting compound or a pharmaceutical composition containing it. As used herein, "no substantial interaction" generally means that the co-administration of the FAP polymer would not substantially alter (i.e., neither substantially decrease nor substantially increase) the pharmacological property of the NHE-inhibiting compounds administered alone. For example, FAPs containing negatively charged functionality, such as carboxylates, sulfonates, and the like, may potentially interact ionically with positively charged NHE-inhibiting compounds, preventing the inhibitor from reaching its pharmacological target. In addition, it may be possible that the shape and arrangement of functionality in a FAP could act as a molecular recognition element, and sequestor NHE-inhibiting compounds via "host-guest" interactions via the recognition of specific hydrogen bonds and/or hydrophobic regions of a given inhibitor. Accordingly, in various embodiments of the present disclosure, the FAP polymer may be selected, for co-administration or use with a compound of the present disclosure, to ensure that (i) it does not ionically interact with or bind with the compound of the present disclosure (by means of, for example, a moiety present therein possessing a charge opposite that of a moiety in the compound itself), and/or (ii) it does not possess a charge and/or structural conformation (or shape or arrangement) that enables it to establish a "host-guest" interaction with the compound of the present disclosure (by means of, for example, a moiety present therein that may act as a molecular recognition element and sequester the NHE inhibitor or inhibiting moiety of the compound).

### D. Dosage

It is to be noted that, as used herein, an "effective amount" (or "pharmaceutically effective amount") of a compound disclosed herein, is a quantity that results in a beneficial clinical outcome of the condition being treated with the compound compared with the absence of treatment. The amount of the compound or compounds administered will depend on the degree, severity, and type of the disease or condition, the amount of therapy desired, and the release characteristics of the pharmaceutical formulation. It will also depend on the subject's health, size, weight, age, sex and tolerance to drugs. Typically, the compound is administered for a sufficient period of time to achieve the desired therapeutic effect.

In embodiments wherein both an NHE-inhibitor compound and a fluid-absorbing polymer are used in the treatment protocol, the NHE-inhibiting compound and FAP may be administered together or in a "dual-regimen" wherein the two therapeutics are dosed and administered separately. When the NHE-inhibiting compound and the fluid- absorbing polymer are dosed separately, the typical dosage administered to the subject in need of the NHE-inhibiting compound is typically from about 5 mg per day and about 5000 mg per day and, in other embodiments, from about 50 mg per day and about 1000 mg per day. Such dosages may induce fecal excretion of sodium (and its accompanying anions), from about 10 mmol up to about 250 mmol per day, from about 20 mmol to about 70 mmol per day or even from about 30 mmol to about 60 mmol per day.

The typical dose of the fluid-absorbing polymer is a function of the extent of fecal secretion induced by the non-absorbable NHE-inhibiting compound. Typically the dose is adjusted according to the frequency of bowel movements and consistency of the stools. More specifically the dose is adjusted so as to avoid liquid stools and maintain stool consistency as "soft" or semi-formed, or formed. To achieve the desired stool consistency and provide abdominal relief to patients, typical dosage ranges of the fluid-absorbing polymer to be administered in combination with the NHE-inhibiting compound, are from about 2 g to about 50 g per day, from about 5 g to about 25 g per day or even from about 10 g to about 20 g per day. When the NHE-inhibiting compound and the FAP are administered as a single dosage regimen, the daily uptake may be from about 2 g to about 50 g per day, from about 5 g to about 25 g per day, or from about 10 g to about 20 g per day, with a weight ratio of NHE-inhibiting compound to fluid- absorbing polymer being from about 1 : 1000 to 1 : 10 or even from about 1 :500 to 1 :5 or about 1 : 100 to 1 :5.

A typical dosage of the substantially impermeable or substantially systemically non-bioavailable, NHE-inhibiting compound when used alone without a FAP may be between about 0.2 mg per day and about 2 g per day, or between about 1 mg and about 1 g per day, or between about 5 mg and about 500 mg, or between about 10 mg and about 250 mg per day, which is administered to a subject in need of treatment.

The frequency of administration of therapeutics described herein may vary from once-a-day (QD) to twice-a-day (BID) or thrice-a-day (TID), etc., the precise frequency of administration varying with, for example, the patient's condition, the dosage, etc. For example, in the case of a dual-regimen, the NHE-inhibiting compound could be taken once-a-day while the fluid-absorbing polymer could be taken at each meal (TID). Furthermore, as disclosed in U.S. Application No. 61/584,753 filed January 9, 2012, the NHE-inhibiting compound is administered twice-a-day (BID), or thrice-a-day (TID), and in a more specific embodiment, the NHE-inhibiting compound is administered in an amount ranging from 2-200 mg per dose BID, or 2-100 mg per dose TID. In more specific embodiments, the NHE-inhibiting compound is administered in an amount of about 15 mg per dose, about 30 mg per dose, or about 45 mg per dose, and in a more specific embodiment, in an amount of 15 mg per dose, 30 mg per dose, or 45 mg per dose.

### E. Modes of Administration

The substantially impermeable or substantially systemically non- bioavailable NHE-inhibiting compounds of the present disclosure with or without the fluid-absorbing polymers described herein may be administered by any suitable route. The compound is preferably administrated orally (e.g., dietary) in capsules, suspensions, tablets, pills, dragees, liquids, gels, syrups, slurries, and the like. Methods for encapsulating compositions (such as in a coating of hard gelatin or cyclodextran) are known in the art (Baker, et al., "Controlled Release of Biological Active Agents", John Wiley and Sons, 1986). The compounds can be administered to the subject in conjunction with an acceptable pharmaceutical carrier as part of a pharmaceutical composition. The formulation of the pharmaceutical composition will vary according to the route of administration selected. Suitable pharmaceutical carriers may contain inert ingredients which do not interact with the compound. The carriers are biocompatible, i.e., non-toxic, non-inflammatory, non-immunogenic and devoid of other undesired reactions at the administration site. Examples of pharmaceutically acceptable carriers include, for example, saline, commercially available inert gels, or liquids supplemented with albumin, methyl cellulose or a collagen matrix. Standard pharmaceutical formulation techniques can be employed, such as those described in Remington's Pharmaceutical Sciences, Mack Publishing Company, Easton, Pa.

Pharmaceutical preparations for oral use can be obtained by combining a compound of the present disclosure with a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents can be added, such as crosslinked polyvinyl pyrrolidone, agar, or alginic acid or a salt thereof such as sodium alginate.

Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include push-fit capsules made of a suitable material, such as gelatin, as well as soft, sealed capsules made of a suitable material, for example, gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

It will be understood that, certain compounds of the disclosure may be obtained as different stereoisomers (e.g., diastereomers and enantiomers) or as isotopes and that the disclosure includes all isomeric forms, racemic mixtures and isotopes of the disclosed compounds and a method of treating a subject with both pure isomers and mixtures thereof, including racemic mixtures, as well as isotopes. Stereoisomers can be separated and isolated using any suitable method, such as chromatography.

### F. Delayed Release

HE proteins show considerable diversity in their patterns of tissue expression, membrane localization and functional roles. (See, e.g., The sodium-hydrogen exchanger - From molecule To Its Role In Disease, Karmazyn, M., Avkiran, M., and Fliegel, L., eds., Kluwer Academics (2003).)

In mammals, nine distinct NHE genes (NHE-1 through -9) have been described. Of these nine, five (NHE-1 through -5) are principally active at the plasma membrane, whereas NHE-6, -7 and -9 reside predominantly within intracellular compartments.

NHE-1 is ubiquitously expressed and is chiefly responsible for restoration of steady state intracellular pH following cytosolic acidification and for maintenance of cell volume. Recent findings show that NHE-1 is crucial for organ function and survival (e.g., NHE-1-null mice exhibit locomotor abnormalities, epileptic-like seizures and considerable mortality before weaning).

In contrast with NHE-1 expressed at the basolateral side of the nephrons and gut epithelial cells, NHE-2 through -4 are predominantly expressed on the apical side of epithelia of the kidney and the gastrointestinal tract. Several lines of evidence show that NHE-3 is the major contributor of renal bulk Na+ and fluid re-absorption by the proximal tubule. The associated secretion of H+ by NHE-3 into the lumen of renal tubules is also essential for about 2/3 of renal HCO₃⁻ re-absorption. Complete disruption of NHE-3 function in mice causes a sharp reduction in HCO₃⁻, Na+ and fluid re-absorption in the kidney, which is consistently associated with hypovolemia and acidosis.

In one embodiment, the compounds of the disclosure are intended to target the apical NHE antiporters (e.g. NHE-3, NHE-2 and NHE-8) without substantial permeability across the layer of gut epithelial cells, and/or without substantial activity towards NHEs that do not reside predominantly in the GI tract. This invention provides a method to selectively inhibit GI apical NHE antiporters and provide the desired effect of salt and fluid absorption inhibition to correct abnormal fluid homeostasis leading to constipations states. Because of their absence of systemic exposure, said compounds do not interfere with other key physiological roles of NHEs highlighted above. For instance, the compounds of the disclosure are expected to treat constipation in patients in need thereof, without eliciting undesired systemic effects, such as for example salt wasting or bicarbonate loss leading to hyponatriemia and acidosis among other disorders.

In another embodiment, the compounds of the disclosure are delivered to the small bowel with little or no interaction with the upper GI such as the gastric compartment and the duodenum. The applicant found that an early release of the compounds in the stomach or the duodenum can have an untoward effect on gastric secretion or bicarbonate secretion (also referred to as "bicarbonate dump"). In this embodiment the compounds are designed so as to be released in an active form past the duodenum. This can be accomplished by either a prodrug approach or by specific drug delivery systems.

As used herein, "prodrug" is to be understood to refer to a modified form of the compounds detailed herein that is inactive (or significantly less active) in the upper GI, but once administered is metabolised in vivo into an active metabolite after getting past, for example, the duodenum. Thus, in a prodrug approach, the activity of the NHE- inhibiting compound can be masked with a transient protecting group that is liberated after compound passage through the desired gastric compartment. For example, acylation or alkylation of the essential guanidinyl functionality of the NHE-inhibiting compound would render it biochemically inactive; however, cleavage of these functional groups by intestinal amidases, esterases, phosphatases, and the like, as well enzymes present in the colonic flora, would liberate the active parent compound. Prodrugs can be designed to exploit the relative expression and localization of such phase I metabolic enzymes by carefully optimizing the structure of the prodrug for recognition by specific enzymes. As an example, the antiinflammatory agent sulfasalazine is converted to 5-aminosalicylate in the colon by reduction of the diazo bond by intestinal bacteria.

In a drug delivery approach the NHE-inhibiting compounds of the disclosure are formulated in certain pharmaceutical compositions for oral administration that release the active in the targeted areas of the GI, i.e., jejunum, ileum or colon, or preferably the distal ileum and colon, or even more preferably the colon.

Methods known from the skilled-in-the-art are applicable. (See, e.g., Kumar, P. and Mishra, B., Colon Targeted Drug Delivery Systems - An Overview, Curr. Drug Deliv., 2008, 5 (3), 186-198; Jain, S. K. and Jain, A., Target-specific Drug Release to the Colon., Expert Opin. Drug Deliv., 2008, 5 (5), 483-498; Yang, L., Biorelevant Dissolution Testing of Colon-Specific Delivery Systems Activated by Colonic Microflora, J. Control Release, 2008, 125 (2), 77-86; Siepmann, F.; Siepmann, J.; Walther, M.; MacRae, R. J.; and Bodmeier, R., Polymer Blends for Controlled Release Coatings, J. Control Release 2008, 725 (1), 1-15; Patel, M.; Shah, T.; and Amin, A., Therapeutic Opportunities in Colon-Specific Drug-Delivery Systems, Crit. Rev. Ther. Drug Carrier Syst., 2007, 24 (2), 147-202; Jain, A.; Gupta, Y.; Jain, S. K., Perspectives of Biodegradable Natural Polysaccharides for Site-specific Drug Delivery to the Colon., J. Pharm. Set, 2007, 10 (1), 86-128; Van den, M. G., Colon Drug Delivery, Expert Opin. Drug Deliv., 2006, 3 (1), 111-125; Basit, A. W., Advances in Colonic Drug Delivery, Drugs 2005, 65 (14), 1991-2007; Chourasia, M. K.; Jain, S. K., Polysaccharides for Colon-Targeted Drug Delivery, Drug Deliv. 2004, 11 (2), 129-148; Shareef, M. A.; Khar, R. K.; Ahuja, A.; Ahmad, F. J.; and Raghava, S., Colonic Drug Delivery: An Updated Review, AAPS Pharm. Sci. 2003, 5 (2), E17; Chourasia, M. K.; Jain, S. K., Pharmaceutical Approaches to Colon Targeted Drug Delivery Systems, J. Pharm. Sci. 2003, 6 (1), 33-66; and, Sinha, V. R.; Kumria, R., Colonic Drug Delivery: Prodrug Approach, Pharm. Res. 2001, 18 (5), 557- 564. Typically the active pharmaceutical ingredient (API) is contained in a tablet / capsule designed to release said API as a function of the environment (e.g., pH, enzymatic activity, temperature, etc.), or as a function of time. One example of this approach is Eudracol™ (Pharaia Polymers EBusiness Line of Degussa's Specialty Acrylics Business Unit), where the API-containing core tablet is layered with various polymeric coatings with specific dissolution profiles. The first layer ensures that the tablet passes through the stomach intact so it can continue through the small intestine. The change from an acidic environment in the stomach to an alkaline environment in the small intestine initiates the release of the protective outer layer. As it travels through the colon, the next layer is made permeable by the alkalinity and intestinal fluid. This allows fluid to penetrate to the interior layer and release the active ingredient, which diffuses from the core to the outside, where it can be absorbed by the intestinal wall. Other methods are contemplated without departing from the scope of the present disclosure.

In another example, the pharmaceutical compositions of the invention can be used with drug carriers including pectin and galactomannan, polysaccharides that are both degradable by colonic bacterial enzymes. (See, e.g., U.S. Pat. No. 6,413,494) While pectin or galactomannan, if used alone as a drug carrier, are easily dissolved in simulated gastric fluid and simulated intestinal fluid, a mixture of these two polysaccharides prepared at a pH of about 7 or above produces a strong, elastic, and insoluble gel that is not dissolved or disintegrated in the simulated gastric and intestinal fluids, thus protecting drugs coated with the mixture from being released in the upper GI tract. When the mixture of pectin and galactomannan arrives in the colon, it is rapidly degraded by the synergic action of colonic bacterial enzymes. In yet another aspect, the compositions of the invention may be used with the pharmaceutical matrix of a complex of gelatin and an anionic polysaccharide (e.g., pectinate, pectate, alginate, chondroitin sulfate, polygalacturonic acid, tragacanth gum, arabic gum, and a mixture thereof), which is degradable by colonic enzymes (U.S. Pat. No. 6,319,518).

In yet other embodiments, fluid-absorbing polymers that are administered in accordance with treatment methods of the present disclosure are formulated to provide acceptable/pleasant organoleptic properties such as mouthfeel, taste, and/or to avoid premature swelling/gelation in the mouth and in the esophagus and provoke choking or obstruction. The formulation may be designed in such a way so as to ensure the full hydration and swelling of the FAP in the GI tract and avoid the formation of lumps. The oral dosages for the FAP may take various forms including, for example, powder, granulates, tablets, wafer, cookie and the like, and are most preferably delivered to the small bowel with little or no interaction with the upper GI such as the gastric compartment and the duodenum.

The above-described approaches or methods are some of the many methods reported to selectively deliver an active in the lower part of the intestine.

### IV. Preparation of Compounds

The following Reaction Schemes I-IV illustrate methods for making compounds of this invention, i.e., compounds of Formula (I): 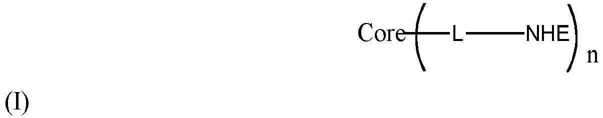 or a pharmaceutically acceptable salt thereof, wherein Core, L and NHE are as defined above. In general reaction schemes I-IV, R₁, R₂, R₃, R₄ and R₅ are as defined above, PG is defined as a protecting group and LG is a leaving group. It is understood that one skilled in the art may be able to make these compounds by similar methods or by combining other methods known to one skilled in the art. It is also understood that one skilled in the art would be able to make, in a similar manner as described below, other compounds of Formula (I) not specifically illustrated below by using the appropriate starting components and modifying the parameters of the synthesis as needed. In general, starting components may be obtained from sources such as Sigma Aldrich, Lancaster Synthesis, Inc., Maybridge, Matrix Scientific, TCI, and Fluorochem USA, etc. or synthesized according to sources known to those skilled in the art (see, for example, Advanced Organic Chemistry: Reactions, Mechanisms, and Structure, 5th edition (Wiley, December 2000)) or prepared as described in this invention.

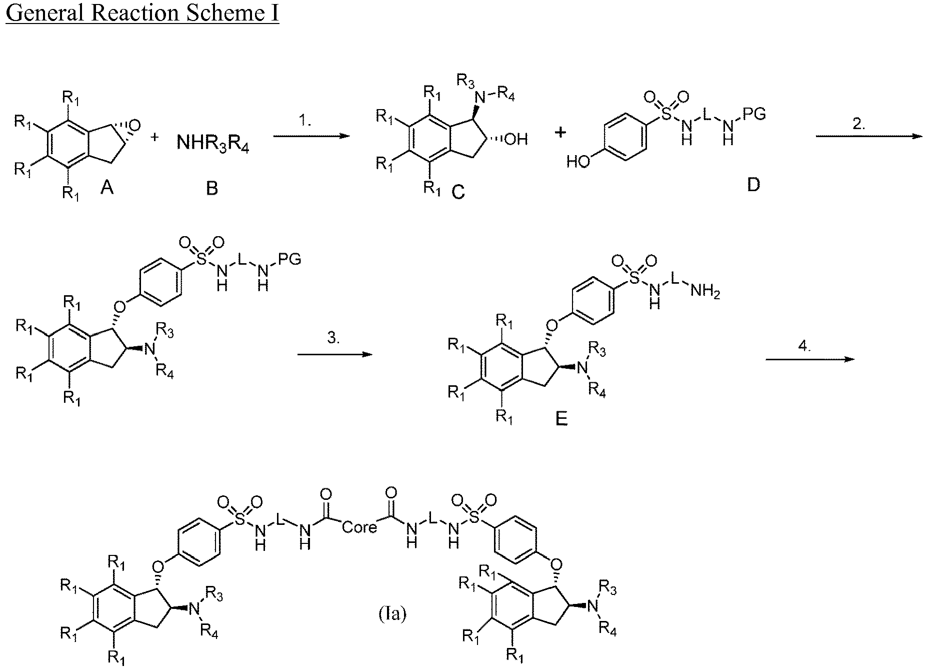

Referring to General Reaction Scheme I, an appropriate indene oxide of structure A can be purchased or prepared according to methods known in the art and combined with an amine (B) to form compounds of the structure C. Compounds of the structure A are chiral and the structures drawn reflect the absolute configuration. Either enantiomer, or a mixture can be used. C may then be reacted with a phenol of structure D in the presence of triphenylphosphine and an azodicarboxylate such as diisopropylazodicarboxylate, diethyl azodicarboxylate or di-tert-butylazodicarboxylate. After removal of the protecting group, compounds of the structure E are reacted with a poly carboxylate or poly isocyanate to give compounds of structure (la).

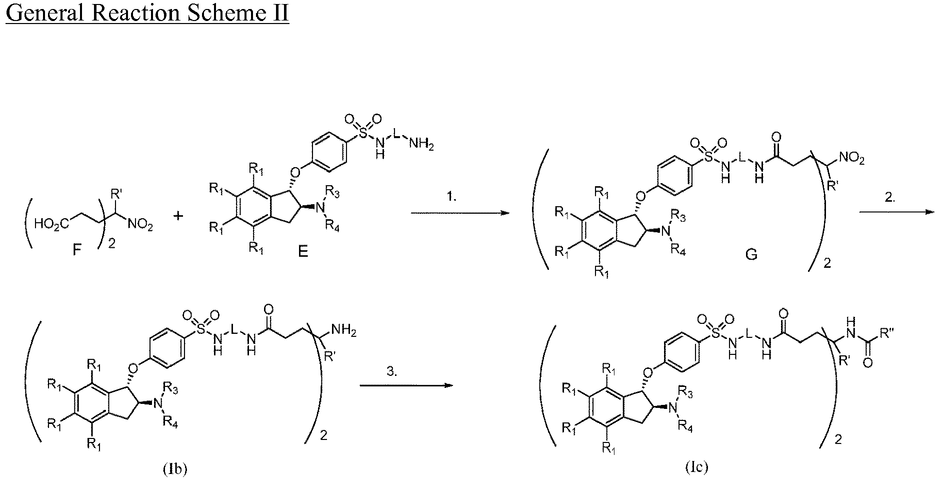

Compounds of Formula (I) may also be prepared according to General Reaction Scheme II. A diacid of the structure F may be reacted with a a compound of the structure E in the presence of a carboxylate activation reagent to give a a dimer of structure G. Reduction of the nitro group of compound G to a compound of structure (lb) may be accomplished with hydrogen and a suitable catalyst such as palladium on carbon or Raney nickel and the like. Compounds of structure (lb) may be converted to compounds of structure (Ic) with an activated carboxylic acid or anhydride.

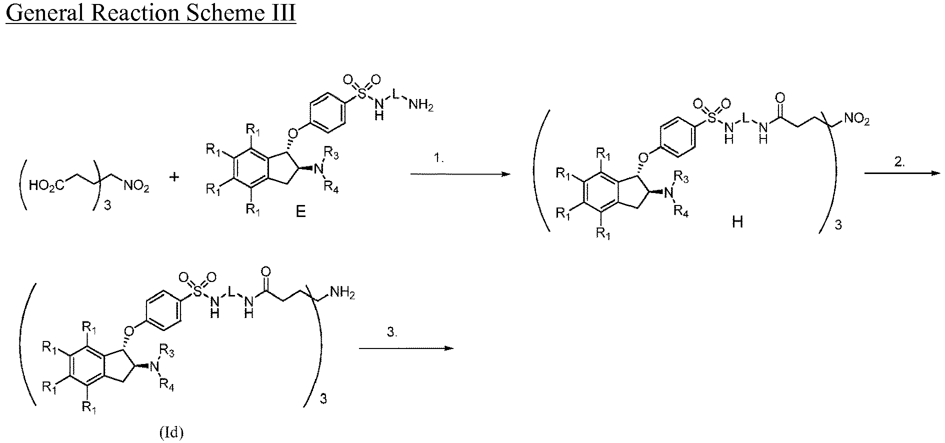

Alternatively, compounds of Formula (I) may be prepared according to General Reaction Scheme III. A compound of structure E may be reacted with a polycarboxylic acid such as 4-(2-carboxyethyl)-4-nitroheptanedioic acid in the presence of a carboxylate activation reagent to give a trimeric nitro compound of structure H. Reduction of the nitro compound H to a compound of structure (Id) may be accomplished with hydrogen and a suitable catalyst such as palladium on carbon or Raney nickel and the like.

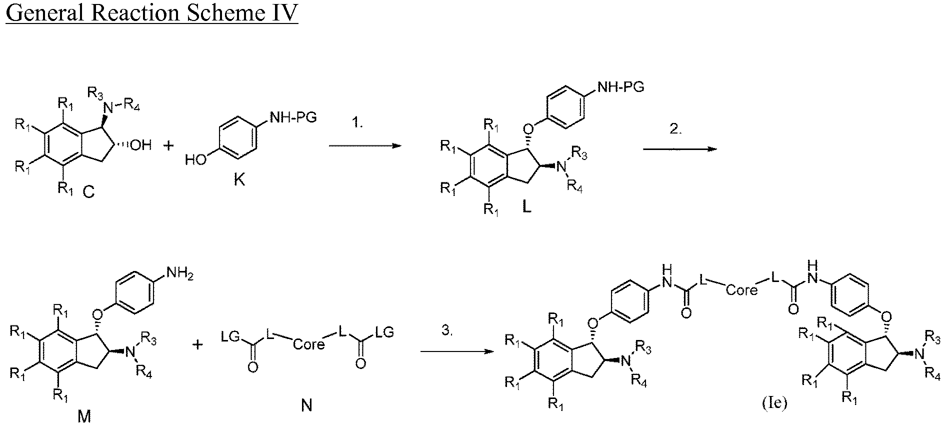

In another embodiment, compounds of Formula (I) are prepared according to General Reaction Scheme IV. An appropriate phenol of structure K is reacted with a compound of structure C in the presence of triphenylphosphine and an azodicarboxylate such as diisopropylazodicarboxylate, diethyl azodicarboxylate or di-*tert*-butylazodicarboxylate to form compounds of structure L, wherein PG is a suitable protecting group. Removal of the protecting group under appropriate conditions gives anilines of the structure M. M is then treated with a compound of the structure N, which has both the core and linking groups present, wherein LG is a leaving group, and an appropriate base to produce compounds of the structure (Ie).

With regard to General Reaction Schemes I-IV, typical carboxylate activation reagents include DCC, EDCI, HATU, oxalyl chloride, thionyl chloride and the like. Typical bases include TEA, DIEA, pyridine, K 2CO₃, NaH and the like. Typical acylation catalysts include HOBt, HOAt, 4-dimethylaminopyridine and the like. Typical catalysts for hydrogenation include palladium on carbon, rhodium on carbon, platinum on carbon, raney nickel and the like.

One skilled in the art will recognize that variations to the order of the steps and reagents discussed in reference to the Reaction Schemes are possible. Methodologies for preparation of compounds of Formula (I) are described in more detail in the following nonlimiting exemplary schemes.

It will also be appreciated by those skilled in the art that in the process described herein the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, *t*-butyldimethylsilyl, *t*-butyldiphenylsilyl or trimethylsilyl), tetrahydropyranyl, benzyl, and the like. Suitable protecting groups for amino, amidino and guanidino include t-butoxycarbonyl, benzyloxycarbonyl, trifluoroacetyl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

The following examples are provided to further illustrate the present disclosure.

### EXAMPLES

### Exemplary Compound Synthesis

### Intermediate A1

### 2(R),3(S)-5,7-dicMoro-1H-indene-2.3 -oxide

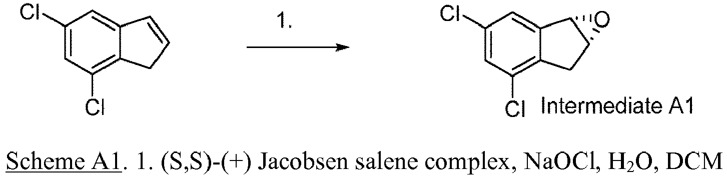

Intermediate **A1**: 2(R), 3(S)-5,7-dichloro-1H-indene-2,3-oxide: 4-(3-Phenylpropyl)pyridine N-oxide (47 mg, 0.22 mmol) was added to a mixture of 5,7-dichloro-1H-indene (1.00 g, 5.40 mmol) and (S,S)-(+) N,N'-bis(3,5-di-fert- butylsalicylidine)-1,2-cyclohexanediaminomanganese (III) chloride (34 mg, 0.054 mmol) in DCM and stirred for 10 minutes. The reaction mixture was cooled to -3 °C and water (1.25 mL) followed by saturated aqueous K₂CO₃ (1.25 mL) was added. Aqueous NaOCl (-5.7% free chlorine, 14.0 mL) was added dropwise over 5 minutes, and then the pH was adjusted to 11-12 by the addition of pH 7.0 phosphate buffer (0.1 M). The mixture was vigorously stirred and warmed from -3 °C to 2 °C over 4 hours. The reaction mixture was extracted with DCM (3 x 25 mL) and the combined organic extracts were washed with 15% Na₂S₂O₃ (20 mL), dried (Na₂SO₄) and concentrated. The residue was purified by flash chromatography on silica gel (20-30% DCM/hexanes) to give the title compound (895 mg) as a light yellow solid. The product was further purified by recrystallization from n-heptane (5 mL) to give the title compound (692 mg) as a white powder. See Jacobsen, E.N.; Zhang, W.; Muci, A.R.; Ecker, J.R.; Deng, L. J. Am. Chem. Soc. 1991, 113, 7063-7064 for Jacobsen epoxidation of indene.

The following intermediates were prepared using the procedure used to make Intermediate A1, substituting the appropriate indene for 5,7-dichloro-1H-indene:

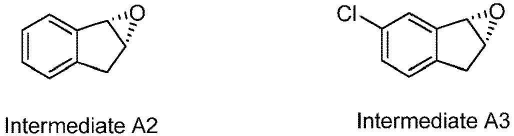

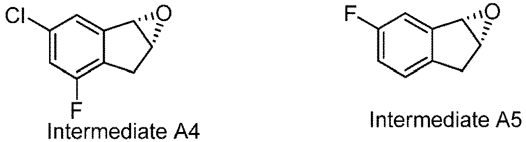

### Chart 1. Epoxide intermediates prepared as described for intermediate A1

The required indenes were made by known methods:
Chiba, Shunsuke; Xu, Yan-Jun; Wang, Yi-Feng; J. Am. Chem. Soc. 2009, 737,(36), 12886 - 12887.

Musso, David L.; Orr, G. Faye; Cochran, Felicia R.; Kelley, James L.; Selph, Jeffrey L.; Rigdon, Greg C; Cooper, Barrett R.; Jones, Michael L. J. Med. Chem. 2003, 46, (3), 409 - 416.

### Intermediate B1

### (R)-3-(tert-butyldiphenylsilyloxy)pyrrolidine

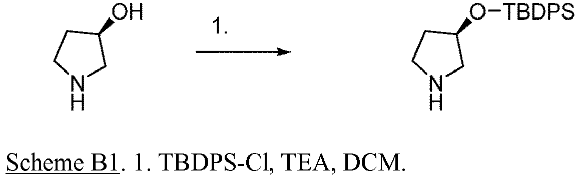

Intermediate **B1** (R)-3-(tert-butyldiphenylsilyloxy)pyrrolidine: To a solution of R-3-pyrrolidinol (815 mg, 9.35 mmol) and imidazole (640 mg, 9.40 mmol) in DCM (20 mL) at 0 °C was added TBDPS-chloride (2.58 g, 9.40 mmol) over 5 minutes. After 1 hour the reaction was warmed to RT and stirred for 3 days. The solvent was removed at reduced pressure, the residue was dissolved in EtOAc (150 mL) and washed with saturated NaHCO₃ (50 mL), water (50 mL) and brine (50 mL), then dried (Na₂SO₄) and concentrated to dryness under vacuum to give the title compound (3.3g) which was used without further purification. MS (*m*/*z*): 326.0 (M+H)⁺.

### Intermediate C1

### tert-butyl (R)-1-((1R,2R)-4,6-dichloro-2-hydroxy-2.3-dihydro-1H-inden-1-yl)piperidin-3-ylcarbamate

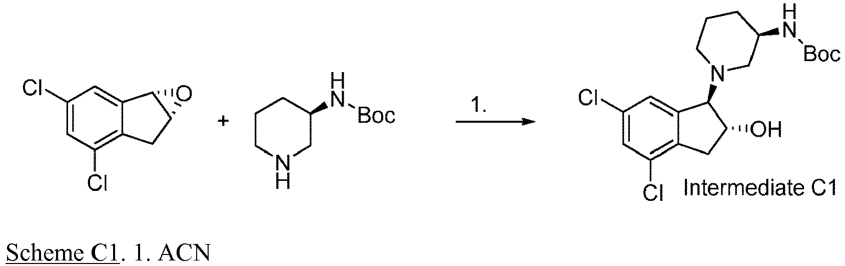

Intermediate **C1**: *tert*-butyl (R)-1-((1R,2R)-4,6-dichloro-2-hydroxy-2,3-dihydro-1H-inden-1-yl)piperidin-3-ylcarbamate: A mixture of 2(R), 3(S)-5,7-dichloro-1H-indene-2,3-oxide (165 mg, 0.82 mmol) and (R)-tert-butyl piperidin-3-ylcarbamate (165 mg, .082 mmol) in ACN (0.55 mL) was heated at 70 °C. After 15 hours, the solvent was removed under vacuum to give the title compound (330 mg) which was used without further purification. MS (*m*/*z*): 401.1 (M+H)⁺.

### Intermediate C2

### (1R,2R)-4.6-dichloro-1-(pyrrolidin-1-yl)-2.3-dihydro-1H-inden-2-ol

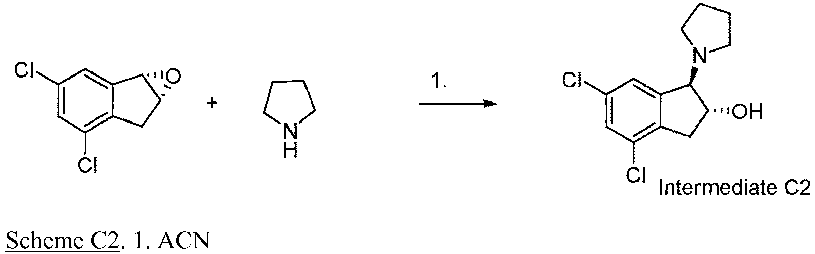

Intermediate **C2**: (1R,2R)-4,6-dichloro-1-(pyrrolidin-1-yl)-2,3-dihydro-1H-inden-2-ol: A mixture of 2(R),3(S)-5,7-dichloro-1H-indene-2,3-oxide (130 mg, 0.65 mmol) and pyrrolidine (69 mg, 0.97 mmol) was heated at 70 °C. After 22 hours, additional pyrrolidine (69 mg, 0.97 mmol) was added and heating at 70 °C was resumed. After 5 hours, the solvent was removed at reduced pressure and the residue was purified by flash chromatography on silica gel (0-100% EtOAc/DCM) to give the title compound (139 mg). MS (*m*/*z*): 272.5 (M+H)⁺.

### Intermediate C3

### (1R,2R)-1-(dimethylamino)-2,3-dihydro-1H-inden-2-ol

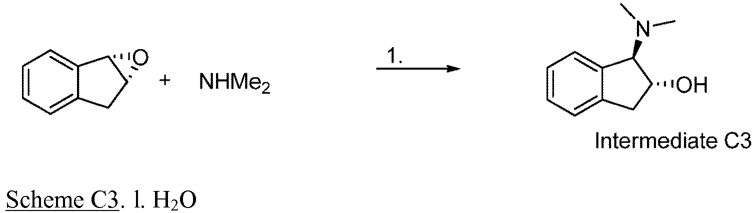

Intermediate **C3**: (1R,2R)-1-(dimethylamino)-2,3-dihydro-1H-inden-2-ol: A mixture of indene oxide (58 mg, 0.44 mmol) and 40% aqueous dimethylamine (0.28 mL) was heated at 50 °C for 90 minutes. After cooling, DCM (5 mL) was added and the mixture was dried (Na₂SO₄) and concentrated. The residue was purified by flash chromatography on silica gel (0-15% MeOH/EtOAc) to give the title compound (71 mg). MS *(m*/*z):* 178.0 (M+H)⁺.

### Intermediate C4

### (1R,2R)-1-((R)-3-(tert-butyldiphenylsilyloxy)pyrrolidin-1-yl)-6-chloro-4-fluoro-2,3-dihydro-1H-inden-2-ol

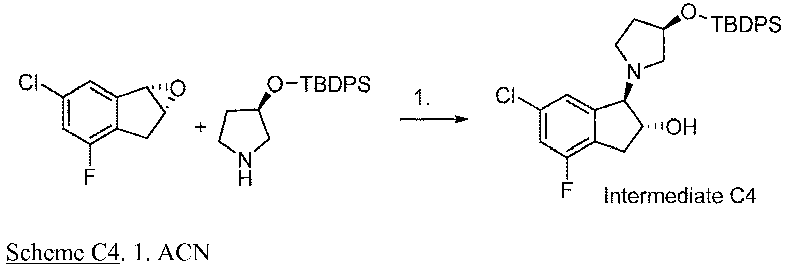

Intermediate **C4**: (1R,2R)-1-((R)-3-(*tert*-butyldiphenylsilyloxy)pyrrolidin-1-yl)-6-chloro-4-fluoro-2,3-dihydro-1H-inden-2-ol: A mixture of 2(R),3(S)-5-chloro-7-fluoro-1H-indene-2,3-oxide (185 mg, 1.0 mmol) and (R)-3-(*tert*-butyldiphenylsilyloxy)pyrrolidine (325 mg, 1.0 mmol) in ACN (2.0 mL) was heated at 70 °C. After 5 hours, the mixture was cooled and concentrated at reduced pressure. The residue was purified by flash chromatography on silica gel (25-60% EtOAc/DCM) to give the title compound (202 mg). MS (m/z): 510.4 (M+H)⁺.

The following intermediates were made by applying the above procedures to the appropriate epoxide and amine:

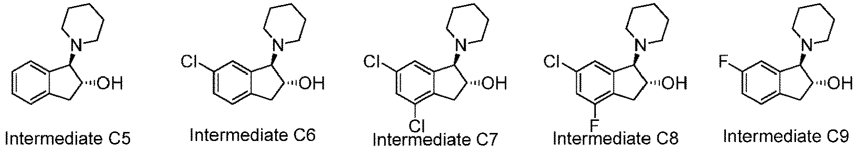

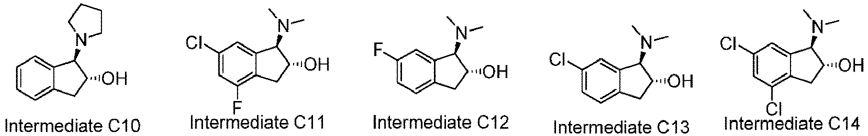

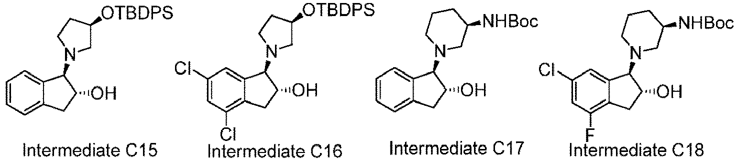

### Chart 2. Intermediates prepared as described for intermediate C1-C4

### Intermediate D1

### tert-butyl 2-(2-(2-(4-hydroxyphenylsulfonamido)ethoxy)ethoxy)ethylcarbamate

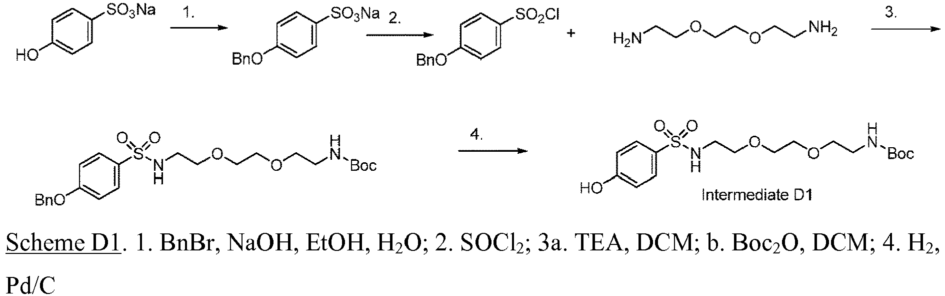

Sodium 4-Phenoxybenzenesulfonate: A mixture of sodium 4-hydroxybenzenesulfonate dihydrate (10.1 g, 43.5 mmol) and sodium hydroxide (1.77 g, 44.3 mmol) in water (22.5 mL) was heated at 50 °C until a clear solution was obtained. A solution of benzyl bromide (7.35 g, 43.0 mmol) in EtOH (18.0 mL) was then added dropwise over 5 minutes. The reaction was heated at 80 °C overnight. The product was collected on a Buchner funnel, washed sequentially with ice water (25 mL), EtOH (25 mL) and MTBE (25 ml) and then dried under vacuum to give the title compound (10.6 g) as a white powder.

4-Phenoxybenzenesulfonyl chloride: A mixture of sodium 4-phenoxybenzenesulfonate (1.00 g, 3.5 mmol), thionyl chloride (6.54 g, 55 mmol) and DMF (50 (µ 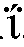 .) was heated at 70 °C for 2 hours. The reaction was cooled and concentrated at reduced pressure. The residue was dissolved in ethyl acetate (75 mL) and washed with water (3 x 25 mL), saturated aqueous NaHCO₃(3 x 25 mL) and brine (25 mL). The organic layer was dried (Na₂SO₄) and concentrated to give the title compound (0.98 g) as a white powder.

*tert*-Butyl 2-(2-(2-(4-(benzyloxy)phenylsulfonamido)ethoxy)ethoxy)-ethylcarbamate: A solution of 4-phenoxybenzenesulfonyl chloride (500 mg, 1.77 mmol) in DCM (5.0 mL) was added dropwise to a solution of 2,2'-(ethane-1,2-diylbis(oxy))diethanamine (1.31 g, 8.84 mmol) and triethylamine (179 mg, 1.77 mmol) in DCM at 0 °C. The ice bath was removed and the reaction stirred at RT for 1 hour. DCM (25 mL) was added and the reaction was washed with water (3 x 25 mL) and brine (25 mL). The organic layer was concentrated and the residue was dissolved in DCM (10 mL). A solution of di-*tert*-butyl dicarbonate (5.45 g, 2.5 mmol) was added and the reaction was stirred for 30 minutes. The reaction mixture was concentrated at reduced pressure and the residue was purified by flash chromatography (50-75% EtOAc/Hexane) to give the title compound (790 mg).

Intermediate **D1**: *tert-*Butyl 2-(2-(2-(4-hydroxyphenylsulfonamido)ethoxy)ethoxy)ethylcarbamate: A mixture of *tert*-butyl 2-(2- (2-(4-(benzyloxy)phenylsulfonamido)ethoxy)ethoxy)ethylcarbamate (790 mg, 1.60 mmol) and 10% Pd/C (160 mg, containing 50% water, 80 mg dry weight) in MeOH (15 mL) was stirred under one atm of H₂ for 1 hour. The reaction mixture was filtered, concentrated and purified by flash chromatography on silica gel (50-80%) EtOAc/hexanes) to give the title compound (660 mg) as a thick oil that solidified on standing. MS (*m*/*z*): 404.7 (M+H)⁺.

### Intermediate D2

### tert-butyl 2-(2-(2-(2-(4-hydroxyphenylsulfonamido)ethoxy)ethoxy)ethoxy)ethylcarbamate

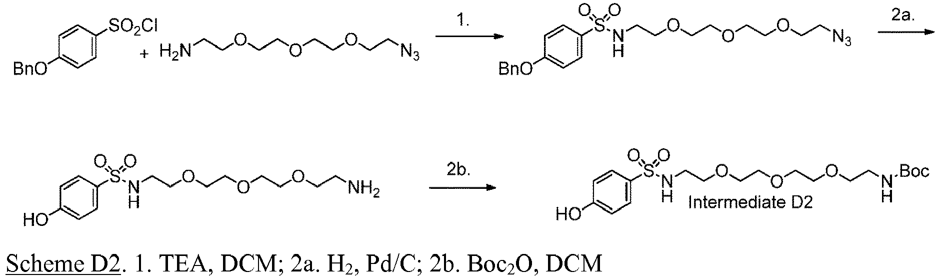

N-(2-(2-(2-(2-azidoethoxy)ethoxy)ethoxy)ethyl)-4-(benzyloxy) benzenesulfonamide: A solution of 4-phenoxybenzenesulfonyl chloride (480 mg, 1.70 mmol) in DCM (5.0 mL) was added dropwise to a solution of 2-(2-(2-(2-azidoethoxy)ethoxy)ethoxy)ethanamine (414 mg, 1.90 mmol) and triethylamine (179 mg, 1.77 mmol) in DCM (5 mL). After 15 minutes, DCM (25 mL) was added and the reaction was washed with water (25 mL) and brine (25 mL), dried (Na₂SO₄) and concentrated. The residue was purified by flash chromatography on silica gel (50-80% EtOAc/hexanes) to give the title compound (625 mg).

Intermediate **D2**: *tert*-Butyl 2-(2-(2-(2-(4-hydroxyphenylsulfonamido)ethoxy)ethoxy)ethoxy)ethylcarbamate: A mixture of N-(2-(2- (2-(2-azidoethoxy)ethoxy)ethoxy)ethyl)-4-(benzyloxy)benzenesulfonamide (625 mg, 1.34 mmol) and 10% Pd/C (150 mg, containing 50% water, 75 mg dry weight) in MeOH (13 mL) was stirred under one atm of H₂ for 4 hour. The reaction mixture was filtered and concentrated at reduced pressure. The residue was dissolved in DCM (5 mL) and a solution of di-*tert*-butyl dicarbonate (305 mg, 1.4 mmol) in DCM (5 mL) was added slowly. After 90 minutes, the reaction mixture was concentrated and the residue was purified by flash chromatography on silica gel (50-100%) EtOAc/DCM) to give the title compound (378 mg). MS (*m*/*z*): 448.7 (M+H)⁺.

### Intermediate D3

### Phenol-3 2,2,2-trifluoro-N-(2-(2-(2-(4-hydroxyphenylsulfonamido)ethoxy)ethoxy)ethyl)acetamide

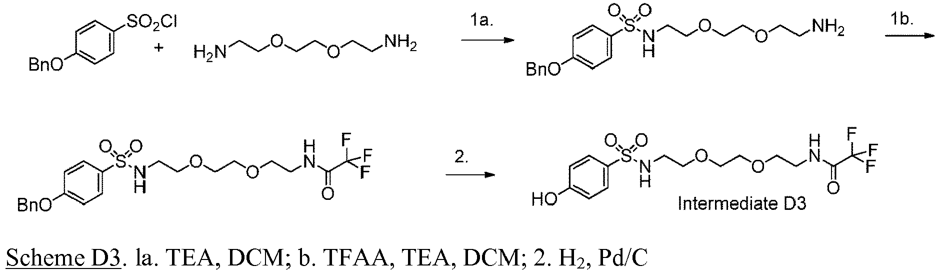

N-(2-(2-(2-(4-(benzyloxy)phenylsulfonamido)ethoxy)ethoxy)ethyl)-2,2,2-trifluoroacetamide: A solution of 4-phenoxybenzenesulfonyl chloride (282 mg, 1.0 mmol) in DCM (5 mL) was added dropwise to a solution of 2,2'-(ethane-1,2-diylbis(oxy))diethanamine (740 mg, 5.0 mmol) in DCM (5 mL). After 30 minutes, the reaction mixture was concentrated at reduced pressure, dissolved in EtOAc (50 mL) and washed with water (4 x 10 mL). The organic layer was dried (Na₂SO₄) and concentrated. The resulting oil was dissolved in DCM (10 mL) and triethylamine (131 mg, 1.30 mmol) at 0 °C. Trifluoroacetic anhydride (252 mg, 1.20 mmol) was added dropwise and the reaction was stirred at 0 °C. After 30 minutes, the reaction mixture was concentrated and purified by flash chromatography on silica gel (50-75% EtOAc/hexanes) to give the title compound (501 mg).

Intermediate **D3**: 2,2,2-trifluoro-N-(2-(2-(2-(4-hydroxyphenylsulfonamido)ethoxy)ethoxy)ethyl)acetamide: A mixture of N-(2-(2-(2-(4-(benzyloxy)phenylsulfonamido)ethoxy)ethoxy)ethyl)-2,2,2-trifluoroacetamide (501 mg, 1.0 mmol) and 10% Pd/C (100 mg, containing 50% water, 50 mg dry weight) in MeOH (7 mL) was stirred under one atm of H₂ for 1 hour. The reaction mixture was then filtered and concentrated at reduced pressure. The residue was purified by flash chromatography (50-100% EtOAc/hexanes) to give the title compound (320 mg). MS (*m*/*z*): 400.9 (M+H)⁺.

### Intermediate E1

### N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide

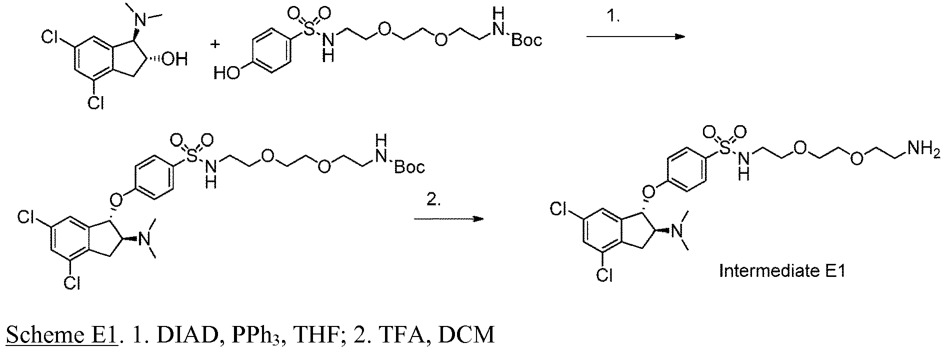

N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide: A solution of diisopropylazodicarboxylate (210 mg, 1.04 mmol) in THF (0.60 mL) was added over 45 minutes to a solution of (1R,2R)-4,6-dichloro-1-(dimethylamino)-2,3-dihydro-1H-inden-2-ol (236 mg, 0.96 mmol), *tert*-butyl 2-(2-(2-(4-hydroxyphenylsulfonamido)ethoxy)ethoxy)ethylcarbamate (360 mmol, 0.90 mmol) and PPh₃ (272 mg, 1.04 mmol) in THF (1.00 mL). After 16 hours, the solvent was removed at reduced pressure and the residue was purified by flash chromatography on silica gel (50- 100% EtOAc/hexanes then 2-10% MeOH/EtOAc to give the title compound (510 mg).

Intermediate **E1** : N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide: N-(2-(2-(2-aminoethoxy)ethoxy)ethyl)-4-((1,2S)-4,6-dichloro-2-(dimethylamino)-2,3-dihydro-1H-inden-1-yloxy)benzenesulfonamide (510 mg, 0.81 mmol) was dissolved in DCM (1.0 mL) and TFA (1.0 mL) was added. After 30 minutes, the solvents were removed at reduced pressure and the residue was purified by reverse phase HPLC (ACN/water/0.1%) TFA). The resulting TFA salt was added to 10% Na₂CO₃ (5 mL) and extracted with DCM (4 x 25 mL). The combined organic extracts were dried and concentrated to give the title compound (295 mg). MS (*m*/*z*): 532.1 (M+H)⁺.

The following intermediates were prepared from the appropriate intermediates C and D using the route shown to make intermediate **E1**:

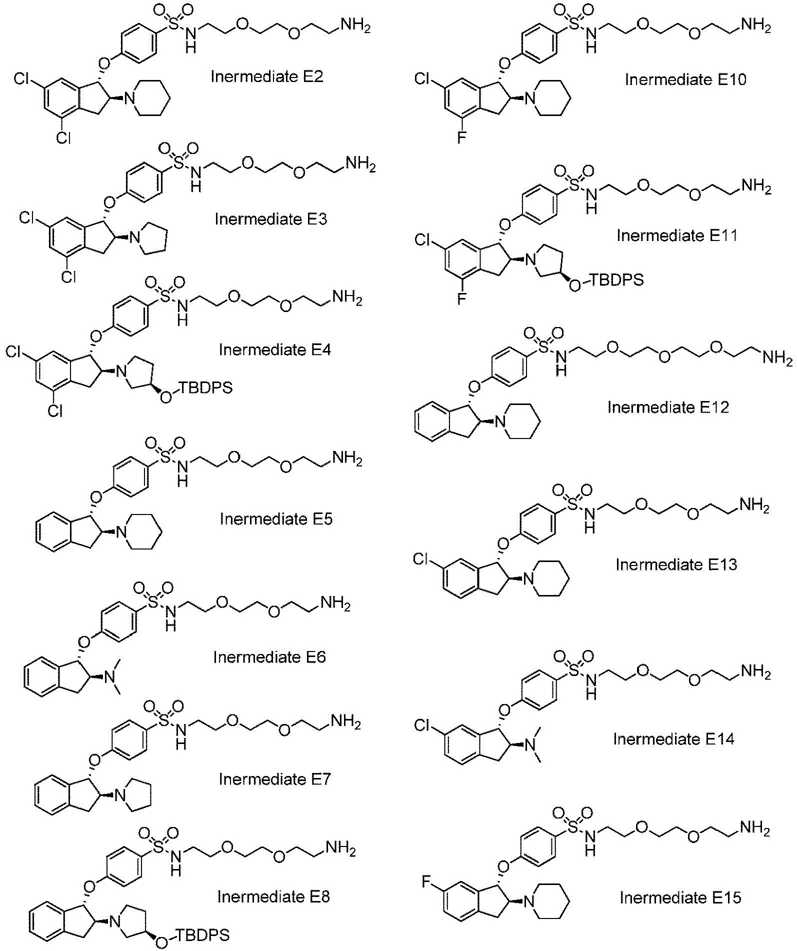

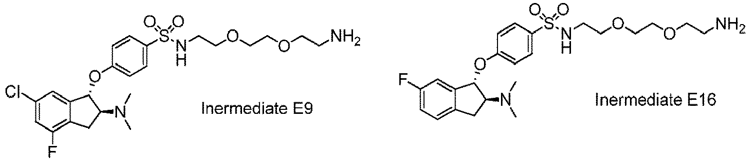

### Chart 3. Intermediates prepared as described for intermediate E1

### Intermediate E17

### tert-butyl(R)1-((1S,2S)-1-(4-(N-(2-(2-(2-aminoethoxy)ethoxy)ethvnsulfamovnphenoxy)-4,6-dichloro-2,3-dihydro-1H-inden-2-yl)piperidin-3-ylcarbamate

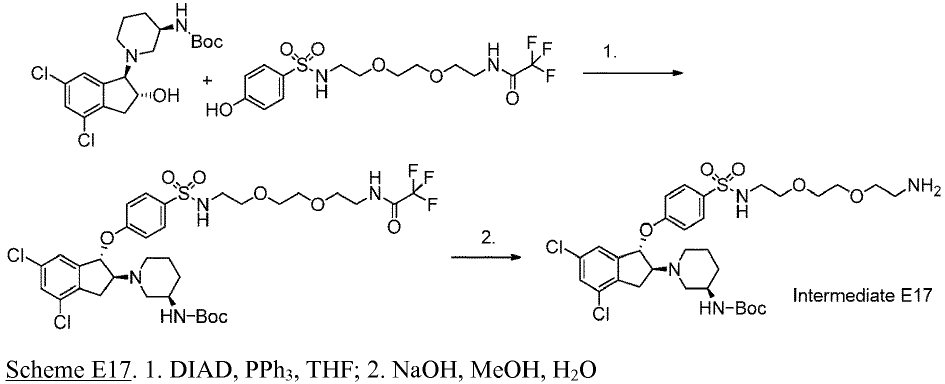

*tert*-butyl (R)-1-((1S,2S)-4,6-dichloro-1-(4-(N-(2-(2-(2-(2,2,2-trifluoroacetamido)ethoxy)ethoxy)ethyl)sulfamoyl)phenoxy)-2,3-dihydro-1H-inden-2-yl)piperidin-3-ylcarbamate: A solution of diisopropylazodicarboxylate (73 mg, 0.36 mmol) in THF (0.30 mL) was added over 30 minutes to a solution of tert-butyl (R)-1-((1R,2R)-4,6-dichloro-2-hydroxy-2,3-dihydro-1H-inden-1-yl)piperidin-3-ylcarbamate (120 mg, 0.30 mmol), 2,2,2-trifluoro-N-(2-(2-(2-(4-hydroxyphenylsulfonamido)ethoxy)ethoxy)ethyl)acetamide (120 mg, 0.30 mmol) and PPh₃ (94 mg, 0.36 mmol) in THF (0.60 mL). After stirring 30 minutes, the solvent was removed at reduced pressure and the residue was purified by flash chromatography on silica gel (50-100% EtOAc/hexanes) to give the title compound (285 mg).

Intermediate **E17**: *tert*-butyl (R)-1-((1S,2S)-1-(4-(N-(2-(2-(2-aminoethoxy)ethoxy)ethyl)sulfamoyl)phenoxy)-4,6-dichloro-2,3-dihydro-1H-inden-2-yl)piperidin-3-ylcarbamate: *tert*-Butyl (R)-1-((1S,2S)-4,6-dichloro-1-(4-(N-(2-(2-(2-(2,2,2-trifluoroacetamido)ethoxy)ethoxy)ethyl)sulfamoyl)phenoxy)-2,3-dihydro-1H-inden-2-yl)piperidin-3-ylcarbamate (285 mg, 0.3 mmol) was dissolved in MeOH (0.9 mL) and 3N NaOH (0.3 mL. 0.9 mmol) was added. After stirring for 1 hour, the solvent was removed at reduced pressure and the residue was purified by reverse phase HPLC (ACN/water/0.1% TFA). The resulting TFA salt was added to 10% Na₂CO₃ (5 mL) and extracted with DCM (3 x 10 mL). The combined organic extracts were dried and concentrated to give the title compound (132 mg). MS (*m*/*z*): 687.1 (M+H)⁺.

The following intermediates were prepared from the intermediate D3 using the route shown to make Intermediate E17:

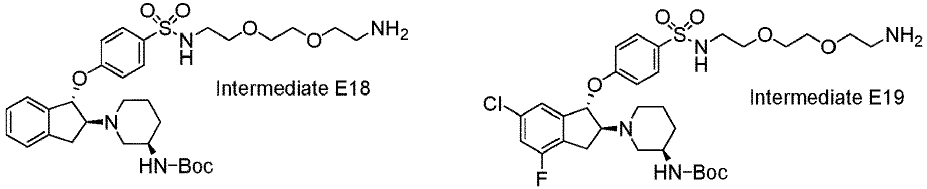

### Intermediate E20

### N-(2-(2-(2-aminoethoxy)ethoxy)ethyl)4-((1S,2S)-4,6-dichloro-2-(dimethylamino)-2,3-dihydro-1H-inden-1-yloxy)-N-methylbenzenesulfonamide

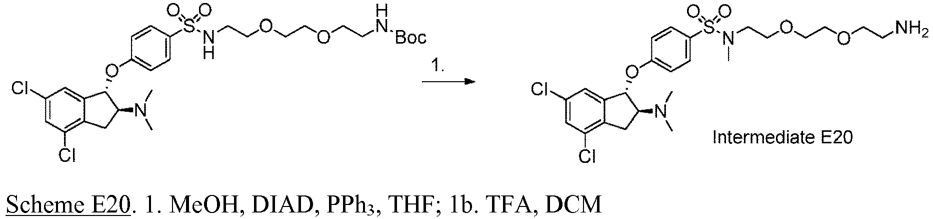

Intermediate **E20**: N-(2-(2-(2-aminoethoxy)ethoxy)ethyl)-4-((1S,2S)-4,6-dichloro-2-(dimethylamino)-2,3-dihydro-1H-inden-1-yloxy)-N-methylbenzenesulfonamide: A solution of DIAD (50 mg, 0.25 mmol) in THF (0.15 mL) was added over 30 minutes to a solution of *tert*-butyl 2-(2-(2-(4-((1S,2S)-4,6-dichloro-2-(dimethylamino)-2,3-dihydro-1H-inden-1-yloxy)phenylsulfonamido)ethoxy)ethoxy)ethylcarbamate (286 mg, 1.32 mmol), MeOH (7.3 mg, 0.23 mmol) and PPh₃ (70 mg, 0.27 mmol) in THF (0.4 mL). After 4 hours additional DIAD (21 mg) was added. After a further 1 hour the reaction mixture was concentrated under vacuum and purified by flash chromatography (12 g SiO₂, 0->100% EtOAc in DCM over 20 minutes) to give the Boc-protected title compound (99 mg). Trifluoroacetic acid (1 mL) was added to a solution of the Boc-protected material in DCM (1.5 mL). After 10 minutes the reaction mixture was concentrated under vacuum and purified by reverse phase HPLC (ACN/water/0.1 % TFA) to give a TFA salt of the title compound (77.2 mg). This salt was diluted in DCM (2 mL) and neutralized with aqueous Na₂CO₃. The aqueous layer was extracted with DCM (8 x 5 mL) and dried over Na₂SO₄ to give the free base of the title compound (60.5 mg). MS: 546.31 (M+H)⁺.

### EXAMPLE 1

### (S,S)-N,N'-(10,17-dioxo-3,6,21,24-tetraoxa-9,11,16,18-tetraazahexacosane-1,26-diyl)bis(4-((1S,2S)-4,6-dichloro-2-(dimethylamine)-2,3-dihydro-1H-inden-1-yloxy)benzenesulfonamide)

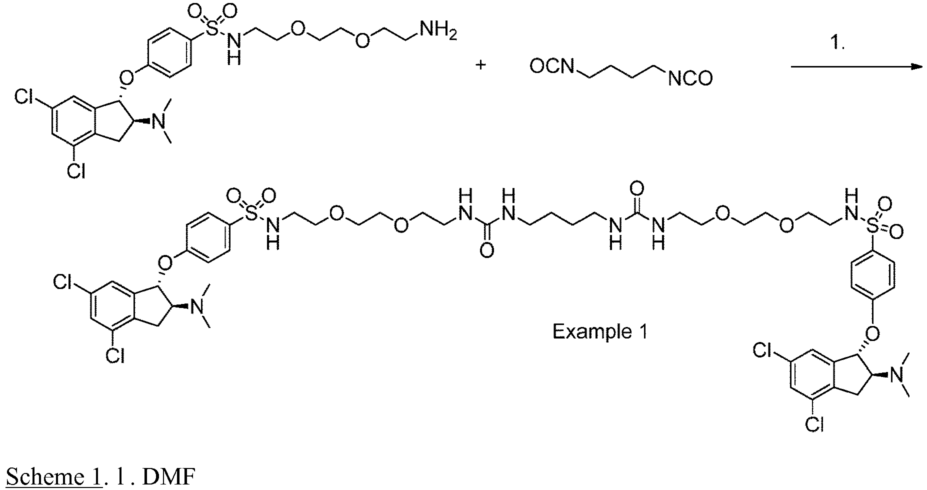

Example **1**: (S,S)-N,N'-(10,17-dioxo-3,6,21,24-tetraoxa-9,11,16,18-tetraazahexacosane-1,26-diyl)bis(4-((1S,2S)-4,6-dichloro-2-(dimethylamino)-2,3-dihydro-1H-inden-1-yloxy)benzenesulfonamide): 1,4-Diisocyanatobutane (5.6 mg, 0.040 mmol) was added to a solution of N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide (43 mg, 0.080 mmol) in DMF (0.80 mL). After 3 hours, the reaction mixture was concentrated under vacuum and purified by reverse phase HPLC (ACN/water/0.1% TFA) to give a TFA salt of the title compound (44 mg). ¹H-NMR (400 MHz, CD₃OD): δ 7.91 (d, *J* = 9.0 Hz, 4H), 7.51 (d, *J* = 1.4 Hz, 2H), 7.35 (d, *J* = 9.0 Hz, 4H), 7.10 (d, *J* = 1 Hz, 2H), 6.45 (d, *J* = 6.6 Hz), 4.41 (dd, *J*_{1,2} = 15.5 Hz, *J*_{1,3}= 8.6 Hz, 2H), 6.65 (dd, *J*_{1,2}= 16.7 Hz, *J*_{1,3}= 8.7 Hz, 2H), 3.57-3.50 (m, 8H), 3.48 (t, *J* = 5.3 Hz, 8H), 3.27-3.19 (m, 6H), 3.07 (m, 8H), 3.02 (s, 12H), 1.44 (m, 4H). MS (*m*/*z*): 1203.0 (M+H)⁺.

### EXAMPLE 2

### (S,S)-N,N'-(2,2'-(2,2'-(2,2-(1,4-phenylenebis(azanediyl))bis(oxomethylene)bis(azanediyl)bis(ethane-2,1-diyl))bis(oxy)bis(ethane-2,1-diyl))bis(oxy)bis(ethane-2,1-diyl))bis(4-((1S,2S)-4,6-dichloro-2-(dimethylamino)-2,3-dihydro-1H-inden-1-yloxy)benzenesulfonamide)

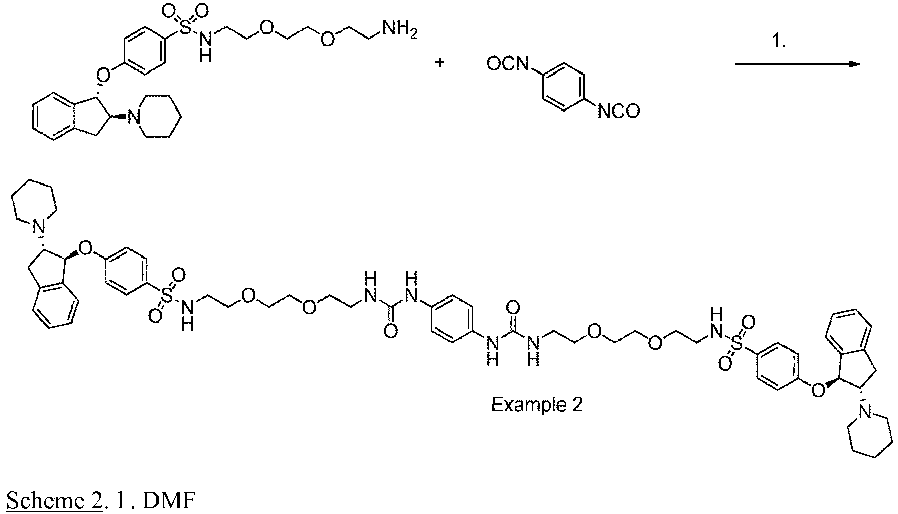

Example **2**: (S,S)-N,N'-(2,2'-(2,2'-(2,2-(1,4-phenylenebis(azanediyl))bis(oxomethylene)bis(azanediyl)bis(ethane-2,1-diyl))bis(oxy)bis(ethane-2,1-diyl))bis(oxy)bis(ethane-2,1-diyl))bis(4-((1S,2S)-4,6-dichloro-2-(dimethylamino)-2,3-dihydro-1H-inden-1-yloxy)benzenesulfonamide): 1,4-Diisocyanatobenzene (5.9 mg, 0.037 mmol) was added to a solution of N-(2-(2-(2-aminoethoxy)ethoxy)ethyl)-4-((1S,2S)-2-(piperidin-1-yl)-2,3-dihydro-1H-inden-1-yloxy)benzenesulfonamide (39 mg, 0.073 mmol) in DMF (0.40 mL). After 40 minutes, the reaction mixture was concentrated under vacuum and purified by reverse phase HPLC (ACN/water/0.1% TFA) to give a TFA salt of the title compound (29 mg). ¹H-NMR. (400 MHz, CD₃OD) δ 7.87 (d, *J* = 8.4 Hz, 2H). 7.44 - 7.32 (m, 3H), 7.29 (d, *J* = 8.7 Hz, 2H), 7.26 - 7.12 (m, 5H), 6.34 (d, *J* = 6.3 Hz, 1H), 4.26 (dd, *J* = 15.0, 8.2 Hz, 2H), 3.67 - 3.57 (m, 10H), 3.57 - 3.49 (m, 6H), 3.46 (t, *J* = 5.4 Hz, 4H), 3.33 (t, *J* = 5.2 Hz, 3H), 3.26 - 3.17 (m, 2H), 3.16 - 3.09 (m, 2H), 3.05 (t, *J* = 5.4 Hz, 3H), 2.04 - 1.64 (m, 7H), 1.53 (s, 2H). MS 1255.21 (M+H)⁺.

### EXAMPLE 3

### (S,S,R)-N,N'-(10,17-dioxo-3,6,21,24-tetraoxa-9,11,16,18-tetraazahexacosane-1,26-diyl)bis(4-(Y1S,2S)-2-((R)-3-aminopiperidin-1-yl)-4,6-dichloro-2,3-dihydro-1H-inden-1-yloxy)benzenesulfonamide)

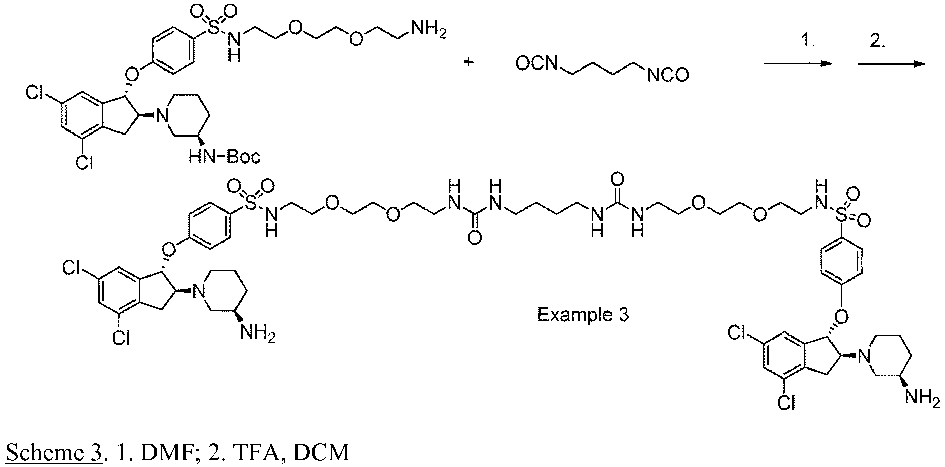

Example **3**: (S,S,R)-N,N'-(10,17-dioxo-3,6,21,24-tetraoxa-9,11,16,18-tetraazahexacosane-1,26-diyl)bis(4-((1S,2S)-2-((R)-3-aminopiperidin-1-yl)-4,6-dichloro-2,3-dihydro-1H-inden-1-yloxy)benzenesulfonamide): 1,4-Diisocyanatobutane (6.7 mg, 0.048 mmol) was added to a solution of *tert*-butyl (R)-1-((1S,2S)-1-(4-(N-(2-(2-(2-aminoethoxy)ethoxy)ethyl)sulfamoyl)phenoxy)-4,6-dichloro-2,3-dihydro-1H-inden-2-yl)piperidin-3-ylcarbamate (66 mg, 0.096 mmol) in DMF (0.90 mL). After 30 minutes, the solvent was concentrated under vacuum. The residue was dissolved in DCM (0.5 mL) and TFA (0.5 mL) was added. After 30 minutes, the solvents were removed under reduced pressure and the residue was purified by reverse phase HPLC (ACN/water/0.1% TFA) to give a TFA salt of the title compound (55 mg). ¹H-NMR (400 MHz, CD₃OD): δ 7.86 (d, *J* = 9.0 Hz, 4H), 7.42 (d, *J* = 1.8 Hz, 2H), 7.29 (d, *J* = 9.0 Hz, 4H), 7.12 (d, *J* = 1.2 Hz, 2H), 6.12 (d, *J* = 5.7 Hz), 3.80 (dd, *J*_{1,2}= 13.5 Hz, *J*_{1,3}= 7.6 Hz, 2H), 3.57-3.51 (m, 8H), 3.48 (t, *J* = 5.7 Hz, 8H), 3.39 (m, 2H), 3.31 (m, 2H), 3.28-3.25 (m, 4H), 3.09-3.00 (m, 12H), 2.84 (m, 2H), 2.73-2.66 (m, 4H), 1.96-1.86 (m, 4H), 1.72-1.68 (m, 4H), 1.60-1.57 (m, 4H), 1.44 (m, 4H). MS (*m*/*z*): 1313.3 (M+H)⁺.

### EXAMPLE 4

### (S,S,R)-N,N'-(10,17-dioxo-3,6,21,24-tetraoxa-9,11,16,18-tetraazahexacosane-1,26-diyl)bis(4-((1S,2S)-6-chloro-4-fluoro-2-((R)-3-hydroxypyrrolidin-1-yl)-2,3-dihvdro-1H-inden-1-yloxy)benzenesulfonamide)

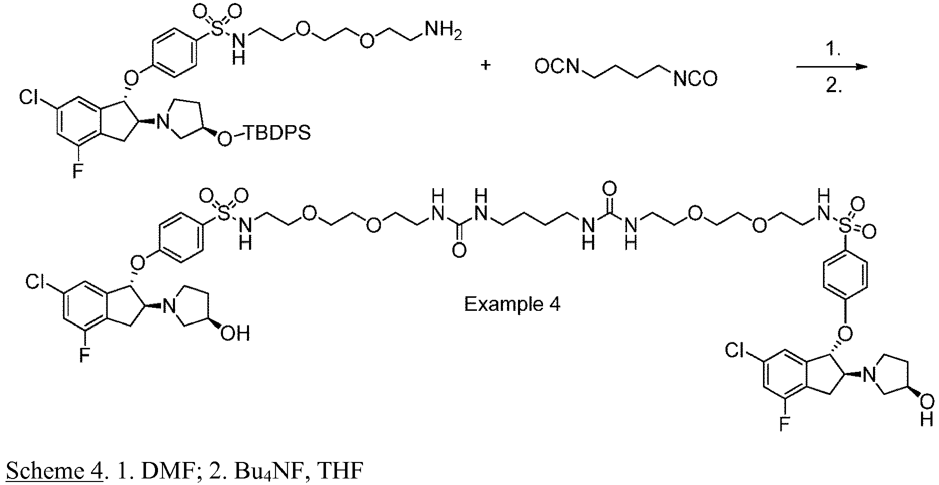

Example **4**: (S,S,R)-N,N'-(10,17-dioxo-3,6,21,24-tetraoxa-9,11,16,18-tetraazahexacosane-1,26-diyl)bis(4-((1S,2S)-6-chloro-4-fluoro-2-((R)-3-hydroxypyrrolidin-1-yl)-2,3-dihvdro-1H-inden-1-yloxy)benzenesulfonamide): 1,4- Diisocyanatobutane (11 mg, 0.080 mmol) was added to a solution of N-(2-(2-(2-aminoethoxy)ethoxy)ethyl)-4-(1S,2S)-2-((R)-3-*tert*-butyl)-6-chloro-4-fluoro-2,3-dihydro-1H-inden-1-yloxy)benzenesulfonamide (249 mg, 0.31 mmol) in DMF (2.0 mL). After 2 hours, the reaction was concentrated under vacuum and the residue was purified by reverse phase HPLC (ACN/water/0.1% TFA). The resulting TFA salt was added to 10% Na₂CO₃ (10 mL) and extracted with DCM (3 x 15 mL). The combined organic phases were dried (Na₂SO₄) and concentrated to give the intermediate free base (105 mg). The free base was dissolved in THF (0.6 mL) and 1M Bu₄NF in THF (0.3 mL, 0.3 mmol) was added. After stirring for 3 hours, the reaction mixture was added to DCM (50 mL), washed with saturated Na₂CO₃ (25 mL) and water (2 x 25 mL), dried (Na₂SO₄) and concentrated. The residue was purified by reverse phase HPLC (ACN/water/0.1% TFA) to give a TFA salt of the title compound (64 mg). ¹H-NMR (400 MHz, CD₃OD): δ 7.91 (d, *J* = 9.0 Hz, 4H), 7.34 (d, *J* = 9.0 Hz, 4H), 7.26 (dd, *J*_{1,2} = 8.6 Hz, *J*_{1,3} =1.4 Hz, 2H), 7.00 (s, 2H), 6.40 (d, *J* = 6.8 Hz, 2H), 4.56 (s, 2H), 4.44 (m, 2H), 3.71-3.65 (m, 6H), 3.57-3.50 (m, 10H), 3.49-3.44 (m, 10H), 3.28-3.24 (m, 4H), 3.21-3.13 (m, 2H), 3.11-3.05 (m, 8H), 2.30 (br s, 2H), 2.09-2.04 (m, 2H), 1.44 (m, 4H). MS (m/z): 1255.4 (M+H)⁺.

### EXAMPLE 5

### (2R,3R)-N1,N4-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-2,3-dihydroxysuccinamide

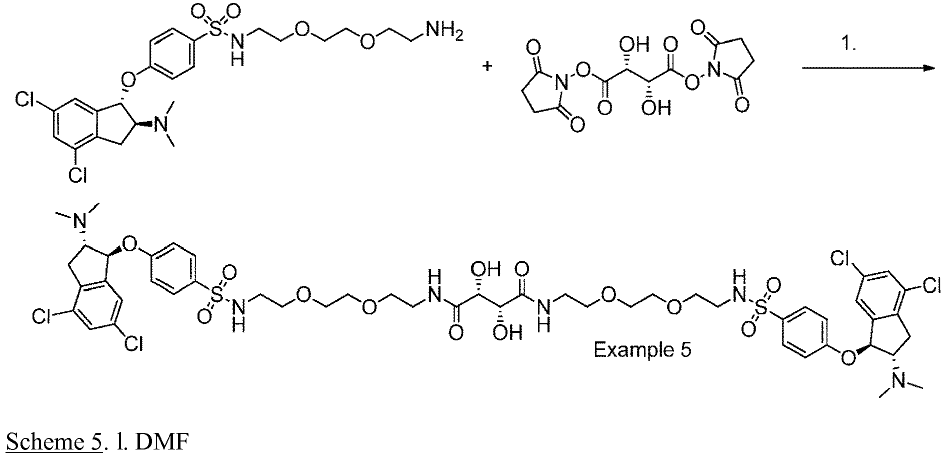

Example **5**: ((2R,3R)-N1,N4-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-2,3-dihydroxysuccinamide: L-Disuccinimidyl tartrate (12.7 mg, 0.037 mmol) was added to a solution of N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide (39 mg, 0.073 mmol) in DMF (0.40 mL). After 1 hour a second portion of L-disuccinimidyl tartrate (5.6 mg) was added, followed by a third portion of L-DST (2.0 mg). After an additional 30 minutes, the reaction mixture was concentrated under vacuum and purified by reverse phase HPLC (ACN/water/0.1% TFA) to give a TFA salt of the title compound (26 mg). ¹H-NMR. (400 MHz, CD₃OD) δ 7.91 (d, *J* = 8.7 Hz, 2H), 7.50 (s, 1H), 7.34 (d, *J* = 8.8 Hz, 2H), 7.09 (s, 1H), 6.43 - 6.37 (m, 1H), 4.44(s, 1H). 4.37 - 4.27 (m, 1H), 3.66 - 3.58 (m, 1H). 3.58 - 3.51 (m, 4H), 3.47 (dd, *J* = 10.9, 5.7 Hz, 4H), 3.40 (s, 1H), 3.22 - 3, 16 (m, 1H), 3.09 (dd, *J* = 11.1, 5.6 Hz, 3H), 2.96 (s, 6H), 2.01 (s, 1H). MS 1177.3 (M+H)⁺.

### EXAMPLE 6

### N1,N4-bis(2-(2-(2-(4-((1S,2S)-2-(piperidin-1-yl)-2,3-dihydro-1H-inden-1-yloxy)phenylsulfonamido)ethoxy)ethoxy)ethyl)terephthalamide

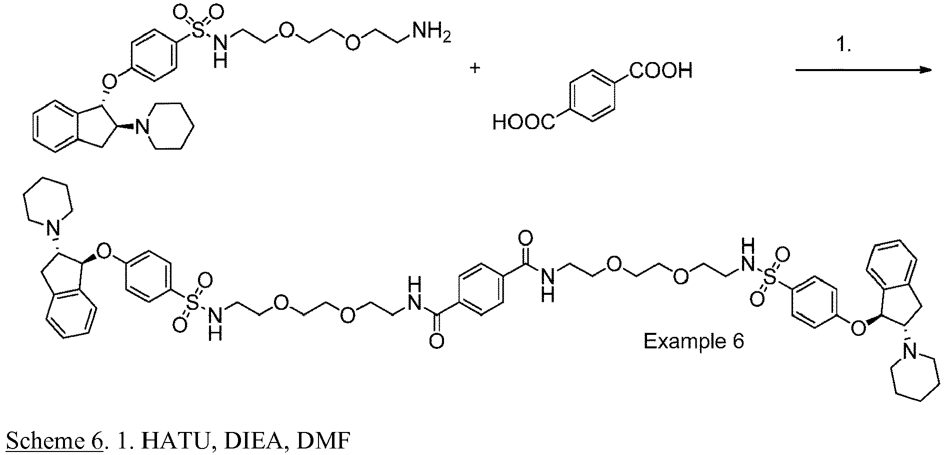

Example **6**: N1,N4-bis(2-(2-(2-(4-((1S,2S)-2-(piperidin-1-yl)-2,3-dihydro-1H-inden-1- yloxy)phenylsulfonamido)ethoxy)ethoxy)ethyl)terephthalamide: HATU (24.7 mg, 0.065 mmol) was added to a solution of N-(2-(2-(2-aminoethoxy)ethoxy)ethyl)-4-((1S,2S)-2-(piperidin-1-yl)-2,3-dihydro-1H-inden-1-yloxy)benzenesulfonamide (30 mg, 0.059 mmol), terephthalic acid (5.0 mg, 0.030 mmol), and DIEA (8.4 mg, 0.065 mmol) in DMF (0.3 mL). After 45 minutes, the reaction mixture was concentrated under vacuum and purified by reverse phase HPLC (ACN/water/0.1% TFA) to give a TFA salt of the title compound (21 mg). ¹H-NMR. (400 MHz, CD₃OD) δ 8.54 (1, *J* = 6.3 Hz, 1H), 7.85 (t, *J* = 4.5 Hz, 4H), 7.40 - 7.34 (m, 2H), 7.29 (d, *J* = 9.0 Hz, 2H), 7.28 -7.13 (m, 2H), 6.34 (d, *J* = 6.3 Hz, IH), 4.31 (dd, *J* = 14.8, 8.3 Hz, 1H), 3.73 - 3.62 (m, 4H), 3.62 - 3.50 (m, 7H), 3.47 (t, *J* = 5.5 Hz,3H), 3.28 - 3.19 (m, 2H), 3.19 - 3.08 (m, 2H), 3.02 (t, *J*= 5.5 Hz,2H), 2.08 - 1.89 (m, 2H), 1.89 - 1.64 (m, 3H), 1.64 - 1.45 (m, 1H). MS 1137.2 (M+H)⁺.

### EXAMPLE 7

### 2,2-dimethyl-N1,N3-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)malonamide

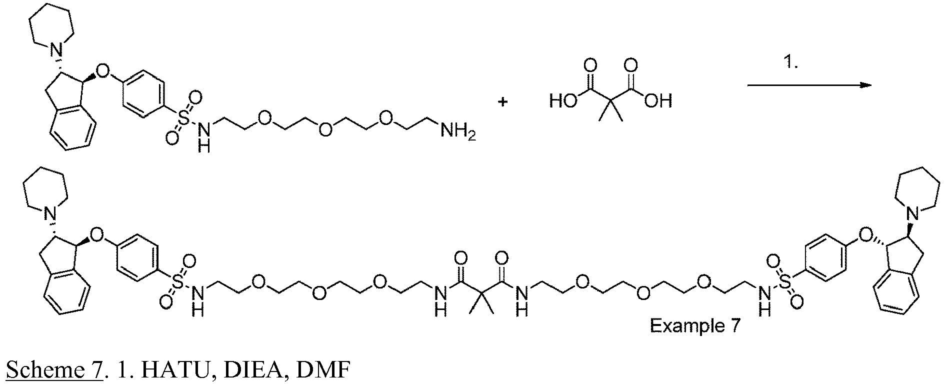

Example **7**: 2,2-dimethyl-N1,N3-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)malonamide: HATU (35.1 mg, 0.092 mmol) was added to a solution of N-(2-(2-(2-(2-aminoethoxy)ethoxy)ethoxy)ethyl)-4-((1S,2S)-2-(piperidin-1-yl)-2,3-dihydro-1H-inden-1-yloxy)benzenesulfonamide (46 mg, 0.84 mmol), 2,2-dimethylmalonic acid (5.5 mg, 0.042 mmol), and DIEA (11.9 mg, 0.092 mmol) in DMF (0.2 mL). After 105 minutes, the reaction mixture was concentrated under vacuum and purified by reverse phase HPLC (ACN/water/0.1% TFA) to give a TFA salt of the title compound (19.5 mg). ¹H-NMR (400 MHz, CD₃OD) δ 7.92 - 7.84 (m, 2H), 7.33 (d, *J* = 9.1 Hz, 4H), 7.25 - 7.15 (m, 2H), 6.35 - 6.29 (m, 1H), 3.59 (s, 7H), 3.55 - 3.41 (m, 8H), 3.34 (s, H), 3.11 (s, 1H), 3.08 - 3.03 (m, H), 1.92 - 1.74 (m, 1H), 1 .37 (s, 3H). MS 1191.2 (M+H)⁺.

### EXAMPLE 8

### N1,N4-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)succinamide

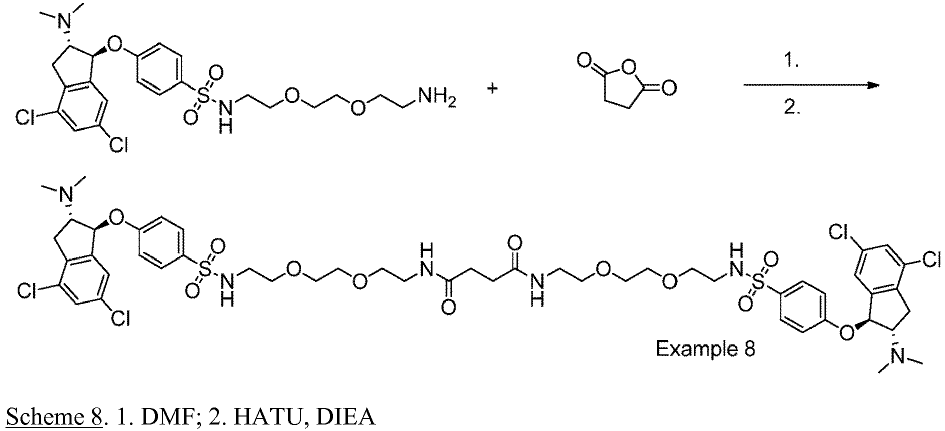

Example **8**: N1,N4-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)succinamide: Succinic anhydride (3.6 mg, 0.0.037 mmol) was added to a solution of N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide (39 mg, 0.73 mmol) in DMF (0.4 mL). After 30 minutes, DIEA (9.4 mg, 0.073 mmol) and HATU (14 mg, 0.037 mmol) were added. After an additional 15 minutes, the reaction mixture was concentrated under vacuum and purified by reverse phase FIPLC (ACN/water/0.1% TFA) to give a TFA salt of the title compound (18.3 mg). ¹H-NMR (400 MHz, CD₃OD) δ 7.91 (d, *J* = 8.9 Hz, 2H), 7.52 (d, *J* = 1.6 Hz, 1H), 7.35 (d, *J* = 8.9 Hz, 2H), 7.11 (s, 1H), 6.41 (s, 1H), 4.41 (dd. *J =* 15.1, 8.3 Hz, 2H), 3.65 (dd, *J=* 16.6, 8.7 Hz, 1H), 3.57 - 3.46 (m, 9H). 3.34 - 3.31 (m, 3H), 3.20 (dd, *J =* 16.6, 9.4 Hz, 2H), 3.07 (t, *J* = 5.5 Hz, 2H), 3.02 (s, 6H), 2.46 (s, 2H). MS 1145.1 (M+H)⁺ .

### EXAMPLE 9

### 2,2'-oxybis(N-(2-(2-(2-(4-((1S,2S)-4,6-dichloro-2-(dimethylamino)-2,3-dihydro-1H-inden-1-yloxy)phenylsulfonamido)ethoxy)ethoxy)ethyl)acetamide)

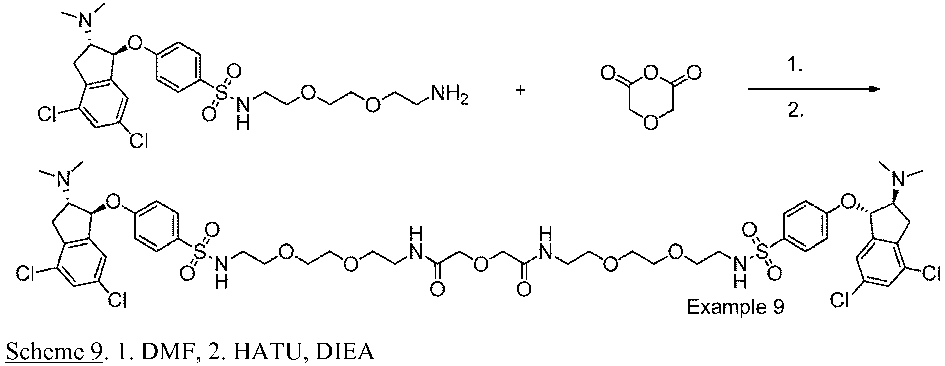

Example **9**: 2,2'-oxybis(N-(2-(2-(2-(4-((1S,2S)-4,6-dichloro-2-(dimethylamino)-2,3-dihydro-1H-inden-1-yloxy)phenylsulfonamido)ethoxy)ethoxy)ethyl)acetamide): Diglycolic anhydride (4.3 mg, 0.037 mmol) was added to a solution of N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide (39 mg, 0.73 mmol) in DMF (0.4 mL). After 30 minutes, DIEA (9.4 mg, 0.073 mmol) and HATU (14 mg, 0.037 mmol) were added. After an additional 45 minutes, the reaction mixture was concentrated under vacuum and purified by reverse phase FIPLC (ACN/water/0.1% TFA) to give a TFA salt of the title compound (11.3 mg). ¹H NMR (400 MHz, CD₃OD) δ 7.90 (d, *J*= 8.9 Hz, 2H), 7.51 (d, *J* = 1.6 Hz, 1H), 7.34 (d, *J* = 9.0 Hz, 2H), 7.11 (d, *J* = 0.7 Hz, 1H), 6.38 (d, *J* = 7.4 Hz, 1H), 4.42 - 4.34 (m, 1H), 4.03 (s, 2H), 3.63 (dd, *J* = 15/7, 8.2 Hz, 1H), 3.59 - 3.55 (m, 4H), 3.55 - 3.50 (m, 3H), 3.50 - 3.45 (m, 3H), 3.45 - 3.39 (m, 2H), 3.19 (dd, *J=* 16.3, 8.8 Hz, 1H), 3.06 (t, *J* = 5.6 Hz, 2H), 2,99 (s, 6H), 2.01 (s, 1H). MS 1161.4 (M+H)⁺.

### EXAMPLE 10

### 4-amino-4-(13-oxo-1-(4-((1S,2S)-2-(piperidin-1-yl)-2,3-dihydro-1H-inden-1-yloxy)phenylsulfonamido)-3,6,9-trioxa-12-azapentadecan-15-yl)-N1,N7-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)heptanediamide

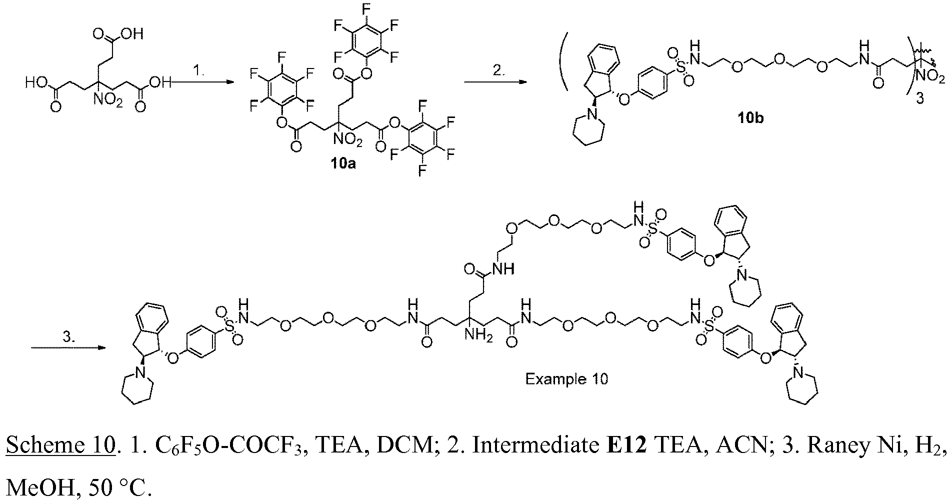

Intermediate **10a:** bis(perfluorophenyl)4-nitro-4-(3-oxo-3-(perfluorophenoxy)propyl)heptanedioate: A solution of 4-(2-carboxyethyl)-4-nitroheptanedioic acid (3.00 g, 10.8 mmol) in DCM (54 mL) was charged in an additional funnel and added dropwise to a solution of perfluorophenyl 2,2,2-trifluoroacetate (6.15 mL, 35.7 mmol) and TEA (9.0 mL, 65 mmol) in DCM (54 mL). Upon completion of addition, the solution was stirred an additional 20 min at room temperature, during which time a white precipitate formed. The precipitate was filtered and washed with 3 : 7 DCM : hexanes and then washed with hexanes to give the title compound (6.87 g, 82%) as a white solid.

Intermediate **10b:** 4-nitro-4-(3-oxo-6-(2-(2-(2-(4-((1S,2S)-2-(piperidin-1-yl)-2,3-dihydro-1H-inden-1-yloxy)phenylsulfonamido)ethoxy)ethoxy)ethoxy)hexyl)-N1,N7-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)heptanediamide: Bis(perfluorophenyl) 4-nitro-4-(3-oxo-3-(perfluorophenoxy)propyl)heptanedioate (39.2 mg, 0.051 mmol) was added to a solution of N-(2-(2-(2-(2-aminoethoxy)ethoxy)ethoxy)ethyl)-4-(1S,2S)-2-(piperidin-1-yl)-2,3-dihydro-1H-inden-1-yloxy)benzenesulfonamide (84.1 mg, 0.154 mmol) and triethylamine (17.1 mg, 0.169 mmol) in acetonitrile (0.3 mL). After 50 minutes the reaction mixture was diluted with water and purified by reverse phase HPLC to give a TFA salt of the title compound (16.8 mg). MS 1865.5 (M+H)⁺.

Example **10**: 4-amino-4-(13-oxo-1-(4-((1S,2S)-2-(piperidin-1-yl)-2,3-dihydro-1H-inden-1-yloxy)phenylsulfonamido)-3,6,9-trioxa-12-azapentadecan-15-yl)-N1,N7-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)heptanediamide: Raney Nickel (∼30 mg, washed with water (4 mL) and MeOH (2 mL)) was added to a solution of 4-nitro-4-(3-oxo-6-(2-(2-(2-(4-((1S,2S)-2-(piperidin-1-yl)-2,3-dihydro-1H-inden-1-yloxy)phenylsulfonamido)ethoxy)ethoxy)ethoxy)hexyl)-N1,N7-bis(2-(2-(2-(2-(4-((1S,2S)-2-(piperidin-1-yl)-2,3-dihydro-1H-inden-1-yloxy)phenylsulfonamido)ethoxy)ethoxy)ethoxy)ethyl)heptanediamide (16.8 mg) in MeOH (1 mL). The vigorously stirred suspension was placed under an atmosphere of hydrogen and heated to 50 °C. After 5 hours the mixture was purged with N₂, cooled and filtered. The solvent was removed and the residue purified by reverse phase HPLC (ACN/water/0.1 % TFA) to give a TFA salt of the title compound (7.0 mg). ¹H-NMR (400 MHz, DMSO-*d₆*) δ 9.94 (s, 1H), 7.98 (t, *J* = 5.3 Hz, 1H), 7.88 (s, 1H), 7.78 (d, *J* = 8.6 Hz, 2H), 7.61 (t, *J* = 5.7 Hz, 1H), 7.41 - 7.29 (m, 4H), 7.25 - 7.17 (m, 1H), 7.13 (d, *J=* 7.8 Hz, 1H), 6.45 (s, 1H), 4.32 (s, 1H), 3.56 - 3.20 (m, 40H), 3.16 (dd, *J=* 11.5, 5.8 Hz, 3H), 3.04 (s, 3H), 2.88 (q, *J=* 5.8 Hz, 2H), 2.22 - 2.07 (m, 2H), 1.84 (s, 2H), 1.76 - 1.50 (m, 5H), 1.50 - 1.31 (m, 1H). MS 1835.7 (M+H)⁺.

### EXAMPLE 11

### 4-amino-N1,N7-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-4-methylheptanediamide

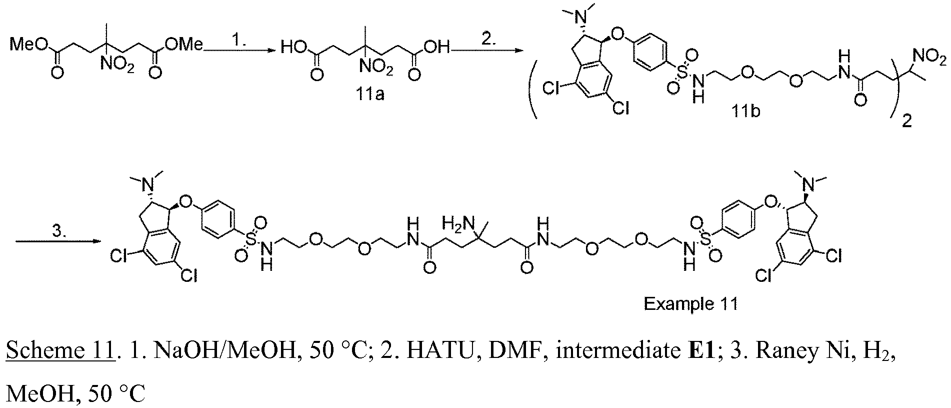

Intermediate **11a**: 4-methyl-4-nitroheptanedioic acid: A solution of dimethyl 4-methyl-4-nitroheptanedioate (51.7 mg, 0.207 mmol) in MeOH (1 mL) with NaOH (0.345mL, 3M) was stirred at 50°C for 3 hours. The reaction mixture was neutralized with 2M HCl and the solvent removed. The crude material was then suspended in DMF and used directly without further purification.
Intermediate **11a:** N1,N7-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-4-methyl-4-nitroheptanediamide: To a suspension of 4-methyl-4-nitroheptanedioic acid (0.207 mmol) in DMF (2 mL) was added N-(2-(2-(2-aminoethoxy)ethoxy)ethyl)-4-((1S,2S)-4,6-dichloro-2-(dimethylamino)-2,3-dihydro-1H-inden-1-yloxy)benzenesulfonamide (220 mg, 0.414 mmol), DIEA (78.0 µL, 0.45 mmol) and HATU (171 mg, 0.45 mmol). After 16 hours the solvent was removed and crude residue was diluted with EtOAc (75 mL), washed with saturated NaHCO₃ (10 mL), water (2x10 mL) and dried over Na₂SO₄. The crude material was purified by flash chromatography (12g SiO₂, 0-5% MeOH in DCM with 0.5% TEA over 15 minutes). The excess TEA was removed by diluting the resulting material in DCM (40 mL) and washing with IN HCl (10 mL) and saturated aqueous NaHCO₃ (10 mL) to give the free base of the title compound (153 mg). MS 1246.2 (M+H) ⁺.

Example **11** : 4-amino-N1,N7-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-4-methylheptanediamide: Raney Nickel (∼400 mg, washed with water (2x4 mL) and MeOH (4 mL)) was added to a solution of N1,N7-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-4-methyl-4-nitroheptanediamide (153 mg) in MeOH (2 mL). The resulting suspension was stirred vigorously under an atmosphere of H₂ and heated to 50 °C. After 7 hours the reaction was filtered and the filter washed with MeOH (∼10 mL) and DMF (∼2 mL). The mixture was concentrated under vacuum and purified by reverse phase HPLC (ACN/water/0.1% TFA) to give a TFA salt of the title compound (106.8 mg). ¹H-NMR (400 MHz, CDCl₃) δ 8.17 (s, 1H), 7.91 (d, *J=* 8.7 Hz, 2H), 7.56 (s. 1H), 7.36 (s, 1H), 7.22 - 7.18 (m, 1H), 7.03 (s, 1H), 6.72 (d, *J* = 7.4 Hz, 1H), 4.00 (q, *J* = 8.6 Hz, 1H), 3.67 - 3.45 (m, 11H), 3.45 - 3.34 (m, 4H), 3.19 - 3.09 (m, 2H), 2.94 (s, 7H), 2.55 - 2.38 (m, 3H), 2.28 (s, 21H), 2.08 - 1.81 (m, 4H), 1.30 (s, 2H). MS 1216.2 (M+H)⁺.

### EXAMPLE 12

### 1-(4-((1S,2S)-4,6-dichloro-2-(dimethylamino)-2,3-dihydro-1H-inden-1-yloxy)phenylsulfonamido)-13-(3-(2-(2-(2-(4-((1S,2S)-4,6-dichloro-2-(dimethylamino)-2,3-dihydro-1H-inden-1-yloxy)phenylsulfonamido)ethoxy)ethoxy)ethylamino)-3-oxopropyl)-13-methyl-10,15-dioxo-3,6-dioxa-9,14-diazaoctadecan-18-oic acid

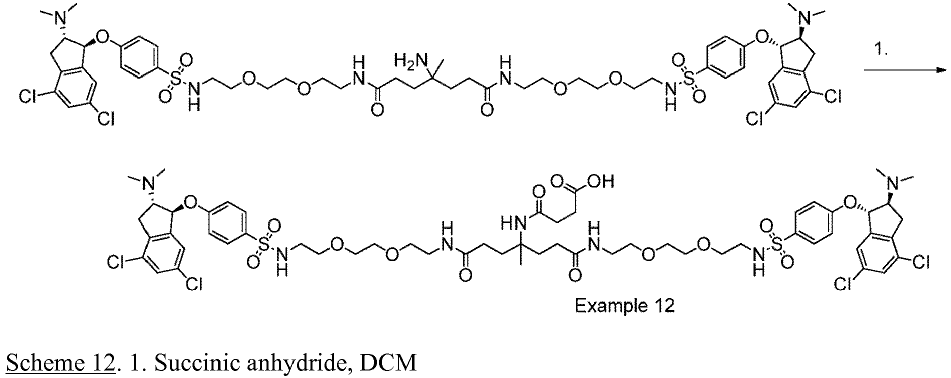

Example **12:** 1-(4-((1S,2S)-4,6-dichloro-2-(dimethylamino)-2,3-dihydro-1H-inden-1-yloxy)phenylsulfonamido)-13-(3-(2-(2-(2-(4-((1S,2S)-4,6-dichloro-2-(dimethylamino)-2,3-dihydro-1H-inden-1-yloxy)phenylsulfonamido)ethoxy)ethoxy)ethylamino)-3-oxopropyl)-13-methyl-10,15-dioxo-3,6-dioxa-9,14-diazaoctadecan-18-oic acid: Succinic anhydride (3.3 mg, 0.033 mmol) was added to a solution of 4-amino-N1,N7-bis(2-(2-(2-(4-((1S,2S)-4,6-dichloro-2-(dimethylamino)-2,3-dihydro-1H-inden-1-yloxy)phenylsulfonamido)ethoxy)ethoxy)ethyl)-4-methylheptanediamide (27 mg, 0.022 mmol) in DCM (0.1 mL). After 4 hours the reaction mixture was concentrated under vacuum and purified by reverse phase HPLC (ACN/water/0.1 % TFA) to give a TFA salt of the title compound (8.5 mg). ¹H-NMR (400 MHz, DMSO-*d₆*) δ 10.50 - 10.23 (m, 1H), 7.80 (d, *J =* 8.9 Hz, 2H), 7.77 - 7.72 (m, 1H), 7.68 (d, *J =* 1.5 Hz, 1H), 7.63 (t, *J =* 6.0 Hz, 1H), 7.35 (d, *J =* 8.9 Hz, 2H), 7.25 (s, 1H), 7.12 (d, *J* = 1.5 Hz, 1H), 6.40 (s, 1H), 4.43 - 4.27 (m, 1H), 3.61 - 3.29 (m, 22H), 3.23 - 3.16 (m, 1H), 3.12 (dd, *J* = 11.5, 5.6 Hz, 3H), 2.92 - 2.75 (m, 9H), 2.35 (t, *J* = 7.1 Hz, 1H), 2.25 (t*, J* = 6.7 Hz, 1H), 2.01 - 1.94 (m, 2H), 1.94 - 1.82 (m, 1H), 1.68 - 1.58 (m, 1H), 1.05 (s, 2H). MS 1316.3 (M+H)⁺.

### EXAMPLE 13

### 1-{2-[2-(2-{[(3-{[(1S,2S)-2-(piperidin-1-yl)-2,3-dihydro-1H-inden-1-yl]oxy}phenyl)carbamoyl]amino}ethoxy)ethoxy]ethyl}-3-{4-[({2-[2-(2-{[(3-{[(1S,2S)-2-(piperidin-1-yl)-2,3-dihydro-1H-inden-1-yl]oxy}phenyl)carbamoyl]amino}ethoxy)ethoxy]ethyl}carbamoyl)amino]butyl}urea

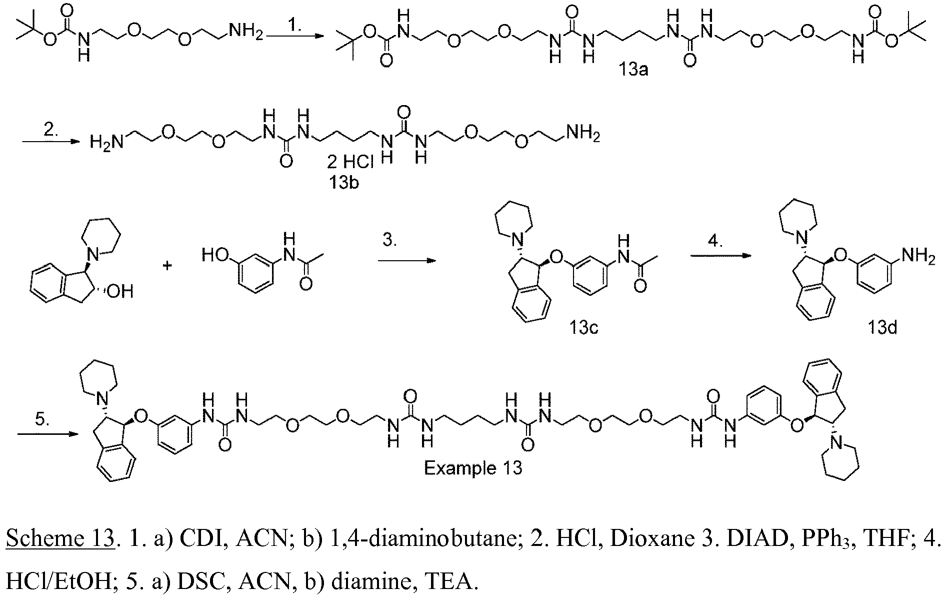

Intermediate **13a:** *tert*-butyl 10,17-dioxo-3,6,21,24-tetraoxa-9,11,16,18-tetraazahexacosane-1,26-diyldicarbamate. *tert*-Butyl (2-(2-(2-aminoethoxy)ethoxy)ethyl)carbamate: (2.5 g, 10.1 mmol) was dissolved in ACN (8 mL). The solution was added to a mixture of carbonyldiimidazole (CDI) (1.65 g, 10.2 mmol), and ACN (15 mL). The resulting mixture was stirred for 1h and analyzed by HPLC for conversion to the intermediate *tert*-butyl (2-(2-(2-(1H-imidazole-1-carboxamido)ethoxy)ethoxy)ethyl)carbamate. Upon completion, 1,4-diaminobutane (0.44 g, 5.0 mmol) was added as a solution in ACN (5.5 mL). The mixture was warmed to 40 °C, and stirred for 2 h. The solvent was removed under reduced pressure. The resulting residue was dissolved in EtOAc (20 mL), and washed twice with 10% NaCl in water (10 mL) followed by 20% aqueous citric acid (10 mL), then finally washed with 25% aqueous NaCl. The organic solution was dried (Na₂SO₄) and the solvent evaporated under reduced pressure to give the title compound (1.17g) as an oil. ¹H NMR (400 MHz, DMSO-*d₆*) 6.78 (t, *J=* 5.5 Hz, 2H), 5.93 (t, *J =* 5.8 Hz, 2H), 5.81 (t, *J =* 5.3 Hz, 2H), 3.49 (s, 16H), 3.39- 3.35 (m, 4H), 3.15-3.11 (m, 4H), 3.08-3.04 (m, 4H), 2.96-2.94 (m, 4H), 2.74 (d, *J* = 15.3 Hz, 2H), 2.63 (d, *J* = 15.3 Hz, 2H), 1.37 (s, 18 H).

Intermediate **13b**: 1,1'-(butane-1,4-diyl)bis(3-(2-(2-(2-aminoethoxy)ethoxy)ethyl)urea) dihydrochloride: To a solution of *tert*-butyl 10,17-dioxo-3,6,21,24-tetraoxa-9,11,16,18-tetraazahexacosane-1,26-diyldicarbamate (1.17 g) in IPA (12 mL) was added 4M HCl in dioxane (10 mL) keeping the temperature under 30 °C. The mixture was stirred overnight during which time the product precipitated. The product was then filtered, washed with IPA, and dried in a vacuum oven 40 °C to give the title compound as a white solid (930 mg, mp 130 °C) that can be recrystallized from 95% isopropanol, 5% water to give the title compound as fine needles (mp 165-168 °C). ¹H NMR (400 MHz, DMSO-*d₆*) 7.16 (d, *J =* 11.3 Hz, 4H), 6.86 (d, *J* = 8.6 Hz, 4H), 6.29 (t, *J =* 5.9 Hz, 2H), 4.15 (d, *J =* 6.0 Hz, 4H), 3.72 (s, 12H), 3.35 (s, 16H).

Intermediate **13c**: N-(3-((1S,2S)-2-(piperidin-1-yl)-2,3-dihydro-1H-inden-1-yloxy)phenyl)acetamide: A solution of DIAD (318 mg, 1.58 mmol) in THF (0.5 mL) was added over 30 minutes to a solution of (1R,2R)-1-(piperidin-1-yl)-2,3-dihydro-1H-inden-2-ol (286 mg, 1.32 mmol), N-(3-hydroxyphenyl)acetamide (199 mg, 1.32 mmol), and PPh₃ (449 mg, 1.72 mmol) in THF (2.6 mL). After 1 hour additional N-(3-hydroxyphenyl)acetamide (79 mg), PPh₃ (138 mg), and DIAD (105 mg) was added. After a further 2 hours the reaction mixture was concentrated under vacuum and purified by flash chromatography (12g SiO₂, 0-100% EtOAc in DCM over 20 minutes) to give the title compound (450 mg).

Intermediate **13d:** 3-((1S,2S)-2-(piperidin-1-yl)-2,3-dihydro-1H-inden-1-yloxy)aniline: Aqueous HCl (3.8mL, 2N) was added to a solution of N-(3-((1S,2S)-2-(piperidin-1-yl)-2,3-dihydro-1H-inden-1-yloxy)phenyl)acetamide (450 mg, 1.28 mmol) in EtOH (3.8 mL). After 4 hours the reaction mixture was concentrated under vacuum and diluted with DCM (5 mL). The resulting solution was neutralized with NaOH (7.7mL, IN), extracted with DCM (3x5 mL), and dried over Na₂SO₄ to give the title compound (332 mg).

Example **13**: 1-{2-[2-(2-{[(3-{[(1S,2S)-2-(piperidin-1-yl)-2,3-dihydro-1H-inden-1-yl]oxy}phenyl)carbamoyl]amino}ethoxy)ethoxy]ethyl}-3-{4-[({2-[2-(2-{[(3-{[(1S,2S)-2- (piperidin-1-yl)-2,3-dihydro-1H-inden-1-yl]oxy}phenyl)carbamoyl]amino}ethoxy)ethoxy]ethyl}carbamoyl)amino]butyl}urea: 2 3-((1S,2S)-2-(piperidin-1-yl)-2,3-dihydro-1H-inden-1-yloxy)aniline (50 mg, 0.162 mmol) in acetonitrile (0.3 mL) was added to a solution of N,N'-disuccinimidyl carbonate (41.4 mg, 0.162 mmol) in acetonitrile (0.3 mL). After 30 minutes 1,1'-(butane-1,4-diyl)bis(3-(2-(2-(2-aminoethoxy)ethoxy)ethyl)urea) dihydrochloride (41 mg, 0.08 mmol) and TEA (16.3 mg, 0.162 mmol) in water (0.2 mL) were added. After 20 minutes the reaction mixture was concentrated under vacuum and purified by reverse phase HPLC (ACN/water/0.1 % TFA) to give a TFA salt of the title compound (30.7 mg). ¹H NMR (400 MHz, CD₃OD) δ 7.53 (t, *J* = 2.2 Hz, 1H), 7.39 - 7.30 (m, 2H), 7.29 - 7.20 (m, 3H), 6.82 (dd, *J* = 8.3, 1.5 Hz, 1H), 6.75 (dd, *J* = 8.3, 2.4 Hz, 1H), 6.14 (d, *J* = 6.8 Hz, 1H), 4.23 (dd, *J* = 15.6, 9.3 Hz, 1H). 3.71 - 3.63 (m, 1H), 3.63 - 3.58 (m, 5H), 3.55 (t, *J=* 5.4 Hz, 3H), 3.49 (t, *J =* 5.5 Hz, 3H), 3.35 (t, *J =* 5.3 Hz, 3H), 3.25 (t, *J* = 5.4 Hz, 2H), 3.22 - 3.08 (m, 3H), 3.08 - 3.02 (m, 2H), 2.05 - 1.88 (m, 2H), 1.88 - 1.64 (m, 3H), 1.64 - 1.48 (m, 1H), 1.45 - 1.38 (m, 2H). MS 1105.5 (M+H)⁺.

### EXAMPLES 14-38

The following examples were prepared using methods specified in Table 2:

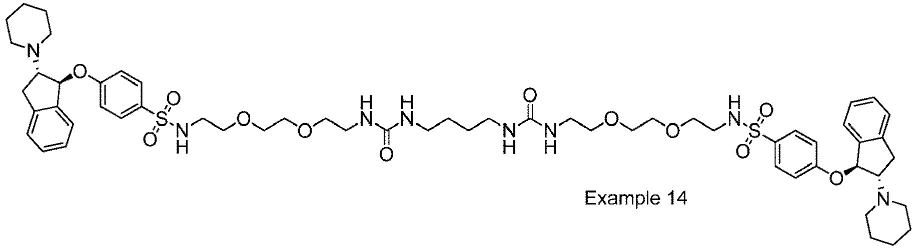

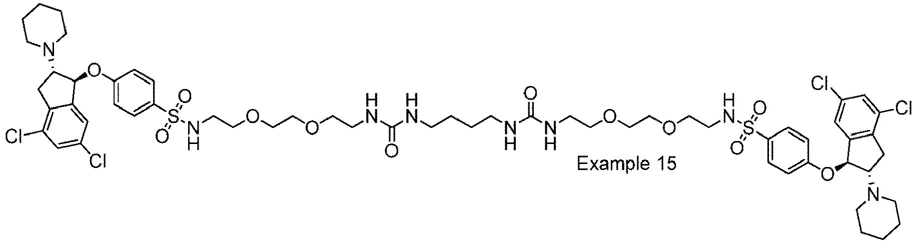

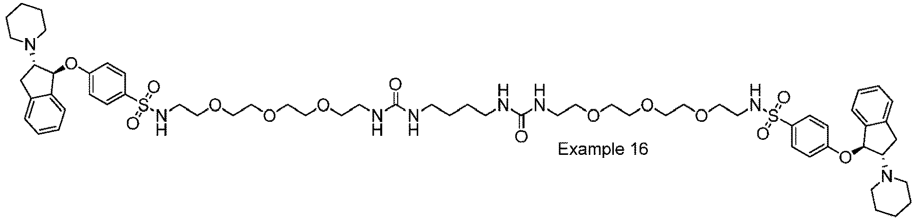

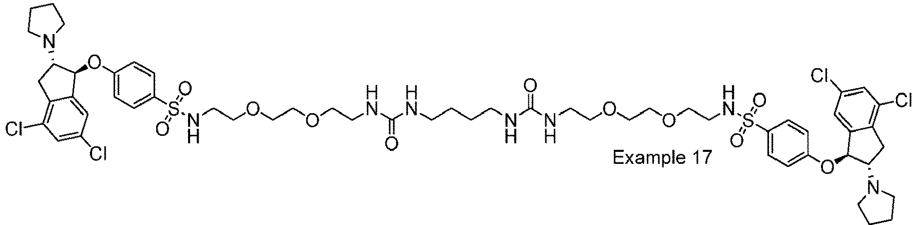

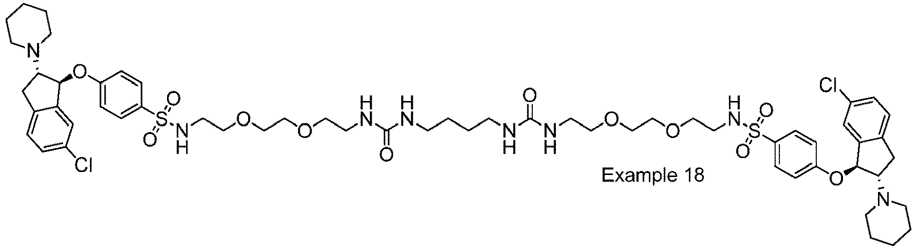

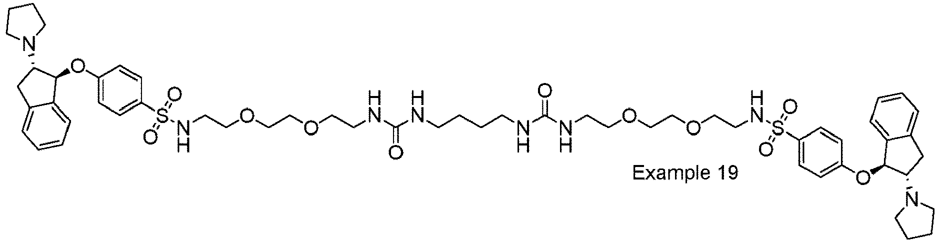

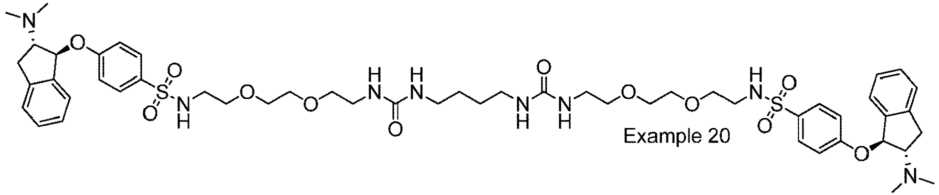

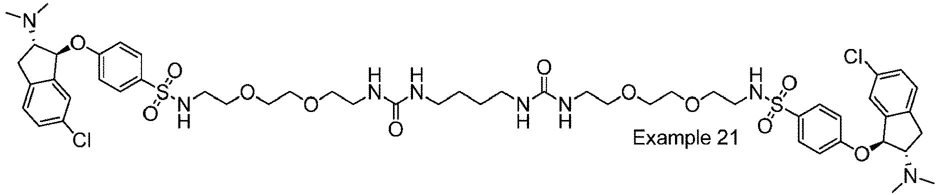

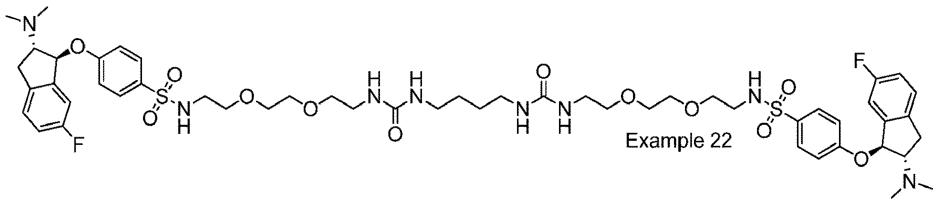

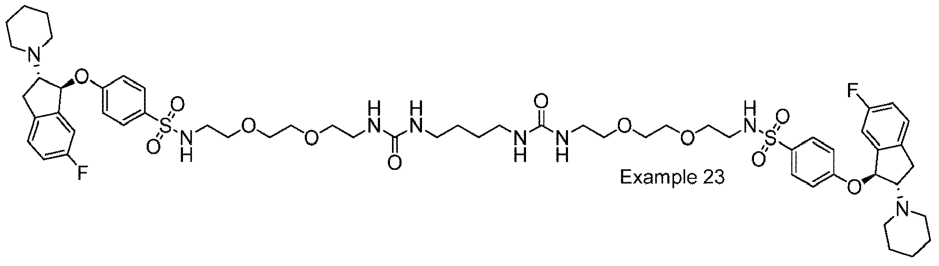

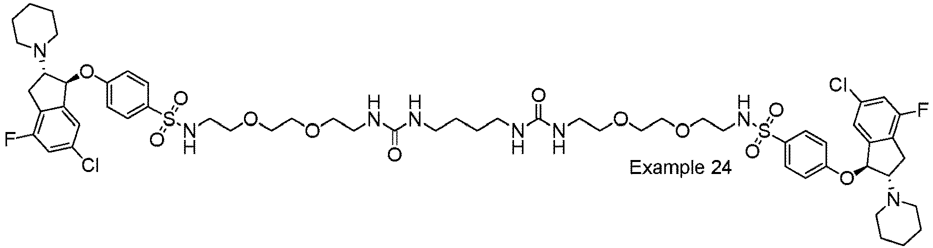

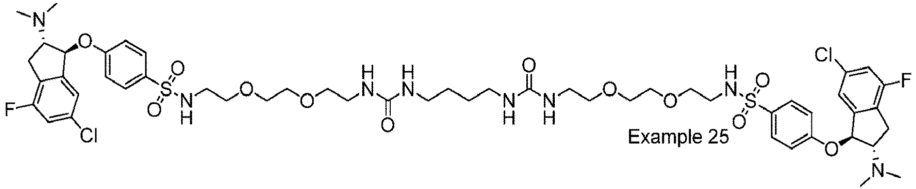

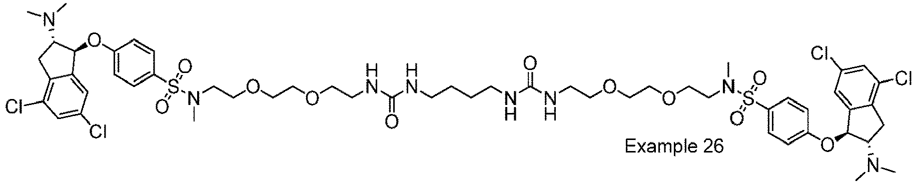

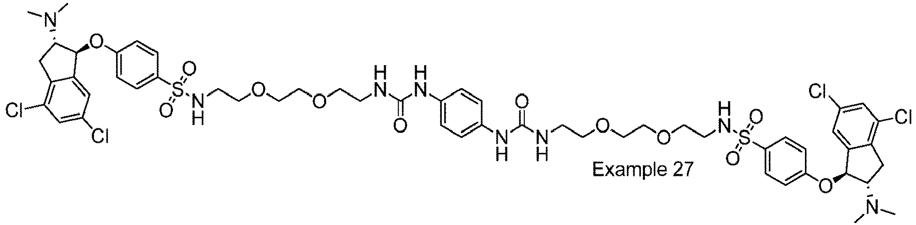

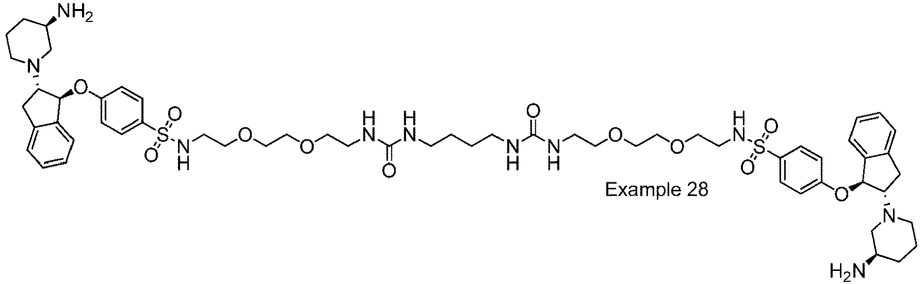

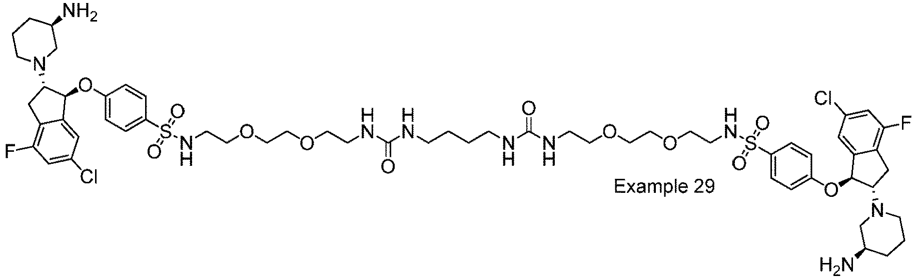

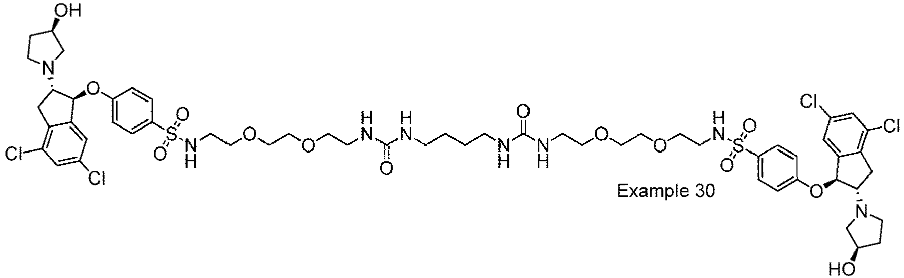

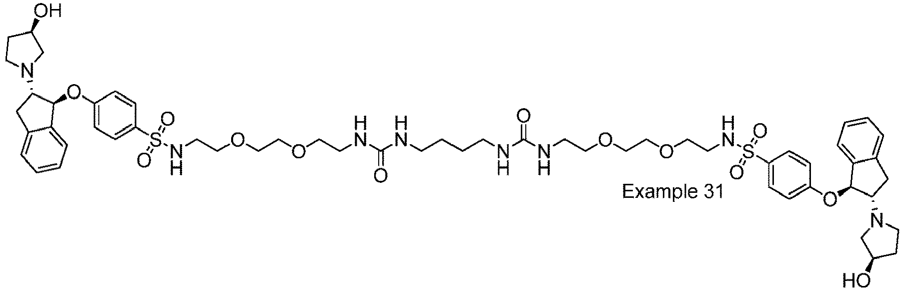

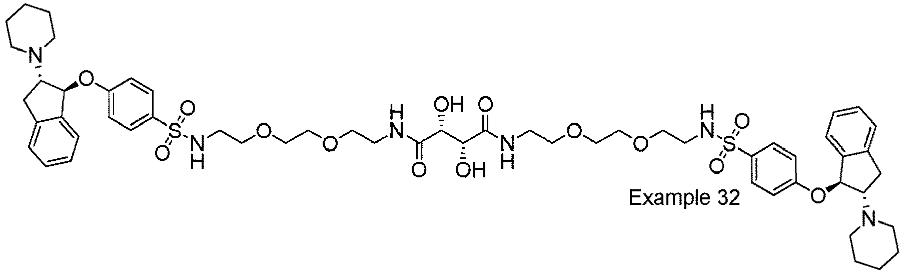

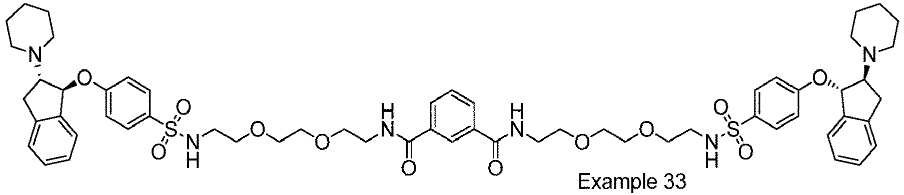

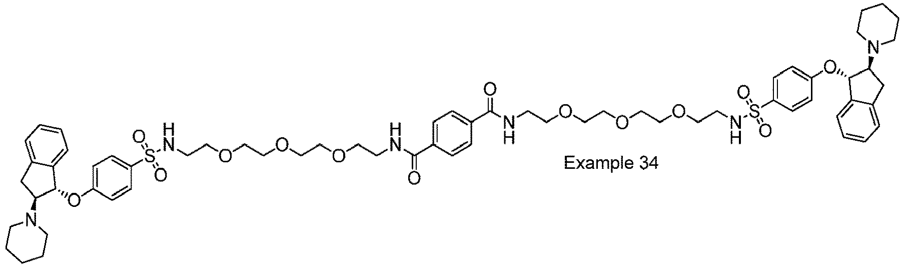

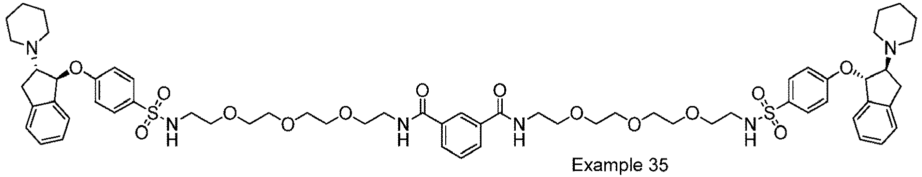

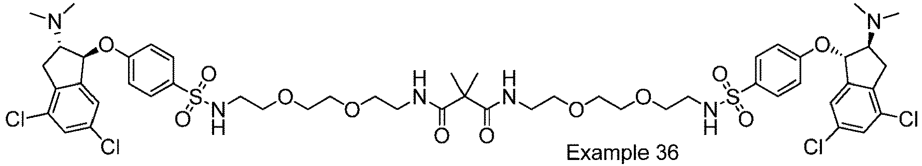

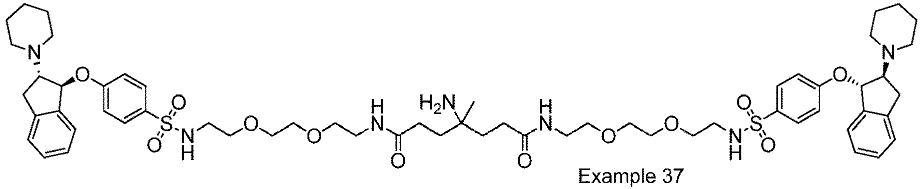

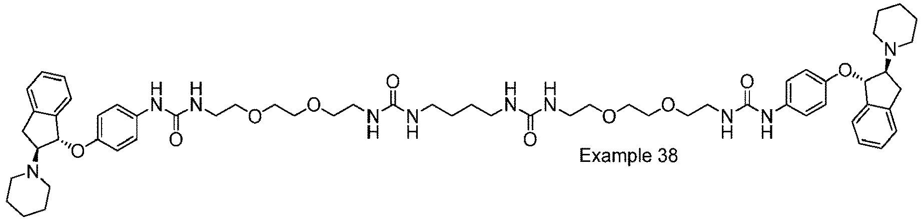

### Chart 4. Structures of Examples 14-38 in Table 2.

**Table 2**

| Example | Synthetic Method | [M] Calc'd | [M+H] Observed |
|---|---|---|---|
| Example 14 | Example 1 | 1146.55 | 1147.3 |
| Example 15 | Example 1 | 1282.39 | 1283.1 |
| Example 16 | Example 1 | 1234.6 | 1235.2 |
| Example 17 | Example 1 | 1254.36 | 1255.1 |
| Example 18 | Example 1 | 1214.47 | 1215.1 |
| Example 18 | Example 1 | 1118.52 | 119.2 |
| Example 20 | Example 1 | 1066.49 | 1067.2 |
| Example 21 | Example 1 | 1134.41 | 1135.1 |
| Example 22 | Example 1 | 1102.47 | 1103.8 |
| Example 23 | Example 1 | 1182.53 | 1183.5 |
| Example 24 | Example 1 | 1250.45 | 1251.4 |
| Example 25 | Example 1 | 1170.39 | 1171.5 |
| Example 26 | Example 1 | 1230.36 | 1231.1 |
| Example 27 | Example 2 | 1222.3 | 1223.1 |
| Example 28 | Example 3 | 1176.57 | 1177.3 |
| Example 29 | Example 3 | 1280.47 | 1281.2 |
| Example 30 | Example 4 | 1286.35 | 1287.1 |
| Example 31 | Example 4 | 1150.51 | 1151.3 |
| Example 32 | Example 5 | 1120.49 | 1121.3 |
| Example 33 | Example 6 | 1136.5 | 1137.3 |
| Example 34 | Example 6 | 1224.55 | 1225.2 |
| Example 35 | Example 6 | 1224.55 | 1225.2 |
| Example 36 | Example 7 | 1158.29 | 1159.3 |
| Example 37 | Example 11 | 1159.57 | 1160.3 |
| Example 38 | Example 13 | 1104.64 | 1105.5 |

### Pharmacological Data

### EXAMPLE 39

### Cell-based assay of NHE-3 activity

Rat or human NHE-3 -mediated Na⁺-dependent H⁺ antiport was measured using a modification of the pH sensitive dye method originally reported by Paradiso (Proc. Natl. Acad. Sci. USA. (1984) 81(23): 7436-7440). Opossum kidney (OK) cells were obtained from the ATCC and propagated per their instructions. The rat NHE-3 gene (GenBank M85300) or the human NHE-3 gene (GenBank NM_004174.1) was introduced into OK cells via electroporation, and cells were seeded into 96 well plates and grown overnight. Medium was aspirated from the wells, cells were washed twice with NaCl-HEPES buffer (100 mM NaCl, 50 mM HEPES, 10 mM glucose, 5mM KCl, 2 mM CaCl₂, 1 mM MgCl₂, pH 7.4), then incubated for 30 min at room temperature with NH₄Cl-HEPES buffer (20 mM NH₄Cl, 80 mM NaCl, 50 mM HEPES, 5 mM KCl, 2mM CaCl₂, 1 mM MgCl₂, pH 7.4) containing 5 µM bis(acetoxymethyl) 3,3'-(3',6'-bis(acetoxymethoxy)-5-((acetoxymethoxy)carbonyl)-3-oxo-3H-spiro[isobenzofuran-1,9'-xanthene]-2',7'-diyl)dipropanoate (BCECF-AM). Cells were washed twice with Ammonium free, Na⁺- free HEPES (100 mM choline, 50 mM HEPES, 10 mM glucose, 5 mM KCl, 2 mM CaCl₂, 1 mM MgCl₂, pH 7.4) and incubated in the same buffer for 10 minutes at room temperature to lower intracellular pH. NHE-3 mediated recovery of neutral intracellular pH was initiated by addition of Na-HEPES buffer containing 0.4 µM ethyl isopropyl amiloride (EIPA, a selective antagonist of NHE-1 activity that does not inhibit NHE-3) and 0-30 µM test compound, and monitoring the pH sensitive changes in BCECF fluorescence (λₑₓ 505nm, λₑₘ 538nm) normalized to the pH insensitive BCECF fluorescence (λₑₓ 439nm, λₑₘ 538nm). Initial rates were plotted as the average 2 or more replicates, and pIC₅₀ values were estimated using GraphPad Prism.

**Table 3**

| Data for examples in human NHE3 inhibition assay | |
|---|---|
| **Result** | **pIC₅₀ Range** |
| **A** | **NHE3 pIC₅₀ < 5** |
| **B** | **NHE3 pIC₅₀ 5-7** |
| **C** | **NHE3 pIC₅₀ > 7** |
| | |

| **Example #** | **Human NHE3 pIC₅₀** |
|---|---|
| 1 | C |
| 2 | C |
| 3 | C |
| 4 | C |
| 5 | C |
| 6 | C |
| 7 | C |
| 8 | C |
| 9 | C |
| 10 | C |
| 11 | C |
| 12 | C |
| 13 | B |
| 14 | C |
| 15 | A |
| 16 | C |
| 17 | C |
| 18 | C |
| 19 | A |
| 20 | C |
| 21 | C |
| 22 | C |
| 23 | C |
| 24 | C |
| 25 | C |
| 26 | C |
| 27 | C |
| 28 | B |
| 29 | C |
| 30 | C |
| 31 | C |
| 32 | C |
| 33 | C |
| 34 | C |
| 35 | C |
| 36 | C |
| 37 | C |
| 38 | C |

### EXAMPLE 40

### Inhibition of intestinal sodium absorption

Urinary sodium concentration and fecal form were measured to assess the ability of selected example compounds to inhibit the absorption of sodium from the intestinal lumen. Eight-week old Sprague-Dawley rats were purchased from Charles River Laboratories (Hollister, CA), were housed 2 per cage, and acclimated for at least 3 days before study initiation. Animals were fed Harlan Teklad Global 2018 rodent chow (Indianapolis, IN) and water *ad libitum* throughout the study and maintained in a standard light/dark cycle of 6AM to 6PM. On the day of the study, between 4PM and 5PM, a group of rats (n=6) were dosed via oral gavage with test compound or vehicle (water) at a volume of 10 mL/kg. After dose administration animals were placed in individual metabolic cages where they were also fed the same chow in meal form and watered *ad libitum.* At 16 h post-dose, the urine samples were collected and fecal form was assessed by two independent observations. Fecal forms were scored according to a common scale associated with increasing fecal water to the wettest observation in the cage's collection funnel (1, normal pellet; 2, pellet adhering to sides of collection funnel due to moisture; 3, loss of normal pellet shape; 4, complete loss of shape with a blotting pattern; 5, liquid fecal streams evident). A rat's fecal form score (FFS) was determined by averaging both observational scores for all rats within a group (n=6). The vehicle group average was 1. These averages are reported in Table 4. For urine samples, the volumes were determined gravimetrically and centrifuged at 3,600 × g. The supernatants were diluted 100-fold in deionized Milli-Q water then filtered through a 0.2 µm GHP Pall AcroPrep filter plate (Pall Life Sciences, Ann Arbor, MI) prior to analysis by ion chromatography. Ten microliters of each filtered extract was injected onto a Dionex ICS-3000 ion chromatograph system (Dionex, Sunnyvale, CA). Cations were separated by an isocratic method using 25 mM methanesulfonic acid as the eluent on an IonPac CS12A 2 mm i.d. × 250 mm, 8 µm particle size cation exchange column (Dionex). Sodium was quantified using standards prepared from a cation standard mix containing Li⁺, Na⁺, NH₄⁺, K⁺, Mg²⁺, and Ca²⁺ (Dionex). The mean mass of sodium urinated for every group in the 16 h period was determined with the vehicle group usually urinating approximately 21 mg sodium. The urine Na (uNa) for rats in the test groups were expressed as a percentage of the vehicle mean and the means were compared to that of the vehicle group by utilizing a one-way analysis of variance coupled with a Dunnett's *post hoc* test.

**Table 4**

| Rat urinary sodium and fecal form 16 h post-dose of test compound at 10 mg/kg | | | |
|---|---|---|---|
| | **uNa Result** | **uNa (% of vehicle)** | |
| | **A** | **>75%** | |
| | **B** | **35-75%** | |
| | **C** | **<35%** | |
| | | | |

| **Example #** | **Dose** | **uNa Result** | **FFS** |
|---|---|---|---|
| 1 | 10 mg/kg | C | 1 |
| 3 | 10 mg/kg | B | 2 |
| 4 | 10 mg/kg | A | 1 |
| 5 | 10 mg/kg | C | 1 |
| 6 | 10 mg/kg | B | 1 |
| 7 | 10 mg/kg | B | 1 |
| 8 | 3 mg/kg | B | 1 |
| 9 | 3 mg/kg | B | 2 |
| 11 | 10 mg/kg | B | 1 |
| 12 | 3 mg/kg | B | 1 |
| 13 | 10 mg/kg | A | 1 |
| 14 | 10 mg/kg | B | 1 |
| 15 | 10 mg/kg | B | 1 |
| 16 | 10 mg/kg | C | 1 |
| 19 | 10 mg/kg | B | 1 |
| 20 | 10 mg/kg | B | 1 |
| 21 | 10 mg/kg | A | 1 |
| 22 | 10 mg/kg | A | 1 |
| 23 | 10 mg/kg | A | 1 |
| 24 | 10 mg/kg | A | 1 |
| 25 | 10 mg/kg | A | 1 |
| 26 | 10 mg/kg | B | 1 |
| 27 | 10 mg/kg | C | 1 |
| 30 | 3 mg/kg | B | 1 |
| 32 | 10 mg/kg | A | 1 |
| 33 | 10 mg/kg | B | 1 |
| 34 | 10 mg/kg | A | 1 |
| 35 | 10 mg/kg | A | 1 |
| 36 | 10 mg/kg | B | 2 |
| 37 | 10 mg/kg | B | 1 |
| 38 | 3 mg/kg | A | 1 |

### EXAMPLE 41

### Plasma PK

Sprague-Dawley rats (n=3) were dosed with test compound by oral gavage. Blood samples were collected at 0.5, 1, 2 and 4 h by retro-orbital bleeds and processed to plasma using K₂EDTA as an anticoagulant. Plasma samples were treated with acetonitrile containing an internal standard and precipitated proteins were removed by centrifugation. Supernatants were analyzed by LC-MS/MS and compound concentrations were determined by interpolation from a standard calibration curve prepared in plasma. Accurate recovery of quality control samples was confirmed to accept each analytical run. Table 5 illustrates data from the pharmacokinetic profiling of an example compound, for which pharmacokinetic parameters were determined. From studies in which one or more rats had samples with test compound levels below the quantitative limit, Cₘₐₓ and AUC (reported as the mean of n=3) may be reported as "<X" to indicate an upper bound.

**Table 5**

| Plasma pharmacokinetics of example compounds | | | | |
|---|---|---|---|---|
| **Example** | **Nominal Dose (mg/kg)** | **LLOQ (ng/mL)** | **Cmax (ng/mL)** | **AUC (ng x hr/mL)** |
| | | | | |
| 1 | 15 | 0.2 | 1.0 | < 3.0 |

### EXAMPLE 42

### Fecal Recovery

Three male Sprague Dawley rats were administered 1 mg/kg test compound by oral gavage. Feces were collected from study animals from 0-48 or 0-72 hours after dosing, dried by lyophilization, and homogenized. Replicate aliquots of 40-60 mg each were subjected to extraction/protein precipitation with 7:1 acetonitrile:water and centrifuged. Supernatants were diluted 1:10 in 50:50 acetonitrile:water prior to analysis by LC-MS/MS. Compound concentrations, determined by interpolation from a standard calibration curve prepared in blank feces matrix, were converted to the percentage of dosed material recovered by taking into account the total collected fecal matter. The percent recovery for each rat was reported as the mean of the calculations from replicate samples. The overall percent recovery (Fecal Recovery [%]) was reported as the mean percent recovery from three rats. Accurate quality control sample recoveries were confirmed in each run and extraction efficiency was periodically verified. Table 6 illustrates fecal recovery data for selected example compounds.

**Table 6**

| Fecal recovery of example compounds | | | |
|---|---|---|---|
| **Example #** | **Nominal Dose (mg/kg)** | **Collection Time (h)** | **Fecal Recovery (%)** |
| 1 | 1 | 48 | 87.7 |
| 3 | 1 | 48 | 69.2 |
| 9 | 1 | 48 | 65.7 |
| 11 | 1 | 48 | 82.1 |
| 30 | 1 | 48 | 66.8 |

## Claims

1. A compound having the following structure of Formula (I):
Core(̵L-NHE)ₙ (I)
or pharmaceutically acceptable salt thereof,
wherein:
(a) n is 2 or 3;
(b) Core is a Core moiety having two or three sites thereon for attachment to two or three NHE-inhibiting small molecule moieties;
(c) L is a bond or linker connecting the Core moiety to the two or three NHE-inhibitory small molecule moieties; and
(d) NHE is a NHE-inhibiting small molecule moiety having the following structure of Formula (XI): 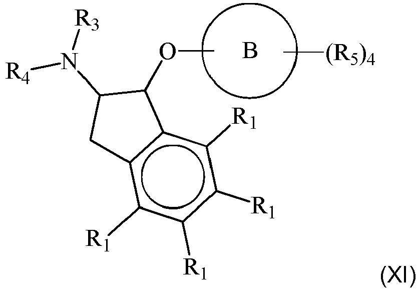 wherein:
B is aryl;
each R₅ is independently selected from the group consisting of hydrogen, halogen, optionally substituted C₁₋₄alkyl, optionally substituted C₁₋₄alkoxy, optionally substituted C₁₋₄thioalkyl, optionally substituted heterocyclyl, optionally substituted heterocyclylalkyl, optionally substituted aryl, optionally substituted heteroaryl, hydroxyl, oxo, cyano, nitro, -NR₇R₈, - NR₇C(=O)R₈, -NR₇C(=O)OR₈, -NR₇C(=O)NR₈R₉, -NR₇SO₂R₈, -NR₇S(O)₂NR₈R₉, -C(=O)OR₇, -C(=O)R₇,
-C(=O)NR₇R₈, -S(O)₁₋₂R₇, and -SO₂NR₇R₈, wherein R₇, R₈, and R₉ are independently selected from the group consisting of hydrogen, C₁₋₄alkyl, or a bond linking the NHE-inhibiting small molecule moiety to L, provided at least one is a bond linking the NHE-inhibiting small molecule moiety to L;
R₃ and R₄ are independently selected from the group consisting of hydrogen, optionally substituted C₁₋₄alkyl, optionally substituted cycloalkyl, optionally substituted cycloalkylalkyl, optionally substituted aryl, optionally substituted aralkyl, optionally substituted heterocyclyl and optionally substituted heteroaryl; or R₃ and R₄ form together with the nitrogen to which they are bonded an optionally substituted 4-8 membered heterocyclyl; and
each R₁ is independently selected from the group consisting of hydrogen, halogen, optionally substituted C₁₋₆alkyl and optionally substituted C₁₋₆alkoxy.

2. The compound, or pharmaceutically acceptable salt thereof, of claim 1 wherein n is 2.

3. The compound, or pharmaceutically acceptable salt thereof, of claim 1 or 2 wherein L is a polyalkylene glycol linker.

4. The compound, or pharmaceutically acceptable salt thereof, of any of claims 1-3 wherein L is a polyethylene glycol linker.

5. The compound, or pharmaceutically acceptable salt thereof, of any of claims 1-4 wherein the Core has the following structure: 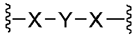 wherein:
X is selected from the group consisting of a bond, -O-, -NH-, -S-, -C₁₋₆alkylene, - NHC(=O)-, -C(=O)NH-, -NHC(=O)NH-, -SO₂NH-, and -NHSO₂-;
Y is selected from the group consisting of a bond, optionally substituted C₁₋₈alkylene, optionally substituted aryl, optionally substituted heteroaryl, a polyethylene glycol linker, -(CH₂)₁₋₆O(CH₂)₁₋₆- and -(CH₂)₁₋₆NY₁(CH₂)₁₋₆-; and
Y₁ is selected from the group consisting of hydrogen, optionally substituted C₁₋₈alkyl, optionally substituted aryl or optionally substituted heteroaryl.

6. The compound, or pharmaceutically acceptable salt thereof, of any of claims 1-5 wherein the Core is selected from the group consisting of:

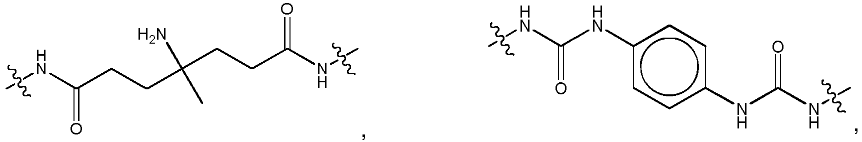

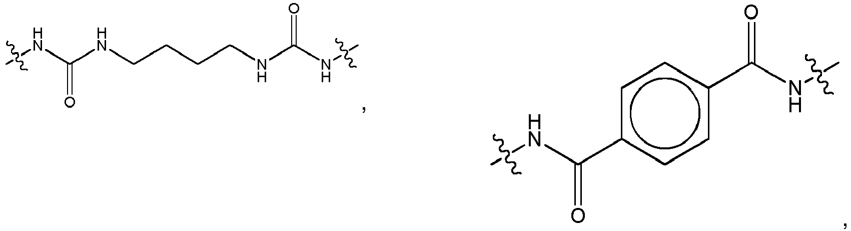

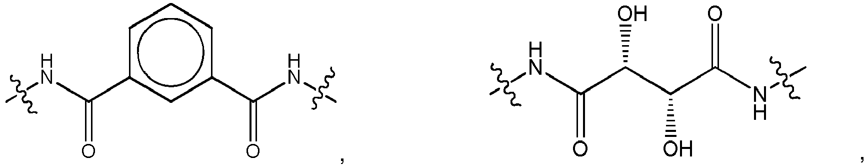

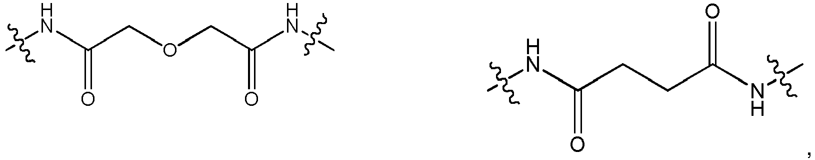

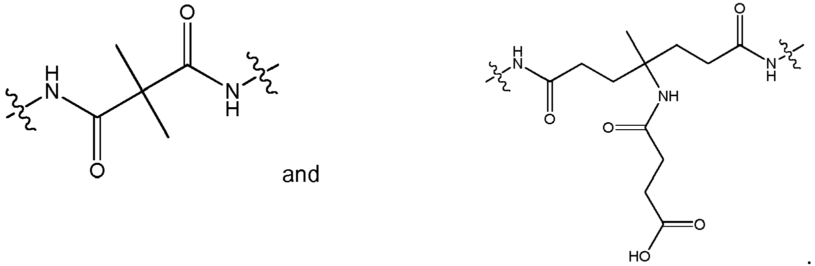

7. The compound, or pharmaceutically acceptable salt thereof, of any of claims 1-6 wherein the NHE-inhibiting small molecule moiety has the following structure of Formula (XII): 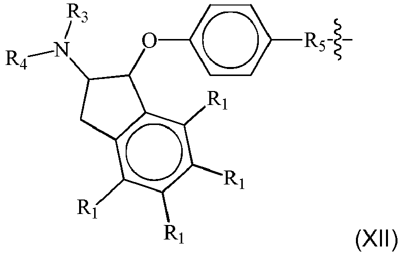 wherein:
each R₃ and R₄ are independently selected from the group consisting of hydrogen and optionally substituted C₁₋₄alkyl, or R₃ and R₄, taken together with the nitrogen to which they are bonded, form an optionally substituted 4-8 membered heterocyclyl;
each R₁ is independently selected from the group consisting of hydrogen, halogen, C₁₋₆alkyl, and C₁₋₆haloalkyl; and
R₅ is selected from the group consisting of -SO₂-NR₇- and -NHC(=O)NH-, wherein R₇ is hydrogen or C₁₋₄alkyl.

8. The compound, or pharmaceutically acceptable salt thereof, of claim 7 wherein R₃ and R₄, taken together with the nitrogen to which they are bonded, form an optionally substituted 5 or 6 membered heterocyclyl.

9. The compound, or pharmaceutically acceptable salt thereof, of claim 8 wherein the optionally substituted 5- or 6- membered heterocyclyl is pyrrolidinyl or piperidinyl.

10. The compound, or pharmaceutically acceptable salt thereof, of claim 7 wherein R₃ and R₄ are independently C₁₋₄alkyl.

11. The compound, or pharmaceutically acceptable salt thereof, of any one of claims 7-10 wherein each R₁ is independently selected from the group consisting of hydrogen or halogen.

12. The compound, or pharmaceutically acceptable salt thereof, of any one of claims 1-11 selected from: 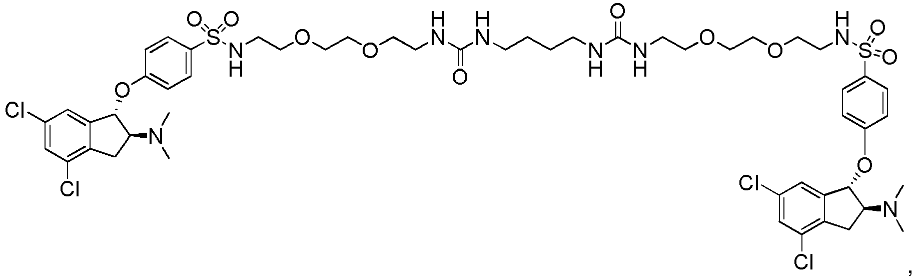 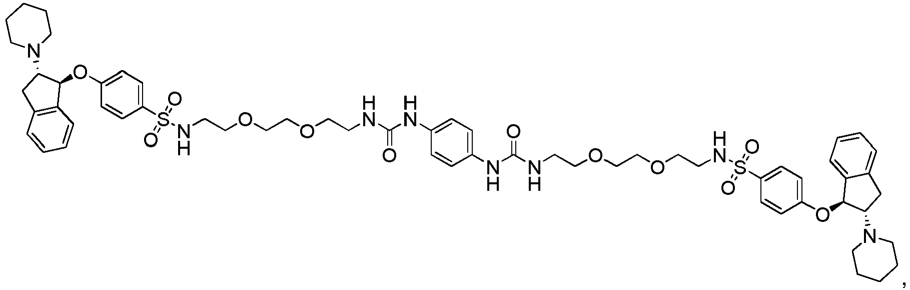 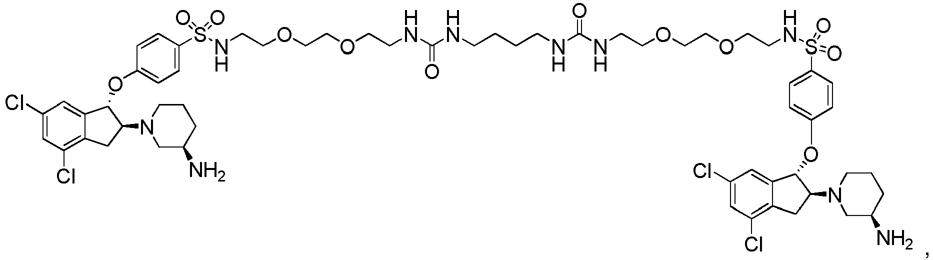 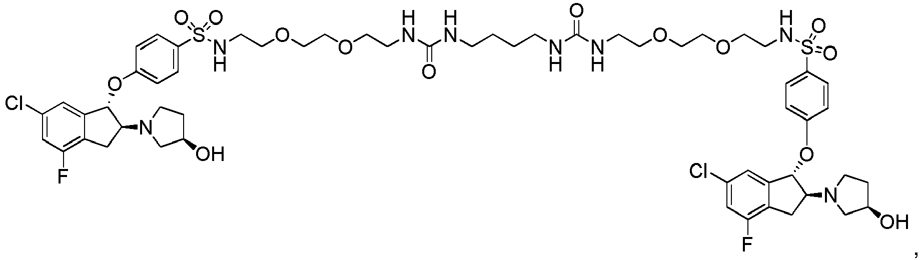 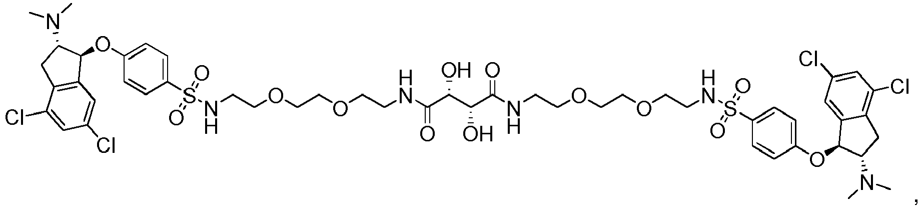 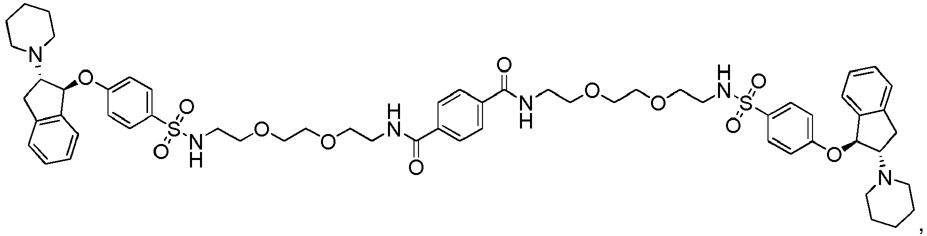 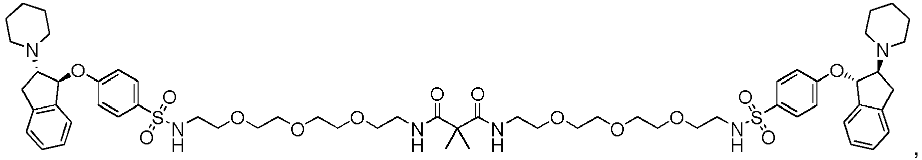 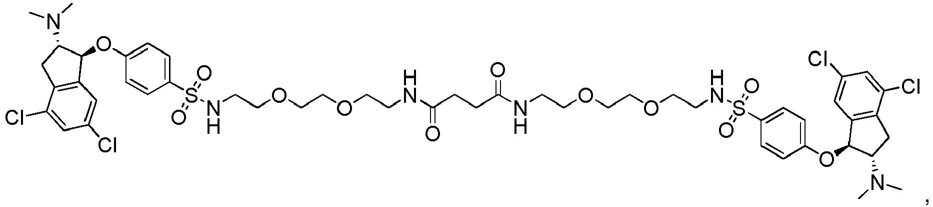 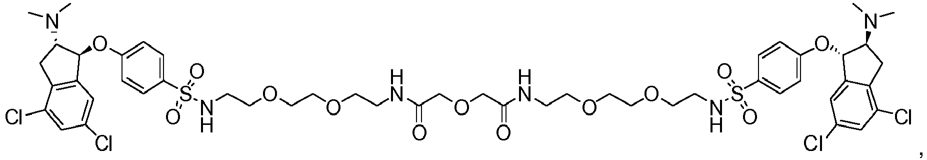 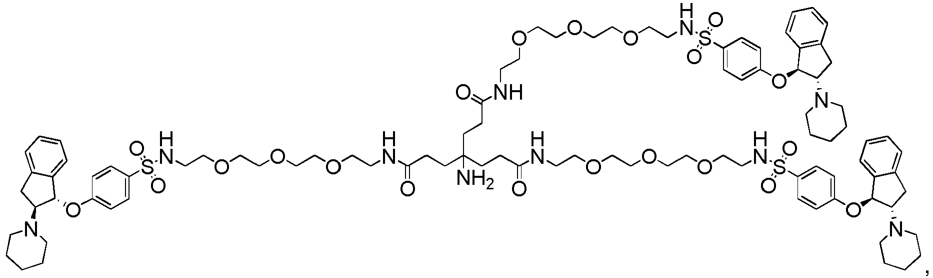 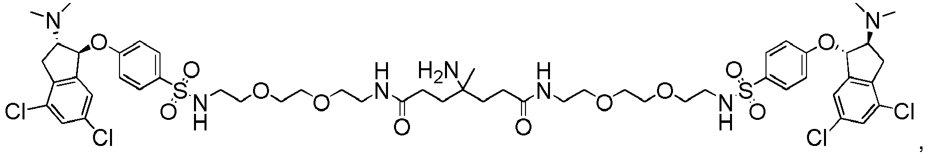 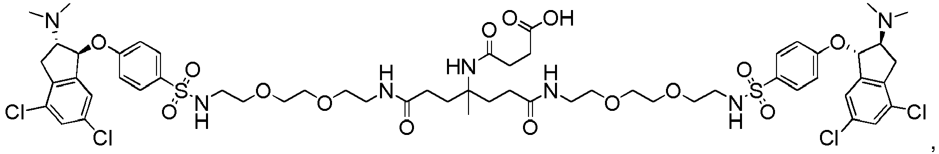 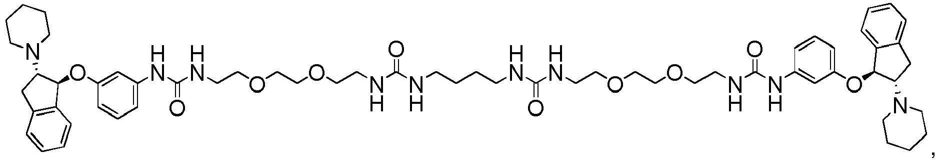 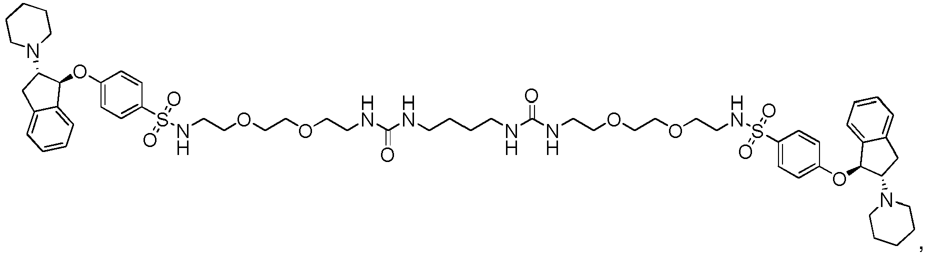 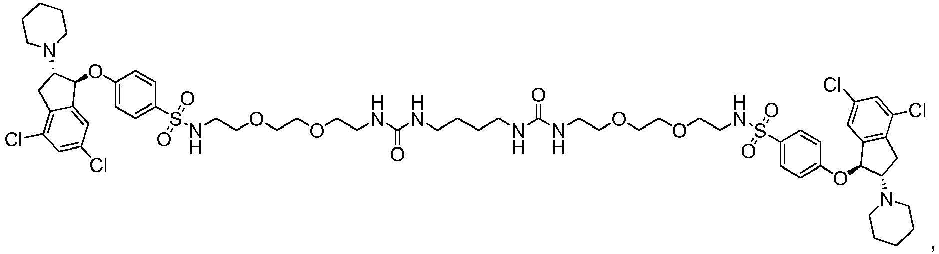 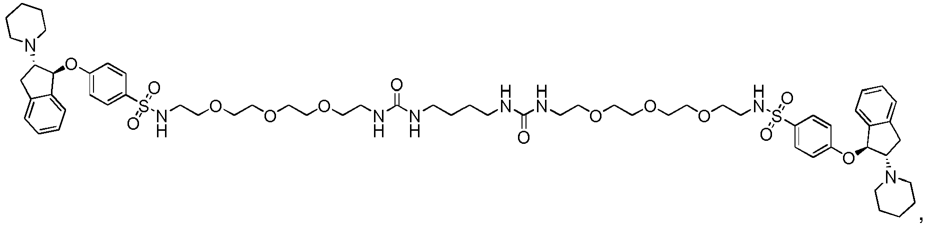 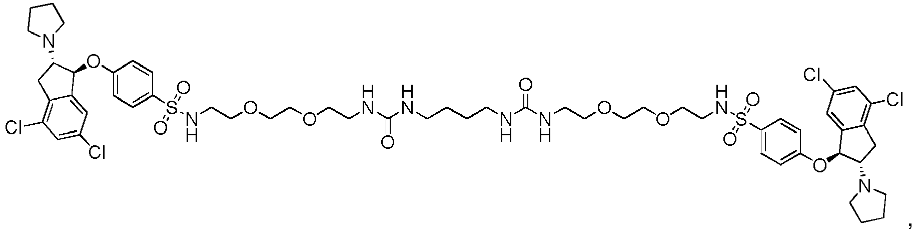 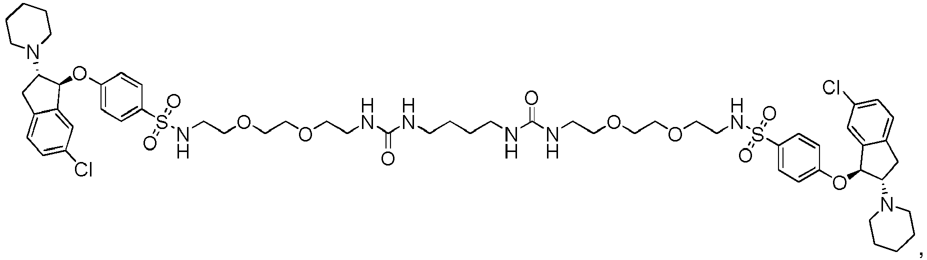 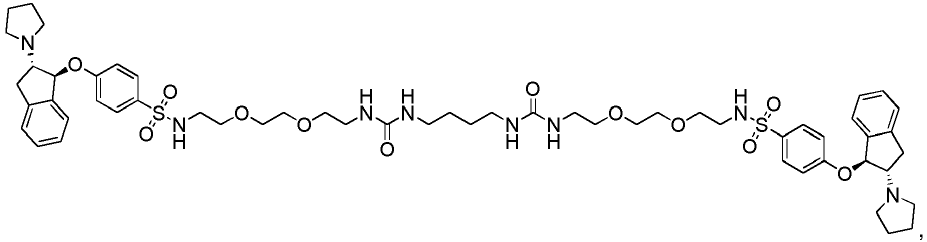 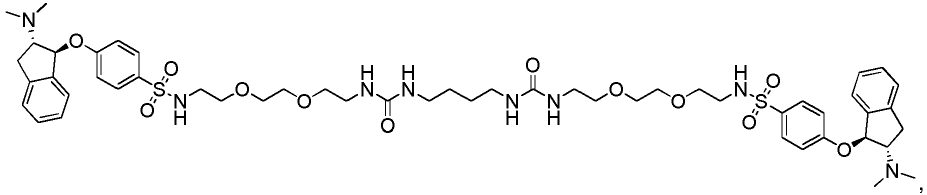 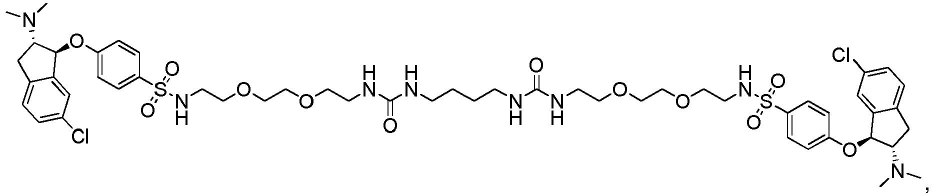 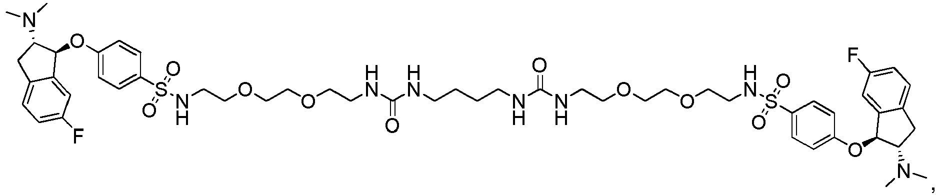 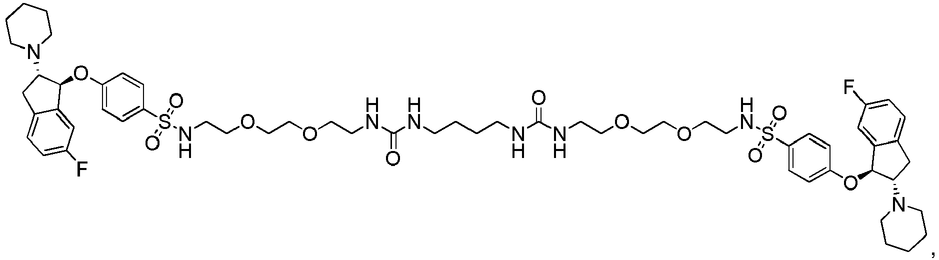 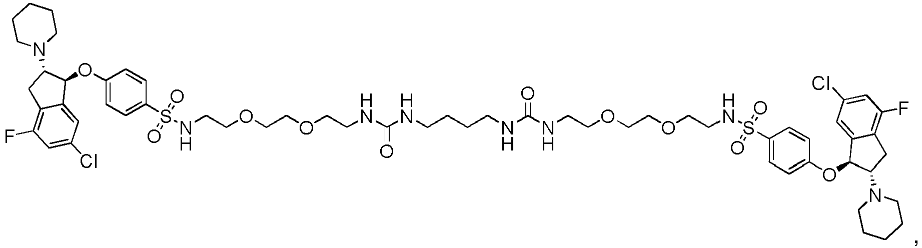 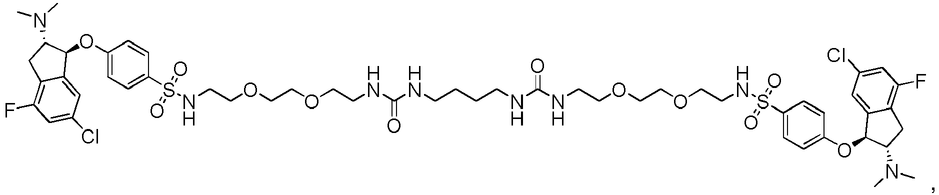 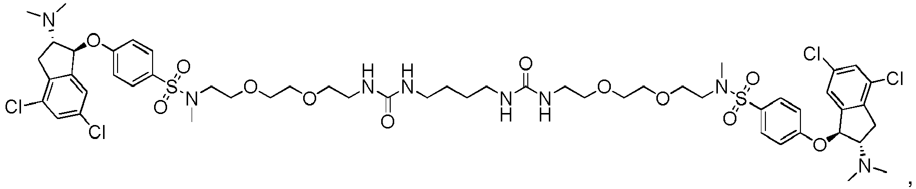 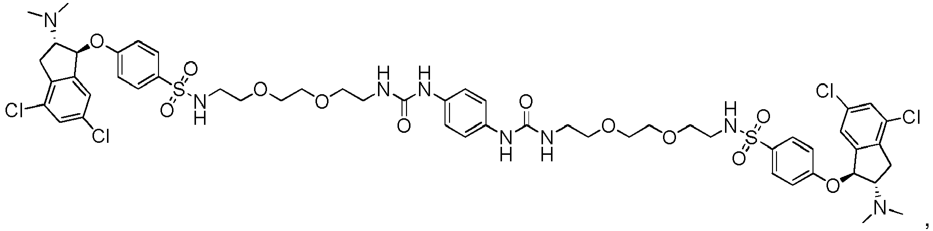 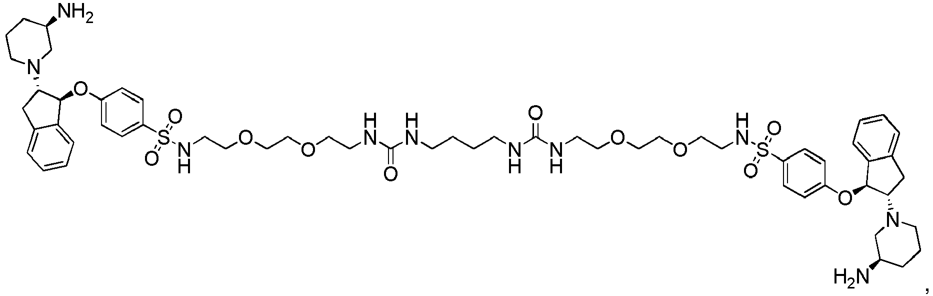 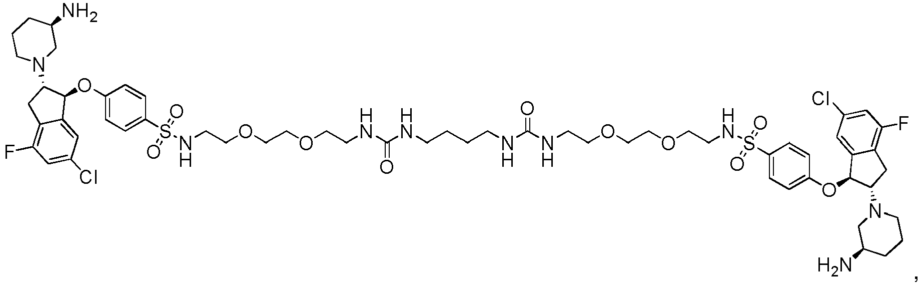 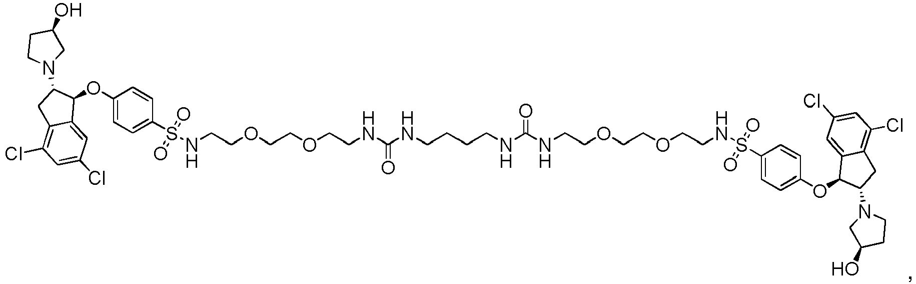 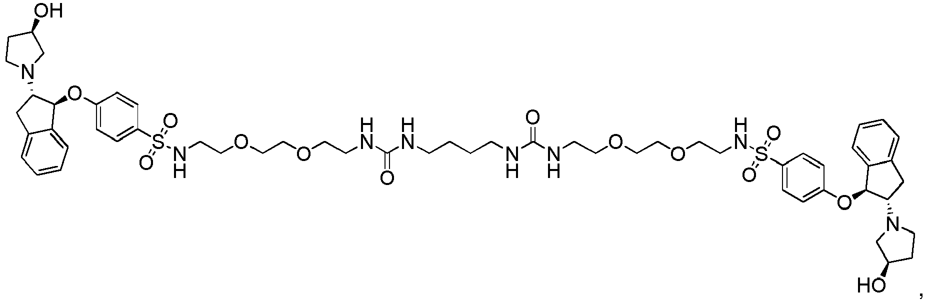 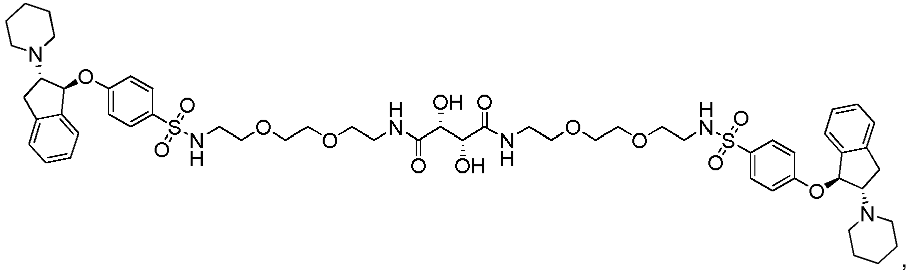 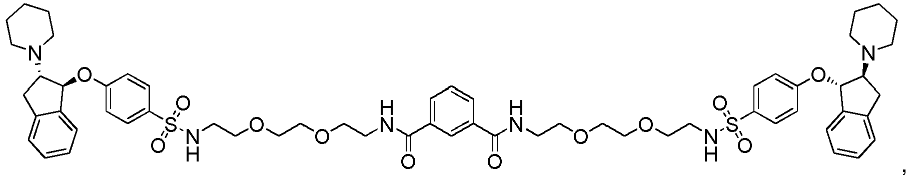 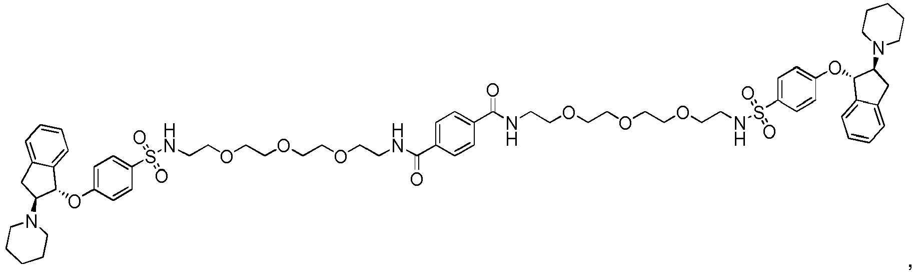 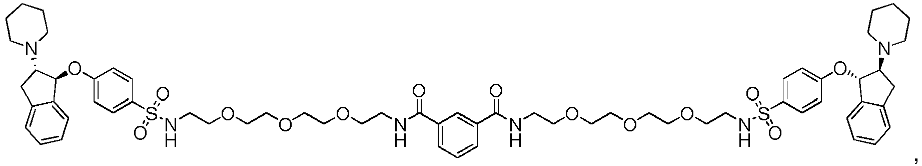 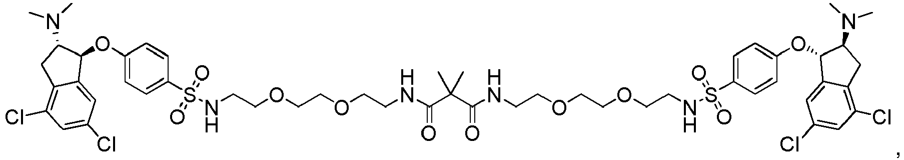 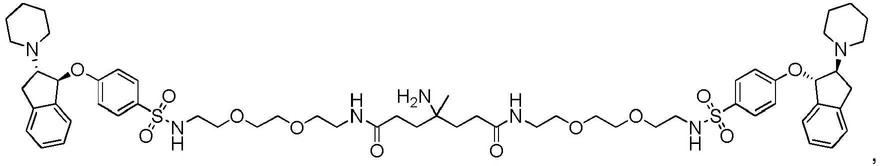 and

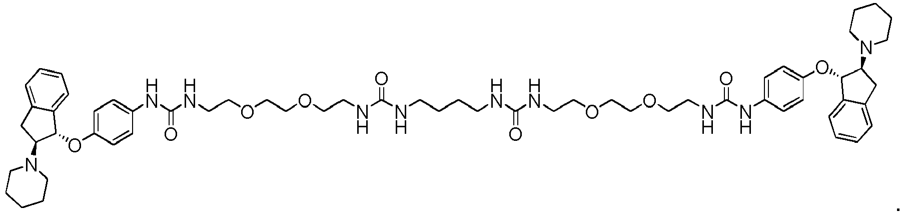

13. A pharmaceutical composition comprising a compound of any one of claims 1-12, or a pharmaceutically acceptable salt thereof, and a pharmaceutically acceptable carrier, diluents or excipient.

14. The compound, or pharmaceutically acceptable salt thereof, of any one of claims 1-12 for inhibiting NHE-mediated antiport of sodium and hydrogen ions.

15. The compound, or pharmaceutically acceptable salt thereof, of any one of claims 1-12 for use in a method of treating a disorder selected from the group consisting of a disorder associated with fluid retention or salt overload, hypertension, edema, a gastrointestinal tract disorder, and irritable bowel syndrome.

16. The compound or pharmaceutically acceptable salt thereof for the use of claim 15, wherein the disorder associated with fluid retention or salt overload is selected from the group consisting of heart failure, chronic kidney disease, end-stage renal disease, liver disease, and peroxisome proliferator-activated receptor (PPAR) gamma agonist-induced fluid retention.

17. The compound or pharmaceutically acceptable salt thereof for the use of claim 15, wherein the disorder is irritable bowel syndrome.

18. The compound or pharmaceutically acceptable salt thereof for the use of claim 16, wherein the disorder associated with fluid retention or salt overload is chronic kidney disease.

19. The compound or pharmaceutically acceptable salt thereof for the use of claim 16, wherein the disorder associated with fluid retention or salt overload is end-stage renal disease.

20. The compound or pharmaceutically acceptable salt thereof for the use of claim 15, wherein the gastrointestinal tract disorder is chronic constipation occurring in cystic fibrosis patients.

21. The compound or pharmaceutically acceptable salt thereof for the use of claim 15, for use in treating or reducing pain associated with the gastrointestinal tract disorder.

22. The compound or pharmaceutically acceptable salt thereof for the use of claim 15, for use in treating or reducing visceral hypersensitivity associated with the gastrointestinal tract disorder.

23. The compound or pharmaceutically acceptable salt thereof for the use of claim 15, for use in treating or reducing inflammation of the gastrointestinal tract.

## Patentansprüche

1. Eine Verbindung mit der folgenden Struktur von Formel (I):
Core(̵L-NHE)ₙ (I)
oder ein pharmazeutisch akzeptables Salz davon,
wobei:
(a) n 2 oder 3 ist;
(b) Core ein Core-Element mit zwei oder drei Stellen zur Bindung an zwei oder drei NHE-inhibierende niedermolekulare Elemente ist;
(c) L eine Bindung oder ein Linker ist, die/der das Core-Element mit den zwei oder drei NHEinhibitorischen niedermolekularen Elementen verbindet, und
(d) NHE ein NHE-inhibierendes niedermolekulares Element mit der folgenden Struktur von Formel (XI) ist: 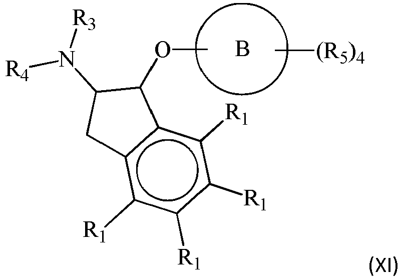 wobei:
B Aryl ist;
jedes R₅ unabhängig ausgewählt ist aus der Gruppe bestehend aus Wasserstoff, Halogen, optional substituiertem C₁₋₄-Alkyl, optional substituiertem C₁₋₄-Alkoxy, optional substituiertem C₁₋₄-Thioalkyl, optional substituiertem Heterocyclyl, optional substituiertem Heterocyclylalkyl, optional substituiertem Aryl, optional substituiertem Heteroaryl, Hydroxyl, Oxo, Cyano, Nitro, -NR₇R₈, - NR₇C(=O)R₈, -NR₇C(=O)OR₈, -NR₇C(=O)NR₈R₉, -NR₇SO₂R₈, -NR₇S(O)₂NR₈R₉, -C(=O)OR₇, -C(=O)R₇, -C(=O)NR₇R₈, -S(O)₁₋₂R₇ und -SO₂NR₇R₈, wobei R₇, R₈ und R₉ unabhängig ausgewählt sind aus der Gruppe bestehend aus Wasserstoff, C₁₋₄-Alkyl, oder einer Bindung, die das NHE-inhibierende niedermolekulare Element mit L verbindet, unter der Voraussetzung, dass mindestens eins eine Bindung ist, die das NHE-inhibierende niedermolekulare Element mit L verbindet;
R₃ und R₄ unabhängig ausgewählt sind aus der Gruppe bestehend aus Wasserstoff, optional substituiertem C₁₋₄-Alkyl, optional substituiertem Cycloalkyl, optional substituiertem Cycloalkylalkyl, optional substituiertem Aryl, optional substituiertem Aralkyl, optional substituiertem Heterocyclyl und optional substituiertem Heteroaryl; oder R₃ und R₄ zusammen mit dem Stickstoff, an den sie gebunden sind, ein optional substituiertes 4-8-gliedriges Heterocyclyl bilden; und
jedes R₁ unabhängig ausgewählt ist aus der Gruppe bestehend aus Wasserstoff, Halogen, optional substituiertem C₁₋₆-Alkyl und optional substituiertem C₁₋₆-Alkoxy.

2. Die Verbindung, oder das pharmazeutisch akzeptable Salz davon, von Anspruch 1 wobei n 2 ist.

3. Die Verbindung, oder das pharmazeutisch akzeptable Salz davon, von Anspruch 1 oder 2, wobei L ein Polyalkylenglycol-Linker ist.

4. Die Verbindung, oder das pharmazeutisch akzeptable Salz davon, von einem der Ansprüche 1-3, wobei L ein Polyethylenglycol-Linker ist.

5. Die Verbindung, oder das pharmazeutisch akzeptable Salz davon, von einem der Ansprüche 1-4, wobei das Core die folgende Struktur hat: 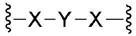 wobei:
X ausgewählt ist aus der Gruppe bestehend aus einer Bindung, -O-, -NH-, -S-, -C₁₋₆-Alkylen, - NHC(=O)-, -C(=O)NH-, -NHC(=O)NH-, -SO₂NH- und -NHSO₂-;
Y ausgewählt ist aus der Gruppe bestehend aus einer Bindung, optional substituiertem C₁₋₈-Alkylen, optional substituiertem Aryl, optional substituiertem Heteroaryl, einem Polyethylenglycol-Linker, -(CH₂)₁₋₆O(CH₂)₁₋₆- und -(CH₂)₁₋₆NY₁(CH₂)₁₋₆-; und
Y₁ ausgewählt ist aus der Gruppe bestehend aus Wasserstoff, optional substituiertem C₁₋₈-Alkyl, optional substituiertem Aryl oder optional substituiertem Heteroaryl.

6. Die Verbindung, oder das pharmazeutisch akzeptable Salz davon, von einem der Ansprüche 1-5, wobei das Core ausgewählt ist aus der Gruppe bestehend aus:

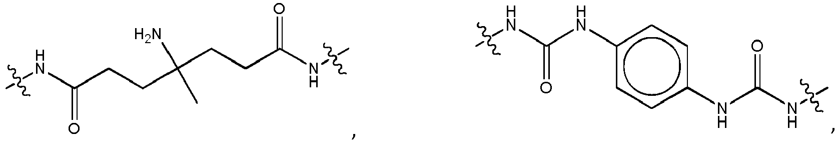

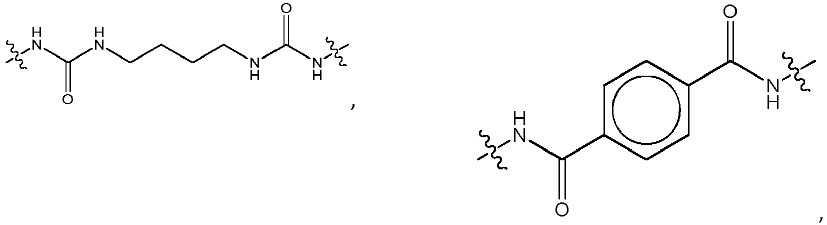

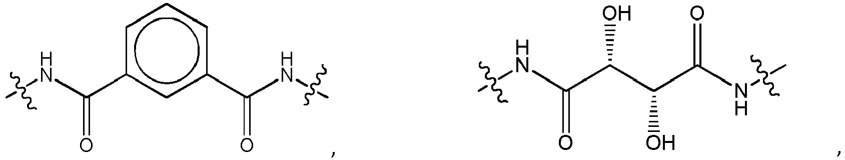

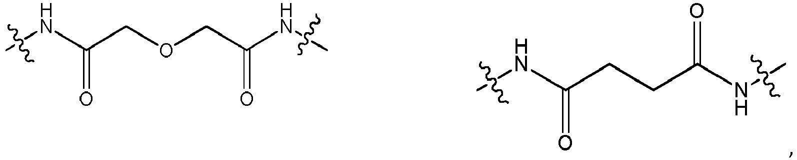

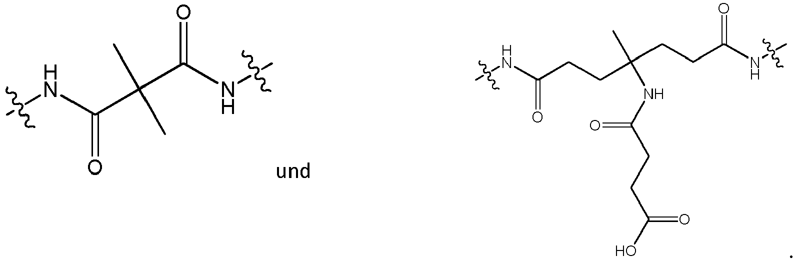

7. Die Verbindung, oder das pharmazeutisch akzeptable Salz davon, von einem der Ansprüche 1-6 wobei das NHE-inhibierende niedermolekulare Element die folgende Struktur von Formel (XII) hat: 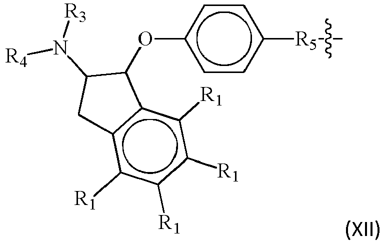 wobei:
jedes R₃ und R₄ unabhängig ist ausgewählt aus der Gruppe bestehend aus Wasserstoff und optional substituiertem C₁₋₄-Alkyl oder R₃ und R₄, zusammengenommen mit dem Stickstoff, an den sie gebunden sind, ein optional substituiertes 4-8-gliedriges Heterocyclyl bilden;
jedes R₁ unabhängig ausgewählt ist aus der Gruppe bestehend aus Wasserstoff, Halogen, C₁₋₆-Alkyl und C₁₋₆-Haloalkyl; und
R₅ ausgewählt ist aus der Gruppe bestehend aus -SO₂-NR₇- und -NHC(=O)NH-, wobei R₇ Wasserstoff oder C₁₋₄-Alkyl ist.

8. Die Verbindung, oder das pharmazeutisch akzeptable Salz davon, von Anspruch 7, wobei R₃ und R₄, zusammengenommen mit dem Stickstoff, an den sie gebunden sind, ein optional substituiertes 5- oder 6-gliedriges Heterocyclyl bilden.

9. Die Verbindung, oder das pharmazeutisch akzeptable Salz davon, von Anspruch 8, wobei das optional substituierte 5- oder 6- gliedrige Heterocyclyl Pyrrolidinyl oder Piperidinyl ist.

10. Die Verbindung, oder das pharmazeutisch akzeptable Salz davon, von Anspruch 7, wobei R₃ und R₄ unabhängig C₁₋₄-Alkyl sind.

11. Die Verbindung, oder das pharmazeutisch akzeptable Salz davon, von einem der Ansprüche 7-10, wobei jedes R₁ unabhängig ausgewählt ist aus der Gruppe bestehend aus Wasserstoff oder Halogen.

12. Die Verbindung, oder das pharmazeutisch akzeptable Salz davon, von einem der Ansprüche 1-11, ausgewählt aus: 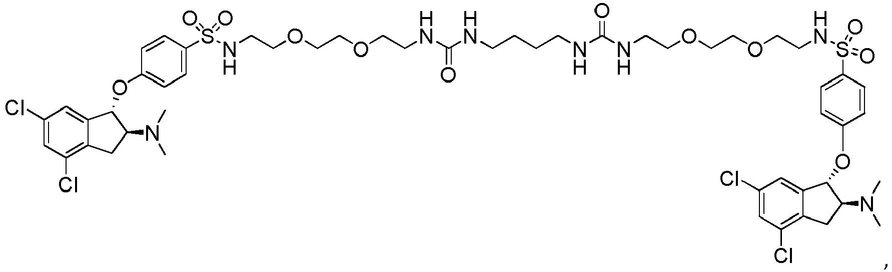 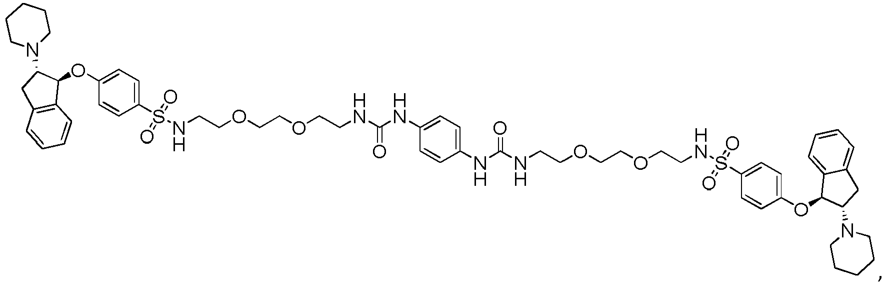 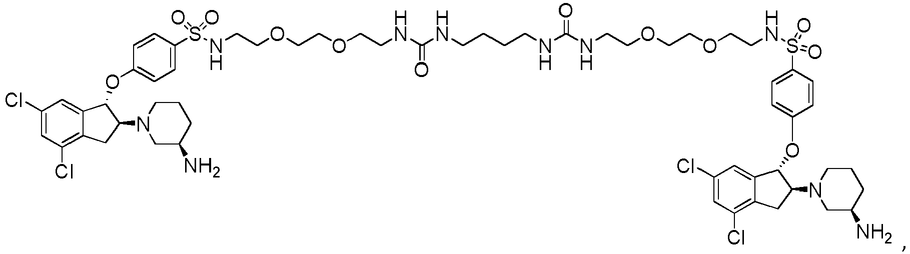 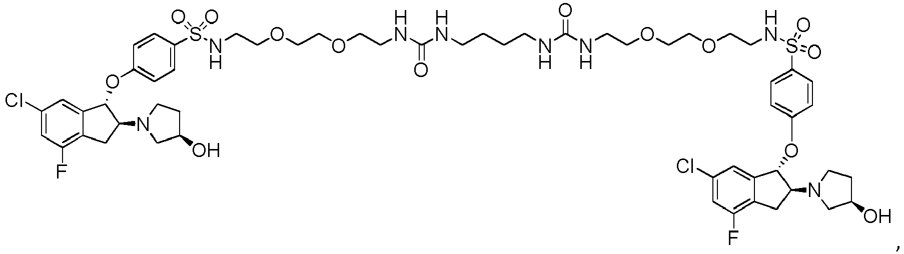 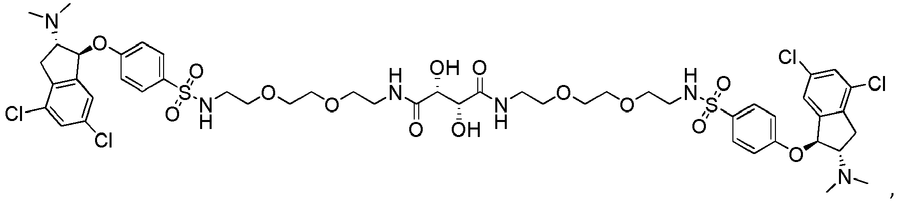 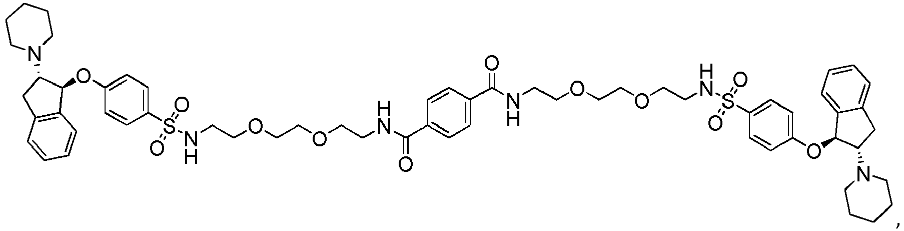 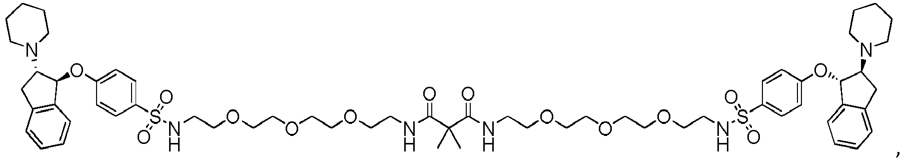 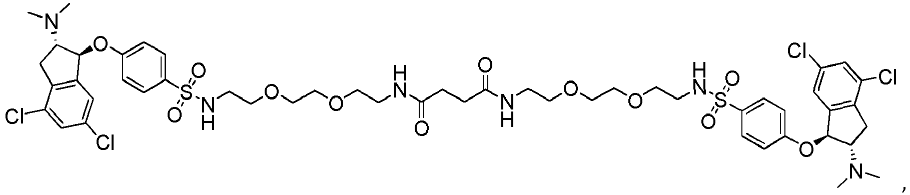 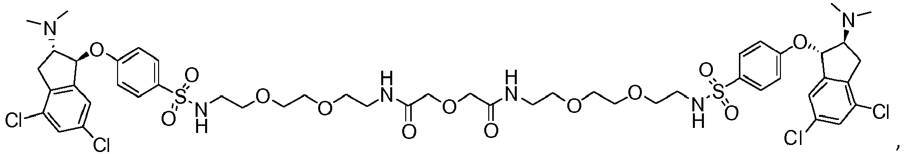 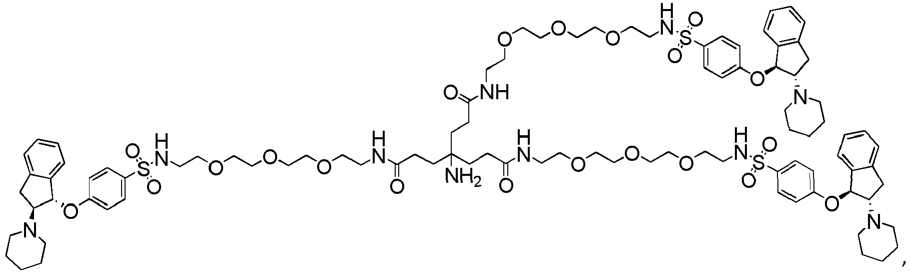 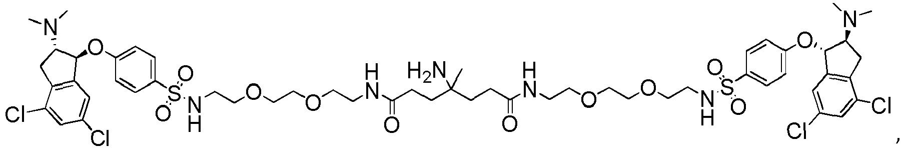 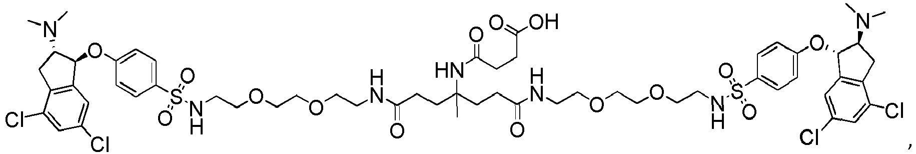 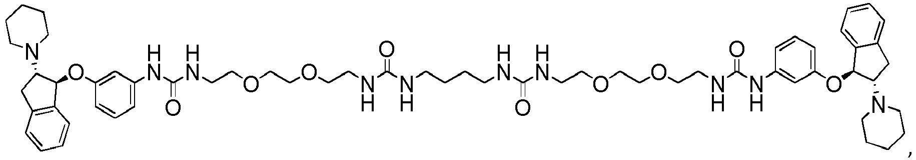 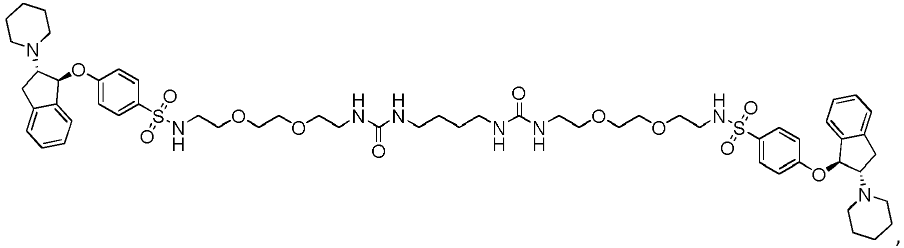 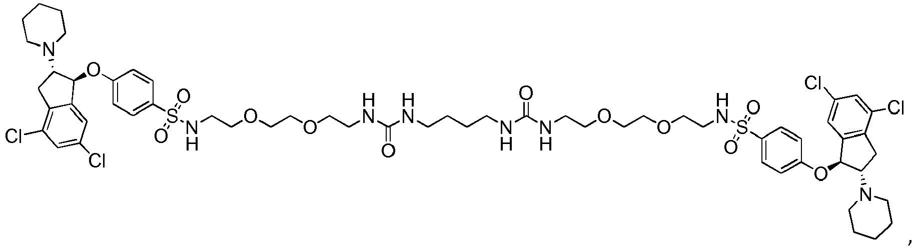 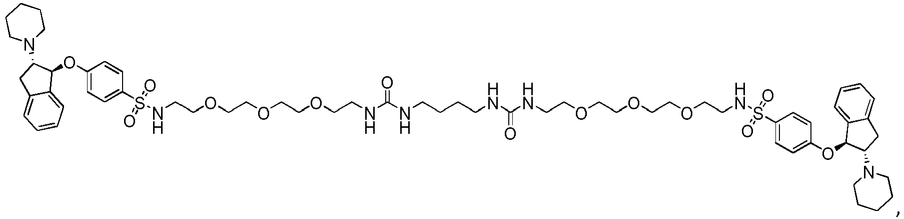 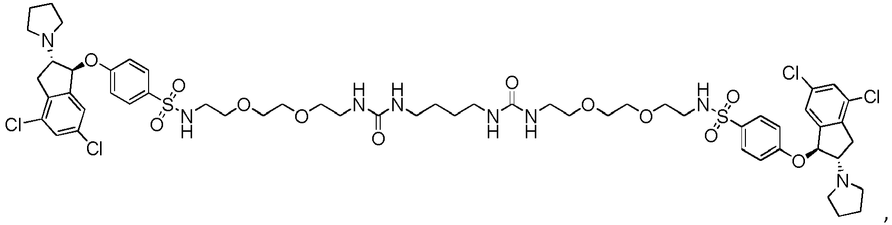 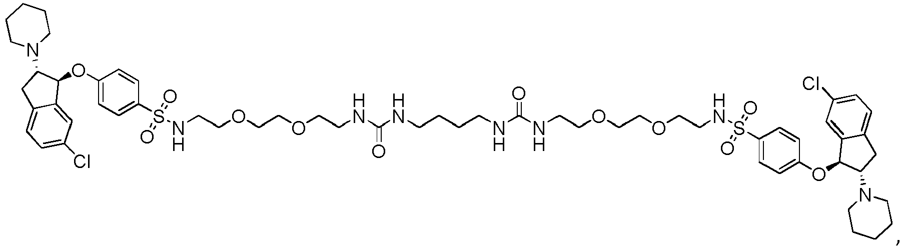 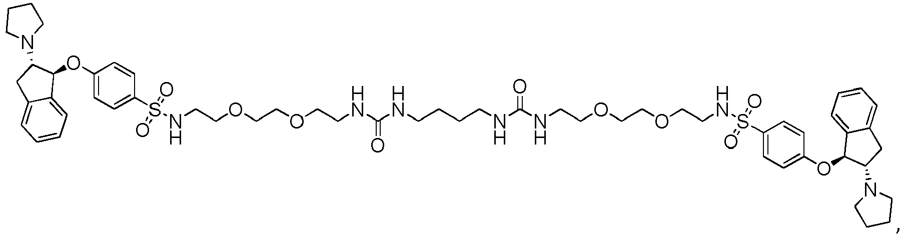 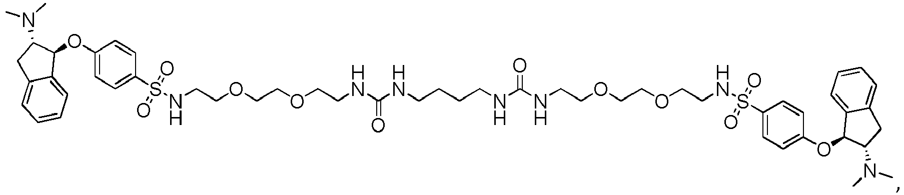 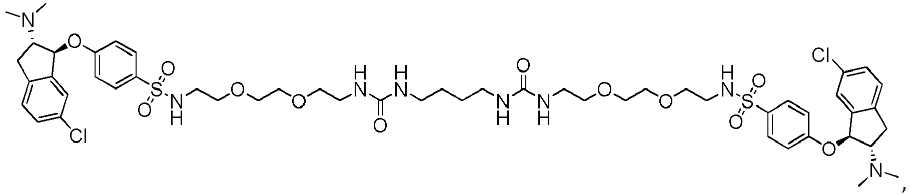 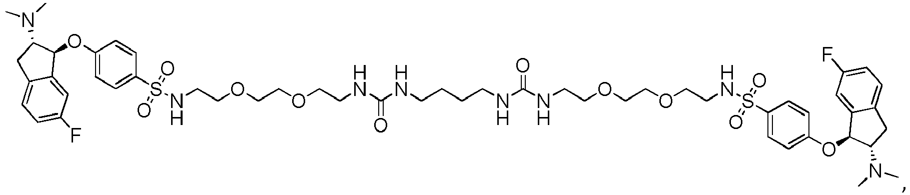 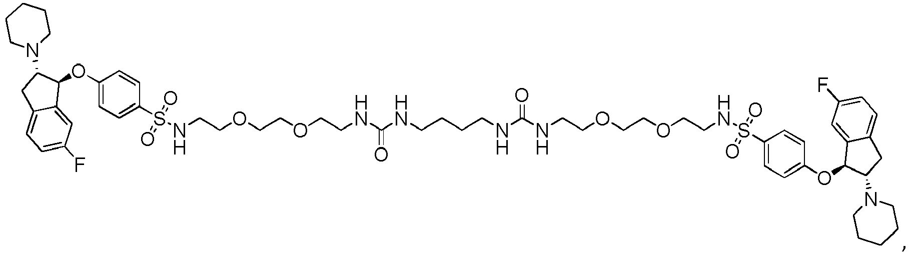 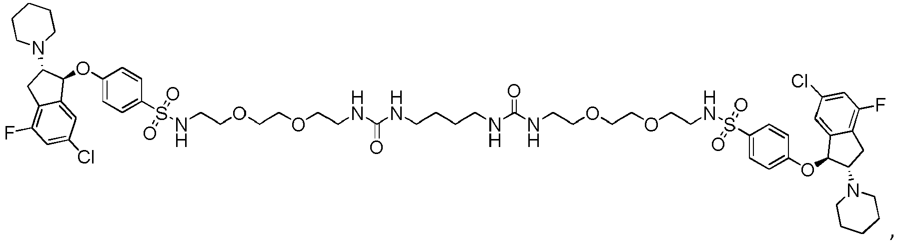 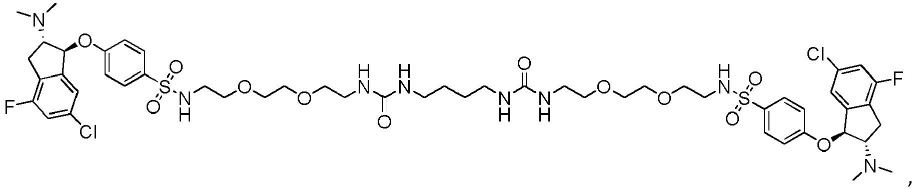 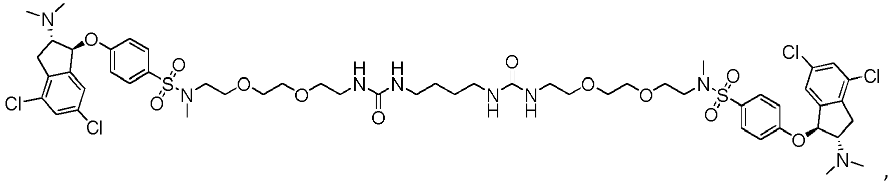 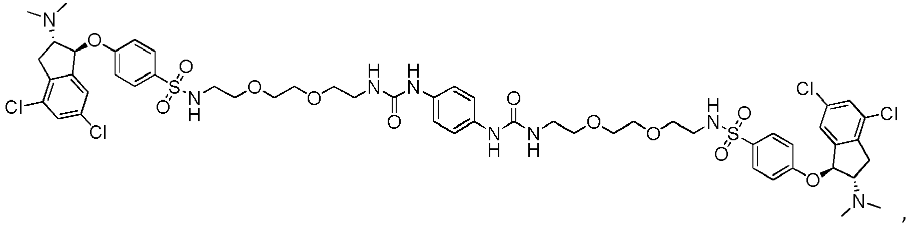 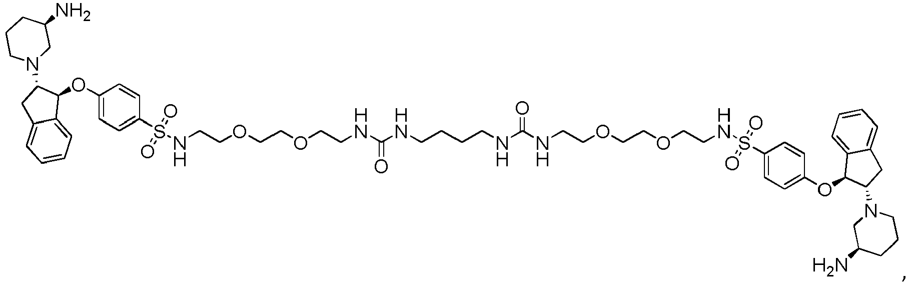 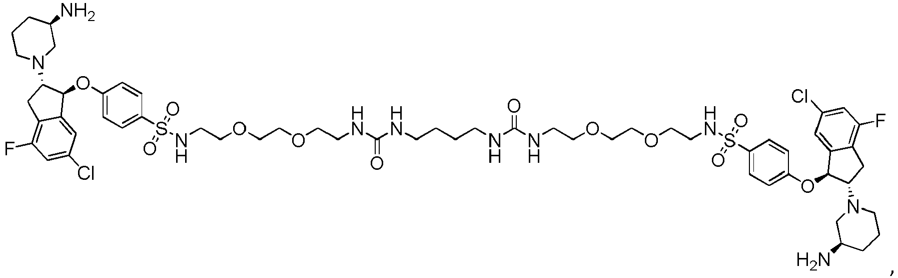 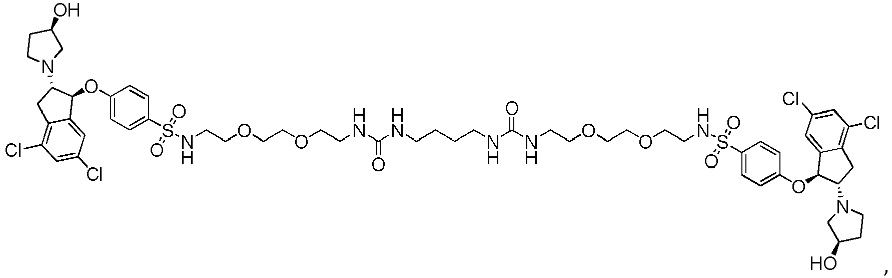 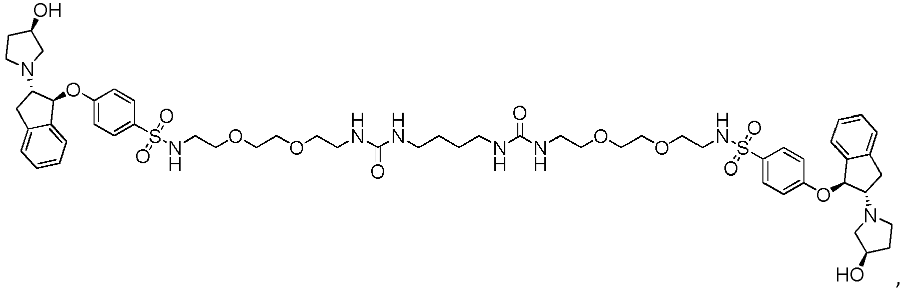 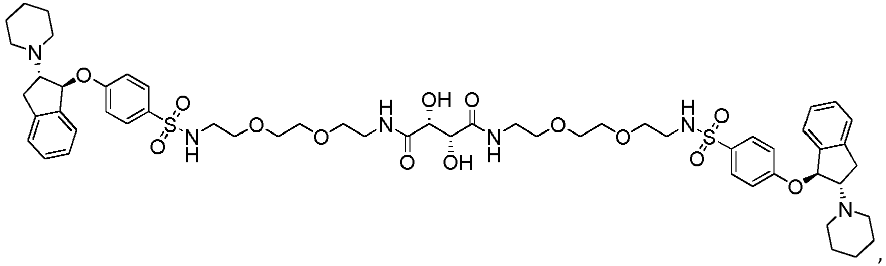 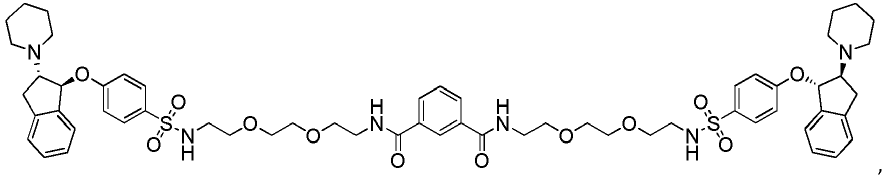 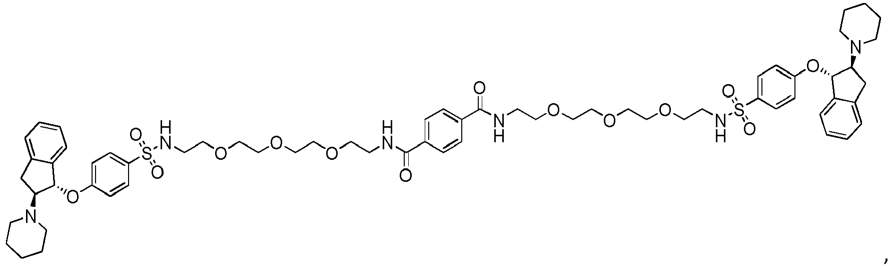 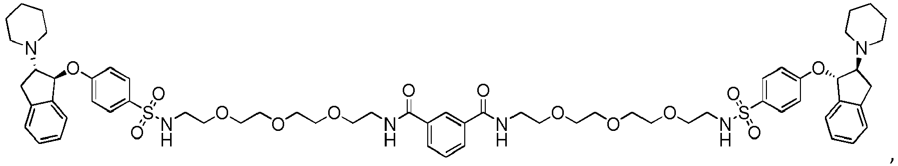 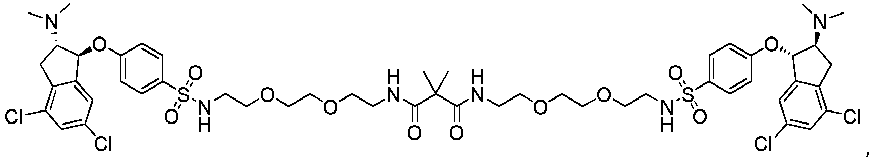 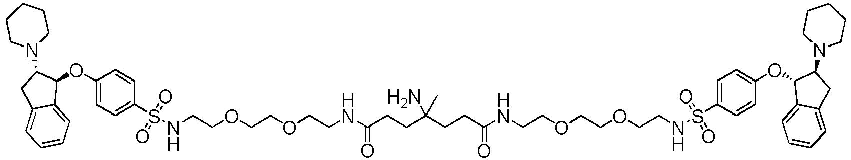 und

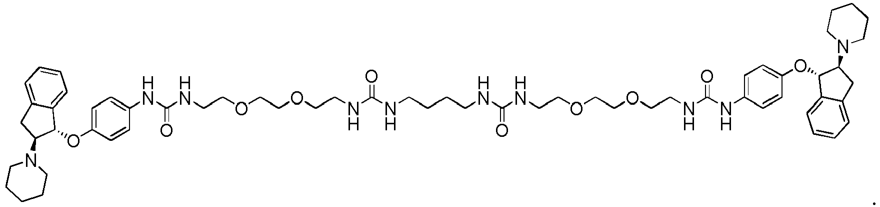

13. Eine pharmazeutische Zusammensetzung, umfassend eine Verbindung von einem der Ansprüche 1-12, oder ein pharmazeutisch akzeptables Salz davon, und einen pharmazeutisch akzeptablen Trägerstoff, Verdünnungsmittel oder einen Hilfsstoff.

14. Die Verbindung, oder das pharmazeutisch akzeptable Salz davon, von einem der Ansprüche 1-12 zur Inhibition von NHE-vermitteltem Antiport von Natrium- und Wasserstoff-Ionen.

15. Die Verbindung, oder das pharmazeutisch akzeptable Salz davon, von einem der Ansprüche 1-12 zur Verwendung in einem Verfahren zur Behandlung einer Erkrankung, ausgewählt aus der Gruppe bestehend aus einer Erkrankung, die mit Flüssigkeitsretention oder Salz-Überlastung assoziiert ist, Bluthochdruck, Ödem, einer Erkrankung des Gastrointestinaltrakts und Reizdarmsyndrom.

16. Die Verbindung oder das pharmazeutisch akzeptable Salz davon zur Verwendung von Anspruch 15, wobei die Erkrankung, die mit Flüssigkeitsretention oder Salz-Überlastung assoziiert ist, ausgewählt ist aus der Gruppe bestehend aus Herzversagen, chronischem Nierenversagen, Nierenversagen im Endstadium, Lebererkrankung und Peroxisom-Proliferator-aktivierter Rezeptor (PPAR) gamma-Agonistinduzierter Flüssigkeitsretention.

17. Die Verbindung oder das pharmazeutisch akzeptable Salz davon zur Verwendung von Anspruch 15, wobei die Erkrankung Reizdarmsyndrom ist.

18. Die Verbindung oder das pharmazeutisch akzeptable Salz davon zur Verwendung von Anspruch 16, wobei die Erkrankung, die mit Flüssigkeitsretention oder Salz-Überlastung assoziiert ist, chronisches Nierenversagen ist.

19. Die Verbindung oder das pharmazeutisch akzeptable Salz davon zur Verwendung von Anspruch 16, wobei die Erkrankung, die mit Flüssigkeitsretention oder Salz-Überlastung assoziiert ist, Nierenversagen im Endstadium ist.

20. Die Verbindung oder das pharmazeutisch akzeptable Salz davon zur Verwendung von Anspruch 15, wobei die Erkrankung des Gastrointestinaltrakts chronische Verstopfung ist, die bei Patienten mit zystischer Fibrose auftritt.

21. Die Verbindung oder das pharmazeutisch akzeptable Salz davon zur Verwendung von Anspruch 15, zur Verwendung bei der Behandlung oder Reduzierung von Schmerz, der mit der Erkrankung des Gastrointestinaltrakts assoziiert ist.

22. Die Verbindung oder das pharmazeutisch akzeptable Salz davon zur Verwendung von Anspruch 15, zur Verwendung bei der Behandlung oder Reduzierung von viszeraler Hypersensitivität, die mit der Erkrankung des Gastrointestinaltrakts assoziiert ist.

23. Die Verbindung oder das pharmazeutisch akzeptable Salz davon zur Verwendung von Anspruch 15, zur Verwendung bei der Behandlung oder Reduzierung von Entzündung des Gastrointestinaltrakts.

## Revendications

1. Composé répondant à la structure suivante de formule (I) :
Noyau(̵L-NHE)ₙ (I)
ou sel pharmaceutiquement acceptable de celui-ci, dans lequel :
(a) n vaut 2 ou 3 ;
(b) Noyau est une fraction de noyau renfermant deux ou trois sites pour fixation à deux ou trois fractions de petite molécule inhibant NHE ;
(c) L est une liaison ou un lieur reliant la fraction de noyau aux deux ou trois fractions de petite molécule inhibitrice de NHE ; et
(d) NHE est une fraction de petite molécule inhibant NHE répondant à la structure suivante de formule (XI) : 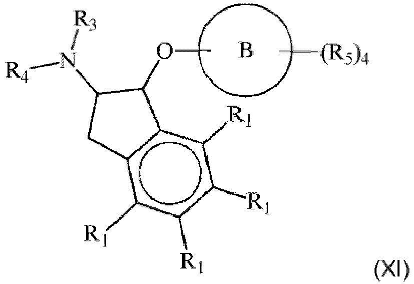 dans lequel :
B est aryle ;
chaque R₅ est indépendamment choisi dans le groupe consistant en hydrogène, halogène, alkyle en C₁ à C₄ facultativement substitué, alcoxy en C₁ à C₄ facultativement substitué, thioalkyle en C₁ à C₄ facultativement substitué, hétérocyclyle facultativement substitué, hétérocyclylalkyle facultativement substitué, aryle facultativement substitué, hétéroaryle facultativement substitué, hydroxyle, oxo, cyano, nitro, -NR₇R₈, -NR₇C(=O)R₈, -NR₇C(=O)OR₈, -NR₇C(=O)NR₈R₉, -NR₇SO₂R₈, -NR₇S(O)₂NR₈R₉, -C(=O)OR₇, -C(=O)R₇,
-C(=O)NR₇R₈, -S(O)₁₋₂R₇, et -SO₂NR₇R₈, dans lequel R₇, R₈, et R₉ sont indépendamment choisis dans le groupe consistant en hydrogène, alkyle en C₁ à C₄, ou une liaison reliant la fraction de petite molécule inhibant NHE à L, à condition qu'au moins l'une soit une liaison liant la fraction de petite molécule inhibant NHE à L ;
R₃ et R₄ sont indépendamment choisis dans le groupe consistant en hydrogène, alkyle en C₁ à C₄ facultativement substitué, cycloalkyle facultativement substitué, cycloalkylalkyle facultativement substitué, aryle facultativement substitué, aralkyle facultativement substitué, hétérocyclyle facultativement substitué et hétéroaryle facultativement substitué ; ou R₃ et R₄ forment conjointement avec l'azote auquel ils sont liés un hétérocyclyle à 4 à 8 chaînons facultativement substitué ; et
chaque R₁ est indépendamment choisi dans le groupe consistant en hydrogène, halogène, alkyle en C₁ à C₆ facultativement substitué et alcoxy en C₁ à C₆ facultativement substitué.

2. Composé, ou sel pharmaceutiquement acceptable de celui-ci, selon la revendication 1, dans lequel n vaut 2.

3. Composé, ou sel pharmaceutiquement acceptable de celui-ci, selon la revendication 1 ou 2, dans lequel L est un lieur polyalkylène glycol.

4. Composé, ou sel pharmaceutiquement acceptable de celui-ci, selon l'une quelconque des revendications 1 à 3, dans lequel L est un lieur polyéthylène glycol.

5. Composé, ou sel pharmaceutiquement acceptable de celui-ci, selon l'une quelconque des revendications 1 à 4, dans lequel le noyau répond à la structure suivante : 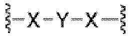 dans lequel ;
X est choisi dans le groupe consistant en une liaison, -O-, -NH-, -S-, alkylène en C₁ à C₆, -NHC(=O)-, -C(=O)NH-, -NHC(=O)NH-, -SO₂NH-, et -NHSO₂- ;
Y est choisi dans le groupe consistant en une liaison, alkylène en C₁ à C₈ facultativement substitué, aryle facultativement substitué, hétéroaryle facultativement substitué, un lieur polyéthylène glycol, - (CH₂)₁₋₆O(CH₂)₁₋₆- et -(CH₂)₁₋₆NY₁(CH₂)₁₋₆- ; et
Y₁ est choisi dans le groupe consistant en hydrogène, alkyle en C₁ à C₈ facultativement substitué, aryle facultativement substitué ou hétéroaryle facultativement substitué.

6. Composé, ou sel pharmaceutiquement acceptable de celui-ci, selon l'une quelconque des revendications 1 à 5, dans lequel le noyau est choisi dans le groupe consistant en :

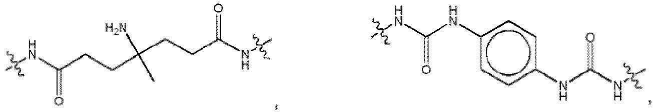

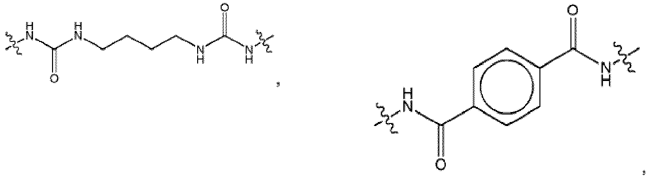

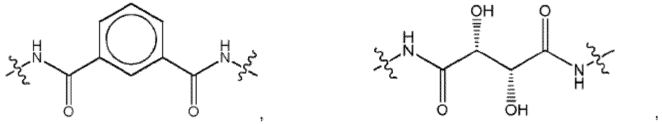

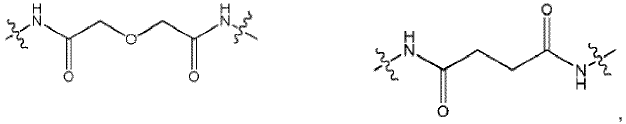

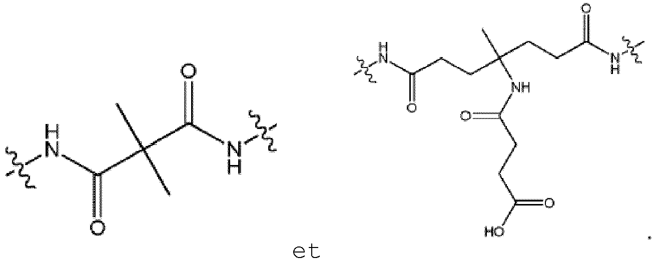

7. Composé, ou sel pharmaceutiquement acceptable de celui-ci, selon l'une quelconque des revendications 1 à 6, dans lequel la fraction de petite molécule inhibant NHE répond à la structure suivante de formule (XII) : 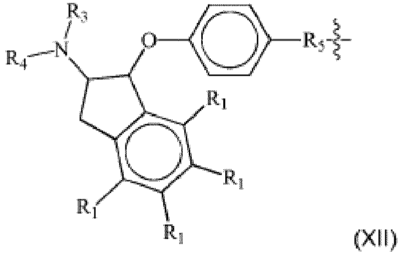 dans lequel :
chacun de R₃ et R₄ est indépendamment choisi dans le groupe consistant en hydrogène et alkyle en C₁ à C₄ facultativement substitué, ou R₃ et R₄, conjointement avec l'azote auquel ils sont liés, forment un hétérocyclyle à 4 à 8 chaînons facultativement substitué ;
chaque R₁ est indépendamment choisi dans le groupe consistant en hydrogène, halogène, alkyle en C₁ à C₆, et halogénoalkyle en C₁ à C₆ ; et
R₅ est choisi dans le groupe consistant en -SO₂-NR₇-, et -NHC(=O)NH-, dans lequel R₇ est hydrogène ou alkyle en C₁ à C₄.

8. Composé, ou sel pharmaceutiquement acceptable de celui-ci, selon la revendication 7, dans lequel R₃ et R₄, conjointement avec l'azote auquel ils sont liés, forment un hétérocyclyle à 5 ou 6 chaînons facultativement substitué.

9. Composé, ou sel pharmaceutiquement acceptable de celui-ci, selon la revendication 8, dans lequel l'hétérocyclyle à 5 ou 6 chaînons facultativement substitué est un pyrrolidinyle ou pipéridinyle.

10. Composé, ou sel pharmaceutiquement acceptable de celui-ci, selon la revendication 7, dans lequel R₃ et R₄ sont indépendamment alkyle en C₁ à C₄.

11. Composé, ou sel pharmaceutiquement acceptable de celui-ci, selon l'une quelconque des revendications 7 à 10, dans lequel chaque R₁ est indépendamment choisi dans le groupe consistant en hydrogène ou halogène.

12. Composé, ou sel pharmaceutiquement acceptable de celui-ci, selon l'une quelconque des revendications 1 à 11 choisi parmi : 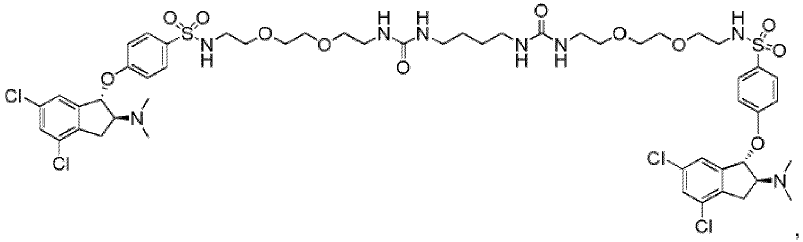 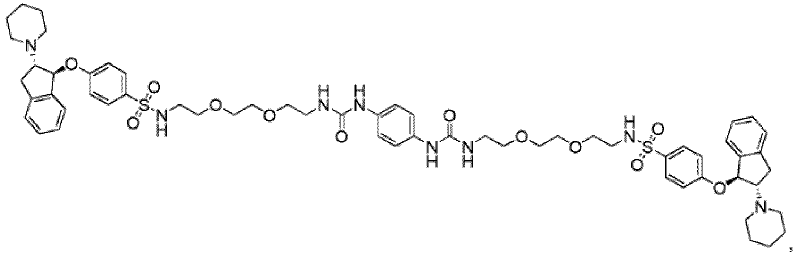 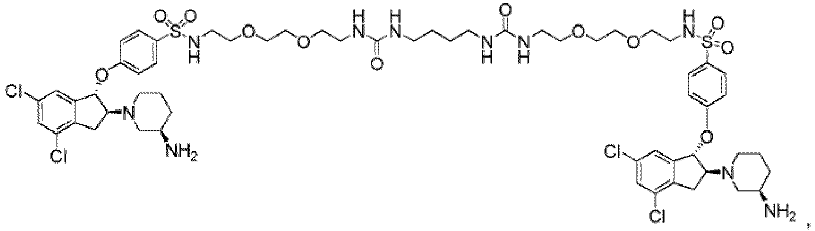 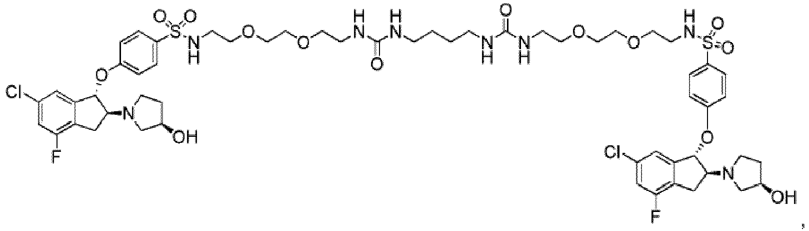 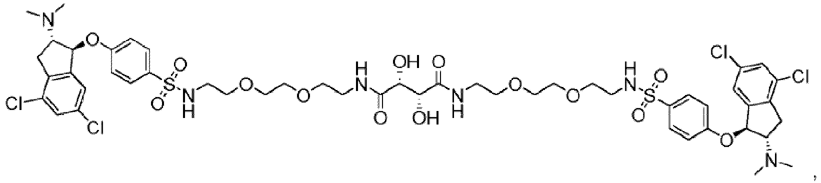 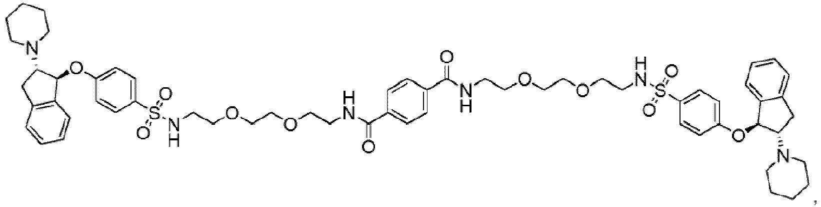 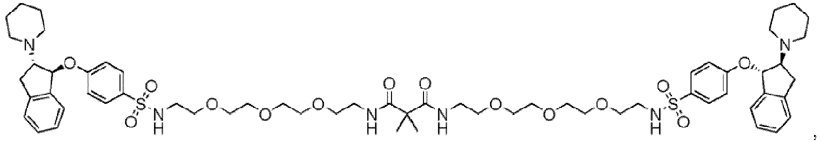 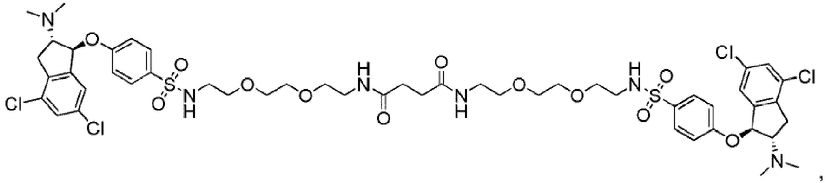 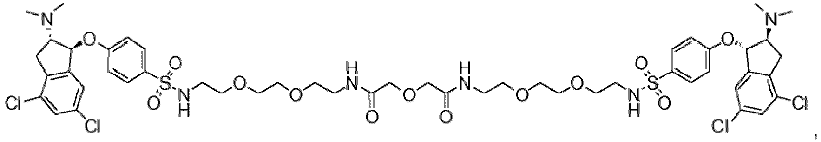 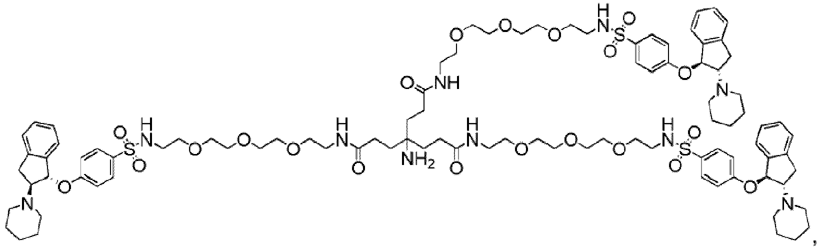 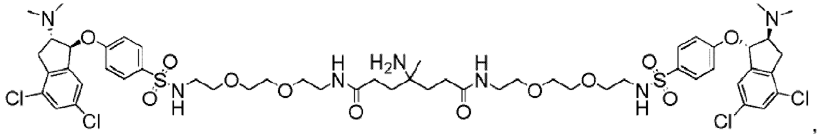 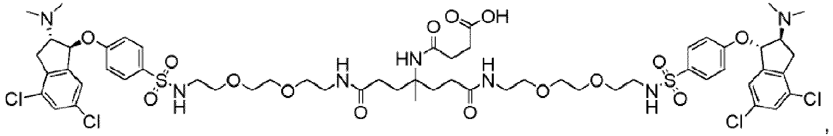 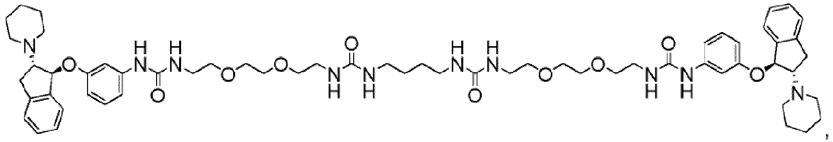 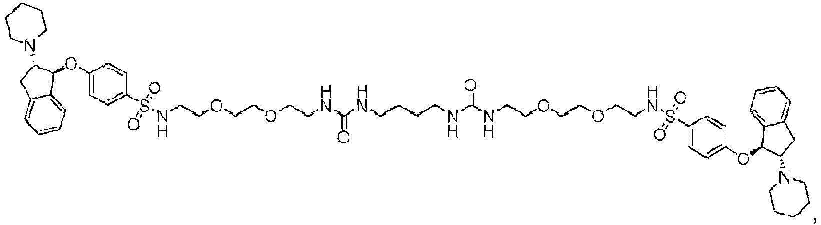 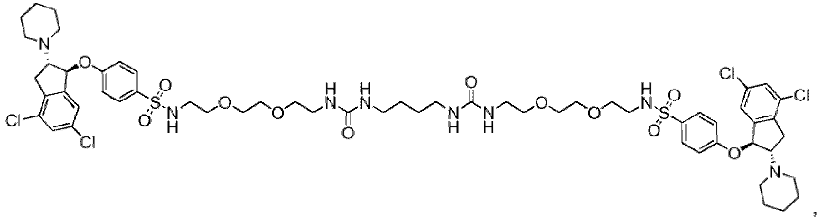 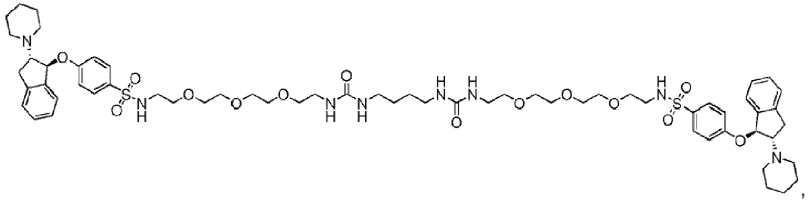 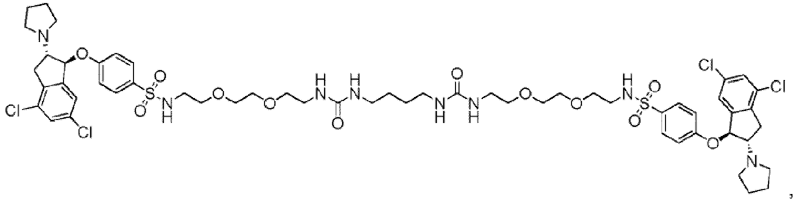 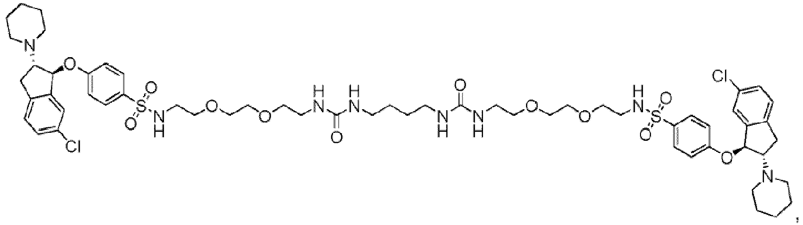 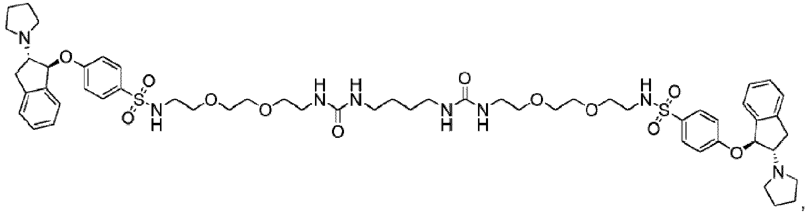 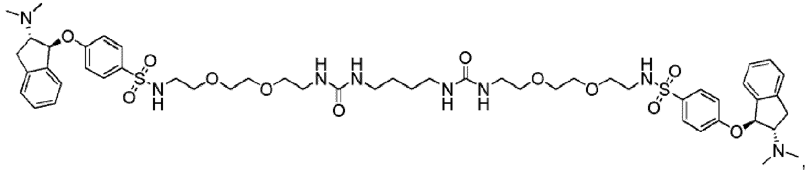 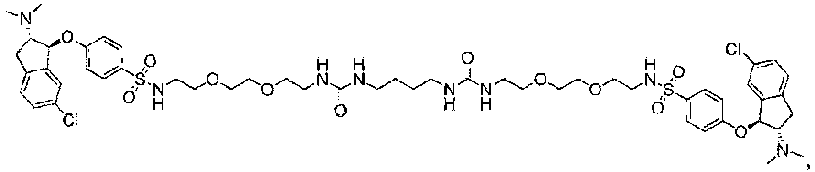 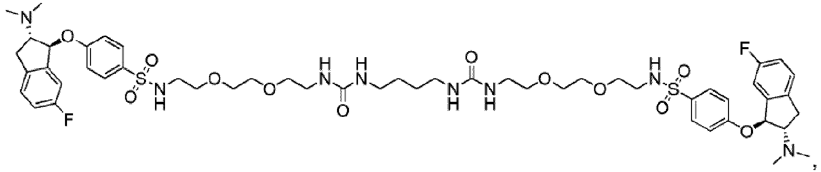 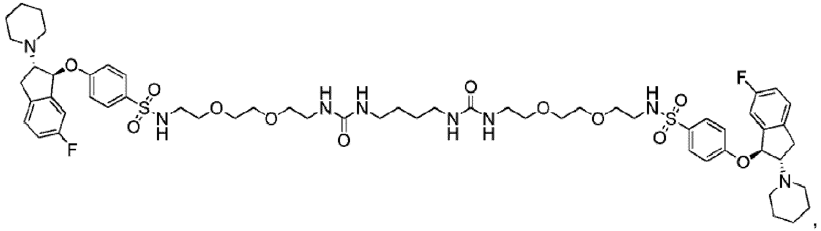 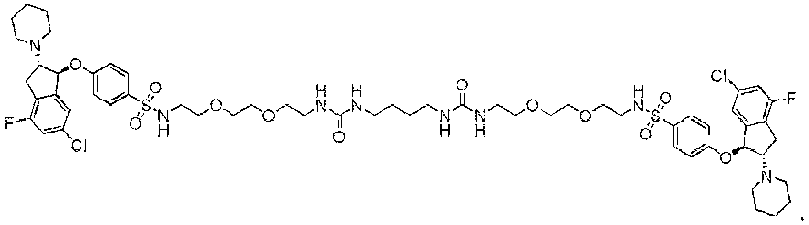 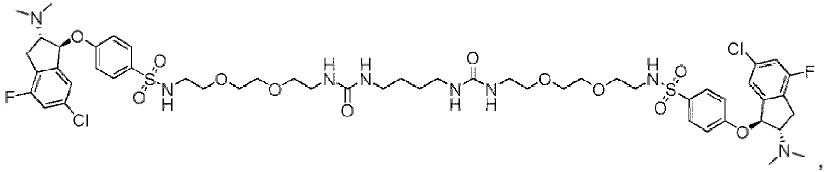 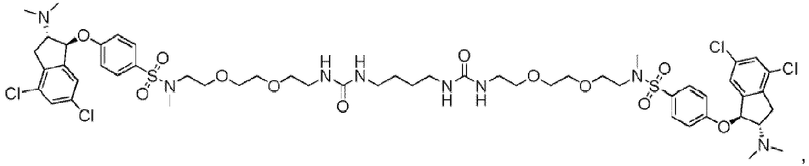 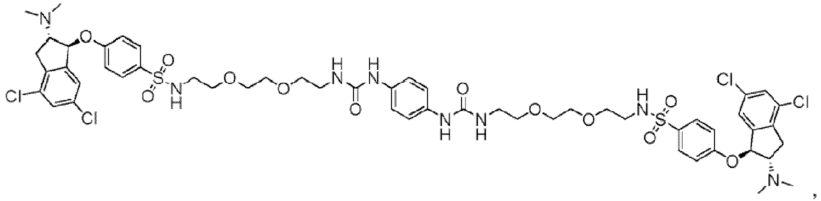 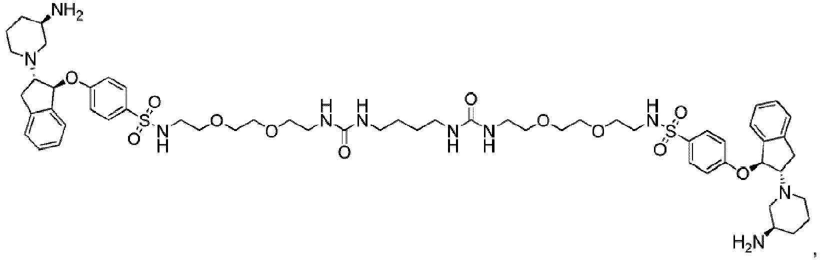 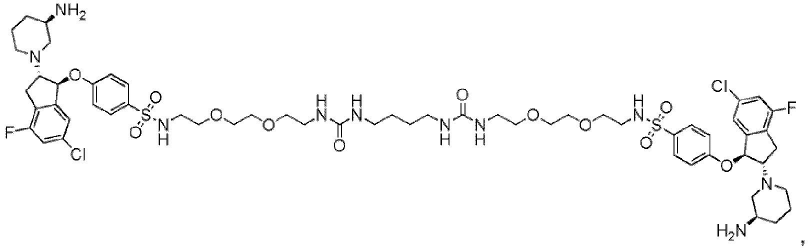 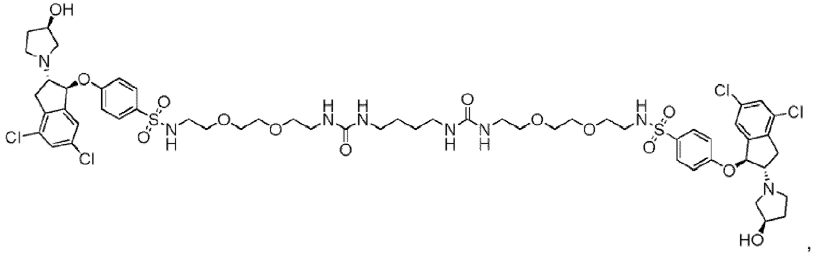 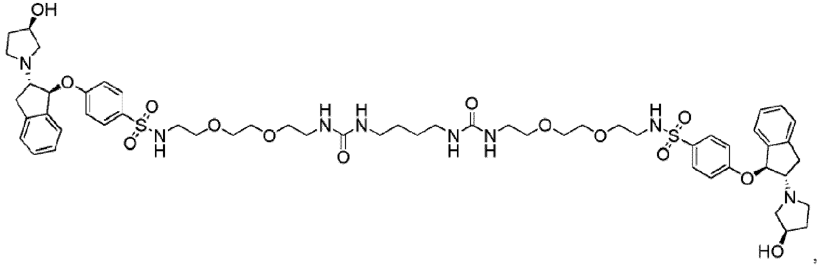 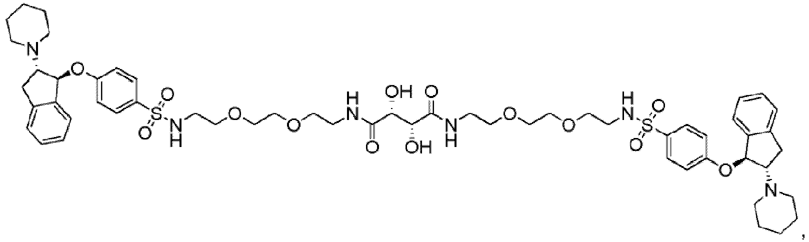 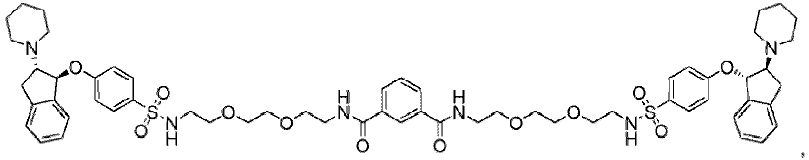 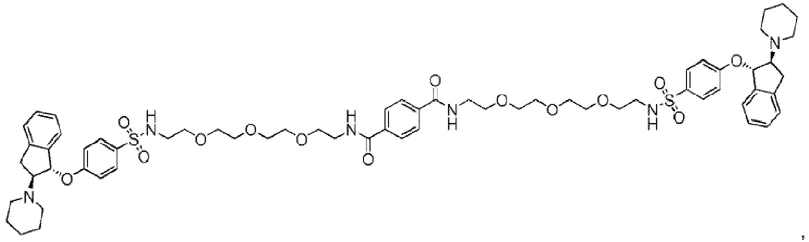 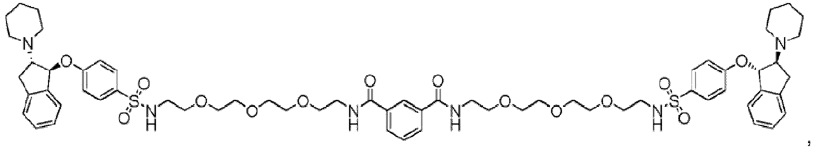 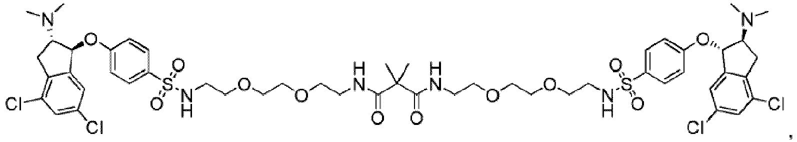 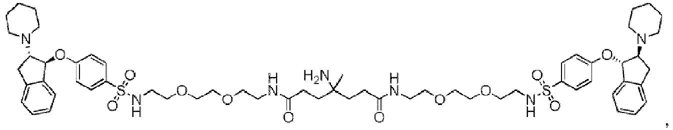 et

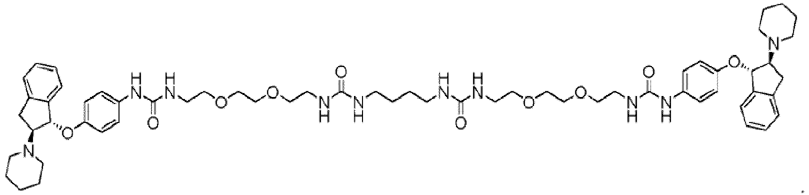

13. Composition pharmaceutique comprenant un composé selon l'une quelconque des revendications 1 à 12, ou un sel pharmaceutiquement acceptable de celui-ci, et un support, des diluants, ou un excipient pharmaceutiquement acceptable(s).

14. Composé, ou sel pharmaceutiquement acceptable de celui-ci, selon l'une quelconque des revendications 1 à 12, destiné à inhiber l'antiport médié par NHE des ions sodium et hydrogène.

15. Composé, ou sel pharmaceutiquement acceptable de celui-ci, selon l'une quelconque des revendications 1 à 12, à utiliser dans un procédé de traitement d'un trouble choisi dans le groupe consistant à un trouble associé à une rétention de fluide ou à une surcharge en sel, l'hypertension, un oedème, un trouble du tractus gastro-intestinal, et le syndrome du côlon irritable.

16. Composé, ou sel pharmaceutiquement acceptable de celui-ci, destiné à l'utilisation selon la revendication 15, dans lequel le trouble associé à la rétention de fluide ou à la surcharge en sel est choisi dans le groupe consistant en l'insuffisance cardiaque, une néphropathie chronique, l'insuffisance rénale terminale, une maladie du foie et une rétention de fluide induite par un agoniste du récepteur activé par les proliférateurs des péroxysomes (PPAR) gamma.

17. Composé, ou sel pharmaceutiquement acceptable de celui-ci, destiné à l'utilisation selon la revendication 15, dans lequel le trouble est le syndrome du côlon irritable.

18. Composé, ou sel pharmaceutiquement acceptable de celui-ci, destiné à l'utilisation selon la revendication 16, dans lequel le trouble associé à la rétention de fluide ou à la surcharge en sel est une néphropathie chronique.

19. Composé, ou sel pharmaceutiquement acceptable de celui-ci, destiné à l'utilisation selon la revendication 16, dans lequel le trouble associé à la rétention de fluide ou à la surcharge en sel est une insuffisance rénale terminale.

20. Composé, ou sel pharmaceutiquement acceptable de celui-ci, destiné à l'utilisation selon la revendication 15, dans lequel le trouble du tractus gastro-intestinal est la constipation chronique se produisant chez les patients atteints de mucoviscidose.

21. Composé, ou sel pharmaceutiquement acceptable de celui-ci, destiné à l'utilisation selon la revendication 15, à utiliser dans le traitement ou la réduction de la douleur associée au trouble du tractus gastro-intestinal.

22. Composé, ou sel pharmaceutiquement acceptable de celui-ci, destiné à l'utilisation selon la revendication 15, à utiliser dans le traitement ou la réduction de l'hypersensibilité viscérale associée au trouble du tractus gastro-intestinal.

23. Composé, ou sel pharmaceutiquement acceptable de celui-ci, destiné à l'utilisation selon la revendication 15, à utiliser dans le traitement ou la réduction de l'inflammation du tractus gastro-intestinal.
